# EUROPEAN PATENT APPLICATION

(11) **EP 4 477 754 A2**
(43) Date of publication of application: **18.12.2024**
(21) Application number: 23753192.6
(22) Date of filing: 09.02.2023
(51) Int. Cl.: C12N 15/63, C12N 9/22, C12N 15/113

(54) **TARGET SYSTEM FOR HOMOLOGY-DIRECTED REPAIR AND GENE EDITING METHOD USING SAME**

(30) Priority: 09.02.2022 KR 20220017179
(71) Applicant: GenKOre Inc., Daejeon 34141 (KR)
(72) Inventor: KIM, Yong Sam, Daejeon 34118 (KR); KIM, Do Yon, Daejeon 34069 (KR); LEE, Jeong Mi, Daejeon 34141 (KR); CHIN, Hyun Jung, Daejeon 34141 (KR)
(74) Representative: Dehns
(86) International application number: PCT/KR2023/001945
(87) International publication number: WO 2023/153845

(57) **Abstract**

The present invention relates to a novel target nucleic acid editing system including a miniaturized nucleic acid editing protein and an engineered guide RNA, uses of homology-directed repair (HDR) in a target gene thereof, and the like. According to one embodiment, homology-directed repair (HDR) using the target nucleic acid editing system has high HDR efficiency compared to other CRISPR/Cas systems due to a tendency to cut the back (outside) of a target nucleic acid, and also has the effect of enabling packaging of a gene editing system including a donor nucleic acid in a single vector, even when the packaging size is very limited like adeno-associated viruses (AAV), and maximizing the HDR efficiency by adding shRNA that inhibits a non-homologous end joining (NHEJ) process.

## Description

### Technical Field

The present disclosure relates to a new hypercompact CRISPR/Cas12f nucleic acid editing system with improved gene editing efficiency by homology-directed repair.

This application claims priority based on Korean Patent Application No. 10-2022-0017179 filed on February 9, 2022, the entire disclosure of which is incorporated herein by reference.

### Background Art

Double-strand breaks in DNA can be repaired through DNA repair mechanism called non-homologous end joining (NHEJ) or homology-directed repair (HDR). In the course of repair by non-homologous end joining (NHEJ), random insertion or deletion of bases (insertion and deletion, indel) occurs between DNA break sites, which results in frameshift mutation or premature mutation in the gene where DNA double-strand breaks have occurred, thereby knocking out the gene. On the other hand, homology-directed repair (HDR) requires a donor DNA (homologous template) to repair broken DNA, and a new sequence with the sequence of this donor DNA as a template can be introduced into a DNA break site. Thus, homology-directed repair (HDR) can be utilized for precise gene editing.

Currently, gene editing technology, represented by the CRISPR/Cas system, is a core technology for development of gene therapy for cancer, genetic diseases, infectious diseases, and the like, and various techniques have been developed in terms of efficiency, safety, and deliverability for gene editing.

Gene editing technology has potential as a gene therapy for various diseases, such as many cancers, genetic diseases, and infectious diseases, because it is possible to delete pathogenic genes that cause diseases (through DNA double-strand breaks and indels). In this connection, gene editing technology, which enables or can improve efficiency of homology-directed repair (HDR) that can introduce a desired gene for therapeutic purpose, may have broader applications.

In addition, for gene therapy, it is most important to efficiently deliver a gene editing system to cells throughout the body, and an efficient vehicle is required for this purpose. Adeno-associated virus (AAV) is an FDA-approved vehicle for gene therapy due to its safety, persistence, and compatibility with mass production (Non-Patent Document 1), and thus it is recognized that a base-editor system, which can contain all components in one AAV vector, will be used as an important tool for treating genetic diseases (Non-patent Document 2). However, due to limited packaging capacity of AAV, genes that can be delivered by AAV have a limited size of less than 4.7 kb. This suggests that there are limitations in clinical application when attempting to use the AAV vector as an intracellular delivery vehicle for gene therapy, as most existing base-editor (CRISPR/Cas) systems generally exceed a size of about 4.7 kb (Non-patent Document 3). For this reason, SaCas9 (Non-Patent Document 4), CjCas9 (Non-Patent Document 5), and the like, which have a molecular weight smaller than Cas9, have been studied as gene editing tools that can be delivered into cells using AAV as a vehicle.

As such, there is a need for a novel system that has a sufficiently small molecular weight with high targeting efficiency and editing activity within cells. In addition, from the viewpoint that the current gene editing technology has low HDR efficiency, there is a need for a gene editing tool that has dramatically enhanced HDR efficiency and thus enables precise gene editing.

### Disclosure of Invention

### Technical Problem

The object of the present disclosure is to solve the above-mentioned problems of the prior art.

Another object of the present disclosure is to provide a gene editing technology which exhibits improved homology directed repair (HDR) efficiency and/or can be implemented as a hypercompact construct that is accommodatable in various vector systems including AAV vectors.

The object of the present disclosure is not limited to the above-mentioned objects. The objects of the present disclosure will become clearer from the following description and may be realized by means and combinations thereof as set forth in the claims.

### Solution to Problem

Representative configurations of the present disclosure to achieve the above-mentioned objects are as follows.

According to an aspect of the present disclosure, there is provided a system or a composition for editing a target nucleic acid, comprising an endonuclease comprising Cas12f1, TnpB, or a variant protein thereof, or a nucleic acid encoding the endonuclease; an engineered guide RNA comprising a guide sequence or a nucleic acid encoding the guide RNA; and a donor nucleic acid molecule or a nucleic acid encoding the donor nucleic acid molecule.

In an embodiment, the system or composition may cause double-strand breaks in the target nucleic acid.

In another embodiment, the system or composition may be such that a desired sequence is introduced in the target nucleic acid or a region adjacent thereto by homology-directed repair of double-strand breaks using the donor nucleic acid molecule as a template.

In yet another embodiment, the endonuclease comprising Cas12f1, TnpB, or a variant protein thereof; and the guide RNA may be included in a form of a ribonucleoprotein (RNP).

In still yet another embodiment, the system or composition may further comprise a molecule that inhibits expression of a gene involved in non-homologous end joining (NHEJ) or activity of a product expressed thereby.

According to another aspect of the present disclosure, there is provided a vector system comprising a first nucleic acid construct to which a nucleotide sequence encoding an endonuclease is operably linked, the endonuclease comprising Cas12f1, TnpB, or a variant protein thereof; a second nucleic acid construct to which a nucleotide sequence encoding an engineered guide RNA is operably linked, the engineered guide RNA comprising a guide sequence that binds complementarily to a target nucleic acid; and a third nucleic acid construct comprising a donor nucleic acid molecule.

In an embodiment, the nucleic acid constructs included in the vector system may be located in the same or different vectors.

In another embodiment, the vector system may further comprise at least one nucleic acid construct to which a nucleotide sequence encoding a molecule is operably linked, the molecule inhibiting expression of a gene involved in non-homologous end joining (NHEJ).

In yet another embodiment, the vector system may be such that respective components in the vector are included in one vector.

In still yet another embodiment, the vector may further comprise a promoter or an enhancer.

In still yet another embodiment, the promoter may be U6 promoter, EFS promoter, EF1-α promoter, H1 promoter, 7SK promoter, CMV promoter, LTR promoter, Ad MLP promoter, HSV promoter, SV40 promoter, CBA promoter, or RSV promoter.

In still yet another embodiment, the vector may be at least one viral vector selected from the group consisting of a retroviral (retrovirus) vector, a lentiviral (lentivirus) vector, an adenoviral (adenovirus vector), an adeno-associated viral (adeno-associated virus; AAV) vector, a vaccinia viral (vaccinia virus) vector, a poxviral (poxvirus) vector, a herpes simplex viral (herpes simplex virus) vector, and a phagemid vector.

In still yet another embodiment, the vector is an adeno-associated viral vector, and the adeno-associated viral vector may be characterized in that all components within the vector can be included in one vector.

In still yet another embodiment, the vector may be at least one non-viral vector selected from the group consisting of plasmid, naked DNA, DNA complex, mRNA (transcript), and PCR amplicon.

In still yet another embodiment, the plasmid may be at least one selected from the group consisting of pcDNA series, pSC101, pGV1106, pACYC177, ColE1, pKT230, pME290, pBR322, pUC8/9, pUC6, pBD9, pHC79, pIJ61, pLAFR1, pHV14, pGEX series, pET series, and pUC19, but is not limited thereto.

According to yet another aspect of the present disclosure, there are provided a virus or virus particle produced by the vector system and a composition comprising the virus or virus particle.

In an embodiment, the virus may be selected from the group consisting of retrovirus, lentivirus, adenovirus, adeno-associated virus, vaccinia virus, poxvirus, herpes simplex virus, and phage.

According to still yet another aspect of the present disclosure, there is provided a method for introducing a desired sequence into a target region on a double-stranded nucleic acid in a cell, comprising bringing, into contact with the cell, the system, the composition, or the vector system, or expressing the same in the cell; and allowing the desired sequence to be introduced in a target nucleic acid or a region adjacent thereto by repair of double-strand breaks using the donor nucleic acid molecule as a template.

In an embodiment, the repair of double-strand breaks may be by homology-directed repair mechanism.

In another embodiment, the cell may be a prokaryotic cell or eukaryotic cell in which a target nucleic acid or target gene is present.

In yet another embodiment, the eukaryotic cell may be a yeast, an insect cell, a plant cell, a non-human-animal cell, or a human cell.

In still yet another embodiment, the bringing-into-contact or the expressing may occur *in vivo* or *ex vivo.*

In still yet another embodiment, the vector system may be introduced into a packaging virus selected from the group consisting of retrovirus, lentivirus, adenovirus, adeno-associated virus, vaccinia virus, poxvirus, herpes simplex virus, and phage, and may be delivered into a prokaryotic cell or eukaryotic cell in a form of a virus produced by the packaging virus.

In still yet another embodiment, the vector system may be delivered into a prokaryotic cell or eukaryotic cell by electroporation, gene gun, sonoporation, magnetofection, transient cell compression or squeezing, cationic liposome method, lithium acetate-DMSO, lipid-mediated transfection, calcium phosphate precipitation, lipofection, polyethyleneimine (PEI)-mediated transfection, DEAE-dextran mediated transfection, or nanoparticle-mediated nucleic acid delivery.

In still yet another embodiment, the vector system may be delivered directly into a prokaryotic cell or eukaryotic cell through at least one lipid nanoparticle (LNP).

Hereinafter, embodiments commonly applied to each of the system, the composition, the vector system, and the method according to the plurality of aspects of the present disclosure will be described.

In an embodiment, the Cas12f1, TnpB, or the variant protein thereof may comprise an amino acid sequence having at least 70% sequence identity with an amino acid sequence selected from the group consisting of SEQ ID NOS: 1 to 5.

In another embodiment, the TnpB protein may comprise an amino acid sequence having at least 70% sequence identity with an amino acid sequence selected from the group consisting of SEQ ID NOS: 202 to 293.

In yet another embodiment, the Cas12f1, TnpB, or the variant protein thereof may comprise one selected from the following sequences: (i) the amino acid sequence of SEQ ID NO: 5; (ii) the amino acid sequence of SEQ ID NO: 1; (iii) an amino acid sequence having the amino acid sequence of SEQ ID NO: 1 in which 1 to 28 amino acids at the N-terminus have been removed or substituted; or (iv) an amino acid sequence having the amino acid sequence of SEQ ID NO: 1 in which 1 to 600 amino acids have been added to the N-terminus or C-terminus.

In still yet another embodiment, the added 1 to 600 amino acids may be the amino acid sequence of SEQ ID NO: 294 or 295.

In still yet another embodiment, the Cas12f1, TnpB, or the variant protein thereof may have at least 70% sequence identity with an amino acid sequence selected from the group consisting of SEQ ID NOS: 1 to 4.

In still yet another embodiment, the Cas12f1, TnpB, or the variant protein thereof may have at least 70% sequence identity with the amino acid sequence of SEQ ID NO: 1.

In still yet another embodiment, the Cas12f1, TnpB, or the variant protein thereof may have at least 70% sequence identity with the amino acid sequence of SEQ ID NO: 5.

In still yet another embodiment, the engineered guide RNA may comprise substitution, deletion, insertion, or addition of one or more nucleotides in reference with a wild-type Cas12f1 guide RNA sequence, and the engineered guide RNA, excluding the guide sequence, may have at least 50% sequence identity with the wild-type Cas12f1 guide RNA.

In still yet another embodiment, the wild-type Cas12f1 guide RNA may comprise trans-activating CRISPR RNA (tracrRNA) and CRISPR RNA (crRNA) which comprise (i) one or more stem regions, (ii) a tracrRNA-crRNA complementarity region, and optionally (iii) a region containing three or more consecutive uracil (U) residues, and the engineered guide RNA may comprise at least one modification selected from the group consisting of (a) to (d): (a) deletion of at least a part of one or more stem regions; (b) deletion of at least a part of the tracrRNA-crRNA complementarity region; (c) replacement of one or more uracil (U) residues with A, G, or C in three or more consecutive U residues when the consecutive U residues are present in the tracrRNA-crRNA complementarity region; and (d) addition of one or more uridine residues to the 3'-end of the crRNA sequence.

In still yet another embodiment, the wild-type Cas12f1 guide RNA may comprise tracrRNA consisting of the nucleotide sequence of SEQ ID NO: 11 and crRNA consisting of the nucleotide sequence of SEQ ID NO: 12.

In still yet another embodiment, the wild-type Cas12f1 guide RNA may comprise tracrRNA and crRNA which sequentially comprise, from the 5'-end, a first stem region, a second stem region, a third stem region, a fourth stem region, and the tracrRNA-crRNA complementarity region, and the engineered guide RNA may comprise at least one modification selected from the group consisting of (a1) deletion of at least a part of the first stem region; (a2) deletion of at least a part of the second stem region; (b) deletion of at least a part of the tracrRNA-crRNA complementarity region; (c) replacement of one or more uracil (U) residues with A, G, or C in three or more consecutive U residues when the consecutive U residues are present in the tracrRNA-crRNA complementarity region; and (d1) addition of a U-rich tail to the 3'-end of the crRNA sequence (in which a sequence of the U-rich tail is represented by 5'-(UₘV)ₙUₒ-3', where V is each independently A, C, or G, m and o are integers between 1 and 20, and n is an integer between 0 and 5).

In still yet another embodiment, the engineered guide RNA may comprise (d1) addition of a U-rich tail to the 3'-end of the crRNA sequence, (c) replacement of one or more uracil (U) residues with A, G, or C in three or more consecutive U residues when the consecutive U residues are present in the tracrRNA-crRNA complementarity region, or both modifications.

In still yet another embodiment, the engineered guide RNA may comprise at least one modification selected from the group consisting of (a1) deletion of at least a part of the first stem region; (a2) deletion of at least a part of the second stem region; and (b) deletion of at least a part of the tracrRNA-crRNA complementarity region.

In still yet another embodiment, the engineered guide RNA may comprise (b 1) deletion of a part of the tracrRNA-crRNA complementarity region. Specifically, the part of the complementarity region may consist of 1 to 54 nucleotides.

In still yet another embodiment, the engineered guide RNA may comprise (b2) deletion of the entire tracrRNA-crRNA complementarity region. Specifically, the entire complementary region may consist of 55 nucleotides.

In still yet another embodiment, the engineered guide RNA (a1) may comprise (a1) deletion of at least a part of the first stem region. Specifically, the at least a part of the stem region may consists of 1 to 20 nucleotides.

In still yet another embodiment, the engineered guide RNA may comprise (a2) deletion of at least a part of the second stem region. Specifically, the at least a part of the stem region may consist of 1 to 27 nucleotides.

In still yet another embodiment, the engineered guide RNAmay comprise (a1) deletion of at least a part of the first stem region; (d1) addition of a U-rich tail to the 3'-end of the crRNA sequence; or both modifications.

In still yet another embodiment, the engineered guide RNA may consist of a sequence represented by Formula (I) or may have at least 80%, at least 85%, at least 90%, or at least 95% sequence identity therewith. Here, the sequence identity may be based on a sequence that comprises or does not comprise at least one of X^{a}, X^{b1}, X^{b2}, X^{c1}, and X^{c2}. X^{g} and (UₘV)ₙUₒ may be excluded in a case of calculating the sequence identity.

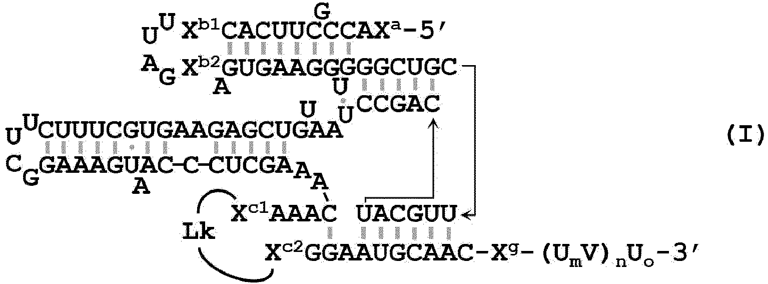

In Formula (I), X^{a}, X^{b1}, X^{b2}, X^{c1}, and X^{c2} each independently consists of 0 to 35 (poly)nucleotides, X^{g} is a guide sequence that consists of 10 to 30 nucleotides and is hybridizable with or complementary to a target sequence, Lk is a polynucleotide linker of 2 to 20 nucleotides or is absent, and (UₘV)ₙUₒ is present as a U-rich tail or absent, and when (UₘV)ₙUₒ is present, U is uridine, V is each independently A, C, or G, m and o are each independently an integer between 1 and 20, and n is an integer between 0 and 5.

In still yet another embodiment, X^{a} may comprise the nucleotide sequence of SEQ ID NO: 14 or a nucleotide sequence having the sequence of SEQ ID NO: 14 from which 1 to 20 nucleotides are deleted.

In still yet another embodiment, X^{b1} may comprise the nucleotide sequence of SEQ ID NO: 25 or a nucleotide sequence having the sequence of SEQ ID NO: 25 from which 1 to 13 nucleotides are deleted.

In still yet another embodiment, X^{b2} may comprise the nucleotide sequence of SEQ ID NO: 29 or a nucleotide sequence having the sequence of SEQ ID NO: 29 from which 1 to 14 nucleotides are deleted.

In still yet another embodiment, the sequence 5'-X^{b1}UUAGX^{b2}-3' in Formula (I) may be any one nucleotide sequence selected from the group consisting of SEQ ID NOS: 34 to 38.

In still yet another embodiment, X^{c1} may comprise the nucleotide sequence of SEQ ID NO: 39 or a nucleotide sequence having the sequence of SEQ ID NO: 39 from which 1 to 28 nucleotides are deleted.

In still yet another embodiment, in a case where three or more consecutive uracil (U) residues are present in a sequence of X^{c1}, the sequence of X^{c1} may comprise a modification in which at least one uracil residue thereof is replaced with A, G, or C.

In still yet another embodiment, X^{c2} may comprise the nucleotide sequence of SEQ ID NO: 58 or a nucleotide sequence having the sequence of SEQ ID NO: 58 from which 1 to 27 nucleotides are deleted.

In still yet another embodiment, in a case where the sequence 5'-ACGAA-3' is present in X^{c2}, the sequence may be replaced with 5'-NGNNN-3', and N may be each independently A, C, G, or U.

In still yet another embodiment, the sequence 5'-X^{c1}-Lk-X^{c2}-3' in Formula (I) may be any one nucleotide sequence selected from the group consisting of SEQ ID NOS: 80 to 86.

In still yet another embodiment, Lk may comprise any one nucleotide sequence selected from the group consisting of 5'-GAAA-3', 5'-UUAG-3', 5'-UGAAAA-3', 5'-UUGAAAAA-3', 5'-UUCGAAAGAA-3' (SEQ ID NO: 76), 5'-UUCAGAAAUGAA-3' (SEQ ID NO: 77), 5'-UUCAUGAAAAUGAA-3' (SEQ ID NO: 78), and 5'-UUCAUUGAAAAAUGAA-3' (SEQ ID NO: 79).

In still yet another embodiment, (UₘV)ₙUₒ may be such that (i) n is 0 and o is an integer between 1 and 6, or (ii) V is A or G, m and o are each independently an integer between 3 and 6, and n is an integer between 1 and 3.

In still yet another embodiment, the engineered guide RNA may comprise an engineered tracrRNA consisting of any one nucleotide sequence selected from the group consisting of SEQ ID NOS: 87 to 132.

In still yet another embodiment, the engineered guide RNA may comprise an engineered crRNA consisting of any one nucleotide sequence selected from the group consisting of SEQ ID NOS: 133 to 148.

In still yet another embodiment, the engineered guide RNA may be a dual guide RNA or a single guide RNA.

In still yet another embodiment, the engineered single guide RNA may consist of any one nucleotide sequence selected from the group consisting of SEQ ID NOS: 149 to 186.

In still yet another embodiment, the donor nucleic acid molecule may be a sequence used as a template in homology-directed repair and may have a length of 1 bp to 20 kb.

In still yet another embodiment, the gene involved in non-homologous end joining may include at least one selected from the group consisting of ATM1, XRCC4, XLF, XRCC6, LIG4, and DCLRE1C, but is not limited thereto.

In still yet another embodiment, the molecule that inhibits expression of a gene involved in non-homologous end joining may be shRNA, siRNA, miRNA, or antisense oligonucleotide, but is not limited thereto.

### Advantageous Effects of Invention

The present disclosure provides a target nucleic acid editing system, comprising an endonuclease based on Cas12f1 or novel TnpB, which was not previously known as an endonuclease, or a variant protein thereof, and an engineered guide RNAthat exhibits excellent homology-directed repair efficiency when used with the endonuclease. The Cas12f1, TnpB, or the variant protein thereof included in the target nucleic acid editing system of the present disclosure tends to cleave the rear (outer) portion of the target nucleic acid, and thus has much higher homology-directed repair efficiency than other CRISPR/Cas systems. In addition, the target nucleic acid editing system of the present disclosure uses a hypercompact endonuclease and an engineered guide RNA that is shorter in length and exhibits excellent editing efficiency. Thus, even in a case of using a delivery vehicle with a very limited packaging size, such as AAV, this editing system allows a single vector to contain various tools necessary for homology-directed repair, including a donor nucleic acid, and therefore has an advantage of enabling construction of a system for various types of gene editing, including homology-directed repair. In particular, such a system allows addition of a molecule that inhibits non-homologous end joining (NHEJ), such as shRNA, thereby having an effect of maximizing homology-directed repair efficiency.

### Brief Description of Drawings

FIG. 1 illustrates each modification site (MS) in the engineered guide RNA of the target nucleic acid editing system (TaRGET system) according to an embodiment.
FIG. 2 illustrates a structure of the donor nucleic acid for comparing homology-directed repair efficiency of Cas9, Cas12a, and TnpB (TaRGET).
FIG. 3 graphically illustrates results obtained by comparing homology-directed repair efficiency of Cas9, Cas12a, and TaRGET (Cas12f and TnpB).
FIG. 4 graphically illustrates homology-directed repair efficiency of the target nucleic acid editing system depending on the length of the donor nucleic acid.
FIG. 5 graphically illustrates results obtained by introducing a construct for homology-directed repair into HEK293T cells, and then measuring, over time, rates of homology-directed repair relative to non-homologous end joining by the Cas9 system and the target nucleic acid editing system (TaRGET system).
FIG. 6 graphically illustrates rates of homology-directed repair relative to non-homologous end joining by Cas9, Cas12a, and TaRGET (Cas 12f and TnpB) for the target genes NLRC4, FUS, and LOC105370393.
FIG. 7 graphically illustrates changes in homology-directed repair efficiency of the target nucleic acid editing system (TaRGET system) in a case where expression of various genes involved in non-homologous end joining or homology-directed repair is inhibited.
FIG. 8 graphically illustrates efficiency of non-homologous end joining and homology-directed repair by the target nucleic acid editing system (TaRGET system) in *DCLRE1C-knockout* HEK293T cells and wild-type HEK293T cells.
FIG. 9 graphically illustrates rates of homology-directed repair relative to non-homologous end joining by Cas9, Cas12a, and the target nucleic acid editing system (TaRGET system), respectively, in *DCLRE1C-knockout* HEK293T cells and wild-type HEK293T cells.

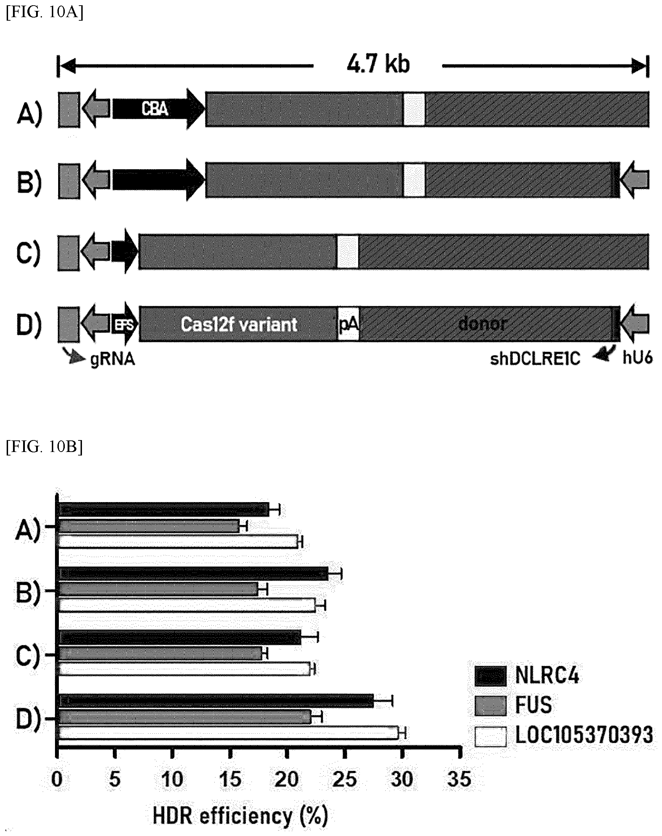

FIGS. 10A and 10B illustrate results obtained by comparing homology-directed repair efficiency by varying respective components of the construct for homology-directed repair which has been constructed to a size of 4.7 kb or smaller so that it can be packaged in AAV, according to an embodiment: FIG. 10A illustrates four types of vectors, each of which has been differently constructed depending on type of promoter, length of donor nucleic acid, and presence or absence of shDCLRE1C; and FIG. 10B graphically illustrates results obtained by investigating homology-directed repair efficiency of the four types of vectors for the target genes NLRC4, FUS, and LOC105370393.

### Modes for Carrying out Invention

The detailed description of the present disclosure set forth below will be described with reference to specific drawings with respect to specific embodiments in which the present disclosure may be practiced; however, the present disclosure is not limited thereto and, if properly described, is limited only by the appended claims, along with the full scope of equivalents to which such claims are entitled. It should be understood that various embodiments/examples of the present disclosure, although different, are not necessarily mutually exclusive. For example, a particular feature, structure, or characteristic described herein may be changed from one embodiment/example to another embodiment/example or implemented in combinations of embodiments/examples without departing from the technical spirit and scope of the present disclosure. Unless defined otherwise, technical and scientific terms used herein have the same meaning as generally used in the art to which the present disclosure belongs. For purposes of interpreting this specification, the following definitions will apply and whenever appropriate, terms used in the singular will also include the plural and vice versa.

### I. Definition

As used herein, the terms "target nucleic acid editing system", "gene editing system", "gene repair system", or "homology-directed repair system" refer to a system that comprises a nucleic acid degrading enzyme, such as nucleic acid editing protein or endonuclease, a nucleic acid-targeting molecule corresponding to the nucleic acid degrading enzyme, and a donor nucleic acid used as a template in gene repair mechanism, and this system binds to or interacts with a target nucleic acid or target gene so that a target region of the target nucleic acid or target gene can be cleaved, edited, repaired, and/or restored. Here, the nucleic acid-targeting molecule may be represented by an engineered guide RNA (gRNA), but is not limited thereto. Meanwhile, the target nucleic acid editing system may exist in any form capable of editing the target nucleic acid. For example, the system may be in a form of a composition that comprises a complex comprising a nucleic acid degrading enzyme and a nucleic acid-targeting molecule and a donor nucleic acid, may be in a form of a kit in which the complex and the donor nucleic acid are each included in separate compositions, or may be a vector system or composition comprising at least one vector that comprises a nucleic acid encoding a nucleic acid degrading enzyme, a nucleic acid encoding a nucleic acid-targeting molecule, and a nucleic acid encoding a donor nucleic acid.

The term "hypercompact TaRGET system" refers to a gene editing system that comprises a nucleic acid degrading enzyme such as hypercompact CRISPR/Cas protein or tiny endonuclease (for example, Cas12f1, TnpB, or a variant thereof) and a nucleic acid-targeting molecule corresponding to the nucleic acid degrading enzyme, and is used for differentiation from the existing gene editing system. Here, the nucleic acid-targeting molecule may be represented by an engineered guide RNA (gRNA), but is not limited thereto. The system may be any type of gene editing system capable of binding to a target nucleic acid or target gene so that a target region of the target nucleic acid or gene is cleaved, edited, repaired, and/or restored. The term "endonuclease" may be used interchangeably with "nucleic acid editing protein," "gene editing protein," "homology-directed repair protein," or "nucleic acid degrading protein," and the molecule referred to as such an endonuclease or protein refers to a (endo-) nuclease that recognizes the targeting nucleic acid, DNA or RNA, or a protospacer adjacent motif (PAM) present in a target gene, and then allows double-strand breaks (DSBs) to occur at nucleotide sequences within or outside the target nucleotide sequence. In addition, the endonuclease, the nucleic acid editing protein, or the like is also referred to as an effector protein that constitutes a nucleic acid editing system or a nucleic acid construct for homology-directed repair. Here, the effector protein may be a nucleic acid degrading protein capable of binding to a guide RNA (gRNA) or engineered gRNA, or may be a peptide fragment capable of binding to a target nucleic acid or target gene.

The term "guide RNA (gRNA)" refers to RNA that is capable of forming a complex with a molecule referred to as an endonuclease, a gene editing protein, a nucleic acid degrading protein, or the like, and interacting with (for example, hybridizing to, forming a complementary bond(s) with, or forming a hydrogen bond(s) with) a target nucleotide sequence, and comprises a guide sequence having sufficient complementarity with the target nucleotide sequence to cause sequence-specific binding of the complex to the target nucleotide sequence. In the present disclosure, a guide RNA and a guide molecule may be used interchangeably.

The terms "tracrRNA (trans-activating crRNA)" and "crRNA (CRISPR RNA)" include all meanings that can be recognized by those skilled in the art in the field of gene editing technology. These terms may be used to refer to respective molecules of a dual guide RNA found in nature, and may also be used to refer to respective portions of a single guide RNA (sgRNA) in which the tracrRNA and the crRNA are connected by a linker. Unless otherwise stated, the description tracrRNA and crRNA simply means tracrRNA and crRNA that constitute a guide RNA in a target nucleic acid editing system (or gene editing system, homology-directed repair system, or the like).

The term "scaffold region" refers collectively to a portion of a guide RNA (gRNA) which can interact with a molecule called endonuclease, homology-directed repair protein, gene editing protein, nucleic acid degrading protein, or the like, and may be used to refer to the remaining portion of a guide RNA found in nature, excluding a spacer.

The terms "guide sequence", "spacer," or "spacer sequence" may be used interchangeably, and refer to a polynucleotide within the CRISPR/Cas system which is capable of interacting with (for example, hybridizing to, forming a complementary bond(s) with, or forming a hydrogen bond(s) with) a target sequence portion. For example, the guide sequence or spacer sequence refers to 10 to 50 consecutive nucleotides linked directly or indirectly through a linker or the like to or near the 3'-end of crRNA, which constitutes a guide RNA, in a target nucleic acid editing system.

The term "engineered" may be used interchangeably with "non-naturally occurring", "artificial," or "modified", and means that something is not in its natural form or state as found in nature. In a case where the term indicates a guide RNA, a guide polynucleotide, or a nucleic acid molecule, the guide RNA, the guide polynucleotide, or the nucleic acid molecule is meant to be substantially free of at least one component that is found in nature or naturally occurring, or to substantially contain at least one component that is not found in nature or non-naturally occurring. For example, the "engineered guide RNA" refers to gRNA obtained by applying artificial modification to a configuration (for example, sequence) of a guide RNA (gRNA) that exists in nature, and may be referred to herein as an "augmented RNA."

The term "wild-type" is a term of the art understood by those skilled in the art and means a typical form of an organism, strain, gene, or characteristic as it occurs in nature to the extent that it is distinguishable from mutant or variant forms. The term "variant" should be understood to mean expression of qualities having a pattern that deviates from what occurs in nature. For example, in a case where Cas12f1, TnpB, or a variant protein thereof is mentioned, the variant protein may mean a variant of (wild-type) Cas12f1 or a variant of (wild-type) TnpB.

The term "donor nucleic acid molecule" may be interchangeably used with "donor DNA," "donor polynucleotide," "donor oligonucleotide," and "donor template," and refers to a nucleic acid or polynucleotide that provides a nucleotide sequence of which at least a portion is intended to be integrated into a target region of a selected target nucleic acid or target gene. Typically, the donor nucleic acid is a single-stranded polynucleotide or a double-stranded polynucleotide. For example, the target nucleic acid editing system of the present disclosure can comprise the donor nucleic acid so that a DNA target sequence in genomic DNA is modified and/or repaired, wherein the genomic DNA is modified and/or repaired such that the DNA target sequence comprises at least a portion of the donor nucleic acid.

The term "target nucleic acid" or "target gene" refers to a gene or nucleic acid that is an object to be cleaved, edited, repaired, and/or restored by, or targeted by a target nucleic acid editing system (for example, homology-directed repair system or TaRGET system). The target nucleic acid or the target gene may be used interchangeably and may refer to the same object. Unless otherwise specified, the target gene may be a unique gene or nucleic acid in a cell of interest, an externally derived gene or nucleic acid, or an artificially synthesized nucleic acid or gene, and may refer to single-stranded DNA, double-stranded DNA, and/or RNA. The target gene or target nucleic acid is not particularly limited as long as it can be an object to be cleaved, edited, repaired, or the like by the system according to the present disclosure.

The term "target region" or "target sequence" refers to a specific sequence present in or around a target nucleic acid or target gene, and this sequence is recognized by the hypercompact nucleic acid editing system of the present disclosure to cleave the target gene or nucleic acid. The target region or target sequence may be appropriately selected depending on the purpose.

The term "homology-directed repair (HDR)" is an intracellular mechanism that repairs double-stranded DNA breaks, lesions, or the like, and the most common type of homology-directed repair is homologous recombination. Homology-directed repair refers to one of the cellular mechanisms for repairing double-stranded DNA breaks, lesions, or the like in a case where homologous pieces of DNA are present in the nucleus, primarily in G2 and S phases of the cell cycle. Homology-directed repair uses a donor DNA, which programs repair, as a template, and may be used to create specific sequence changes, including intended addition of a gene, in the genome. In a case where a donated template is given with a site-specific nuclease, such as the system or TaRGET system of the present disclosure, cellular machinery will repair double-strand breaks by homologous recombination, and this mechanism is enhanced in the presence of DNA double breaks. In a case where a donor DNA is present, homology-directed repair and non-homologous end joining occur simultaneously in a competitive manner; and in a case where a donor DNA is absent, only non-homologous end joining occurs.

Unless otherwise specified, the term "vector" refers collectively to any substance capable of transporting a genetic material into a cell. For example, the vector may be, but is limited to, a DNA molecule that comprises a nucleic acid encoding an effector protein and/or a nucleic acid encoding a guide RNA (gRNA) in which the effector protein and the guide RNA are those in a target nucleic acid editing system that is a genetic material to be delivered. In addition, in the present disclosure, the "vector" may be an "expression vector" that comprises essential regulatory elements operably linked so that an inserted gene is normally expressed. The term "operably linked" means that, in gene expression techniques, a particular component is linked to another component so that the particular component can function as intended.

The terms "nucleotide" and "nucleic acid" may be used interchangeably and refer to a polymeric form of nucleotides of any length, either ribonucleotides or deoxynucleotides. Thus, this term includes, but is not limited to, single-, double-, or multi-stranded DNA or RNA, genomic DNA, cDNA, a DNA-RNA hybrid, or a polymer comprising purine and pyrimidine bases or other natural, chemically or biochemically modified, non-natural, or derivatized nucleotide bases. The terms "polynucleotide" and "nucleic acid" should be understood to include, as applicable to the embodiment being described herein, single-stranded (such as sense or antisense) and double-stranded polynucleotides.

The term "nucleic acid construct" refers to a structure that comprises, as components, a nucleotide sequence encoding an endonuclease, a nucleic acid editing protein, a nucleic acid degrading protein, or the like and/or a nucleotide sequence encoding a guide RNA, and if necessary, may further comprise nucleotide sequences encoding various types of (poly)peptides or linkers. The nucleic acid construct may be used as a component of the CRISPR/Cas system, vector system, or hypercompact TaRGET system for homology-directed repair of the present disclosure.

The terms "protein," "polypeptide," and "peptide" may be used interchangeably and refer to a polymeric form of amino acids of any length which can comprise genetically coded and non-genetically coded amino acids, chemically or biochemically modified or derivatized amino acids, and polypeptides having modified peptide backbones. The term includes fusion proteins, including, but not limited to, fusion proteins with a heterologous amino acid sequence, fusions with heterologous and homologous leader sequences, with or without N-terminal methionine residues; immunologically tagged proteins; and the like.

The terms "A, T, C, G, and U" may be properly interpreted as bases, nucleosides, or nucleotides on DNA or RNA depending on the context and description. For example, in a case where the terms mean bases, they may be interpreted as one selected from adenine (A), guanine (G), cytosine (C), thymine (T), and uracil (U), respectively. In a case where the terms mean nucleosides, they may be interpreted as adenosine (A), thymidine (T), cytidine (C), guanosine (G), or uridine (U), respectively; and in a case where the terms mean nucleotides in a sequence, they may be interpreted to mean nucleotides including their respective nucleosides.

The term "about" refers to an amount, level, value, number, frequency, percent, dimension, size, amount, weight or length that varies by approximately 30%, 25%, 20%, 15%, 10%, 9%, 8%, 7%, 6%, 5%, 4%, 3%, 2% or 1% with respect to a reference amount, level, value, number, frequency, percent, dimension, size, amount, weight or length. For example, in a case of being used in relation to a value x expressed as a number or numerical value, the term "about" may mean x ± 5%.

All technical terms used in the present disclosure, unless otherwise defined, include all meanings recognized by a person skilled in the art and are used in the same sense as generally understood, and can be interpreted appropriately depending on the context. In addition, although preferred methods or samples are described in this specification, those similar or equivalent thereto are also encompassed in the scope of the present disclosure.

### II. Target nucleic acid editing system for highly efficient homology-directed repair and composition comprising same

The present inventors have found that TnpB (transposon-associated transposase B) protein has an amino acid sequence similar to UnCas12f1 protein (accordingly, TnpB with an amino acid sequence similar to UnCas12f1 protein is also named CWCas12f1), is about 1/3 smaller in molecular weight than existing nucleic acid-degrading proteins, including Cas9 protein that has been studied the most to date, and has much higher nucleic acid cleavage efficiency for a target nucleic acid or target gene. In addition, the present inventors have demonstrated for the first time that Cas12f1, TnpB, or a variant protein thereof exhibits highly efficient gene editing protein activity and, in particular, induces homology-directed repair in the presence of a donor nucleic acid.

In addition, in order to cleave, edit, repair, and/or restore a target nucleic acid or target gene within a cell, the present inventors have constructed a system that can be easily loaded onto an adeno-associated virus (AAV) vector and effectively delivered *in vivo,* and this system is a novel hypercompact nucleic acid editing system for homology-directed repair, which comprises a miniature endonuclease comprising Cas12f1, TnpB, or a variant protein thereof, which is a hypercompact nucleic acid editing protein; an engineered guide RNA that exhibits high indel (insertion or deletion) efficiency with respect to the endonuclease; and a donor nucleic acid molecule.

The present inventors have found for the first time that use of the novel hypercompact nucleic acid cleaving protein, Cas12f1, TnpB, or a variant protein thereof, rather than the previously known Cas endonuclease such as Cas9 or Cas12a, enables gene editing, in particular, induction of homology-directed repair, with higher efficiency and a wide range of applications. The present inventors have incorporated all components into a single adeno-associated virus (AAV) vector to construct a novel hypercompact nucleic acid editing system that can be delivered in vivo while efficiently inducing homology-directed repair in various genes, thereby completing the present disclosure.

Therefore, the present disclosure relates to a hypercompact target nucleic acid editing system (or TaRGET system) for causing or inducing homology-directed repair to occur in a site (sequence)-specific and highly efficient manner in a target nucleic acid or target gene, and this system comprises an endonuclease comprising Cas12f1, TnpB, or a variant protein thereof, or a nucleic acid encoding the endonuclease; an engineered guide RNA comprising a guide sequence or a nucleic acid encoding the guide RNA; and a donor nucleic acid molecule or a nucleic acid encoding the donor nucleic acid molecule.

In addition, the present disclosure relates to a gene editing composition for a target nucleic acid, which comprises an endonuclease comprising Cas12f1, TnpB, or a variant protein thereof, or a nucleic acid encoding the endonuclease; an engineered guide RNA comprising a guide sequence or a nucleic acid encoding the guide RNA; and a donor nucleic acid molecule or a nucleic acid encoding the donor nucleic acid molecule, and a method for introducing a desired sequence into a target region of a target nucleic acid or target gene using the system or composition.

In addition, the present disclosure relates to other editing systems, other methods, compositions, vector systems, viruses, or viral compositions for implementing the present disclosure.

The target nucleic acid editing system for homology-directed repair according to the present disclosure is a meaningful result in that the system solves the limitation of most previously studied Cas endonucleases and gene editing systems comprising the same being unable to be loaded onto an adeno-associated virus (AAV) vector that has been approved by the FDA as an intracellular delivery vehicle, due to their size.

Furthermore, even in a case of comprising a donor nucleic acid molecule of sufficient size required to induce highly efficient homology-directed repair, the target nucleic acid editing system can still be produced at a size of about 4.7 kb, which is the packaging limit of the AAV vector, and enables introduction of a desired sequence into a desired target region by cleaving a double-stranded target region and inducing homology-directed repair in a target nucleic acid or target gene. Thus, the target nucleic acid editing system according to the present disclosure can constitute a system for various types of gene editing, including homology-directed repair, in a target nucleic acid or target gene, and can also be widely applied as a new therapy for gene-related diseases.

Hereinafter, respective components of the target nucleic acid editing system/composition provided in the present disclosure and production methods thereof will be described in detail.

### 1. Cas12f1, TnpB, and variant protein thereof

According to an aspect of the present disclosure, there is provided a (miniature) endonuclease comprising Cas12f1, TnpB, or a variant protein thereof, characterized in that it exhibits excellent activity in inducing homology-directed repair by cleaving a target region in a target nucleic acid and has a significantly small size of nucleic acid degrading protein, which is approximately 1/3, as compared with the existing CRISPR/Cas9 system.

The Cas12f1, TnpB, or the variant protein thereof, which is an endonuclease or nucleic acid editing protein included in the target nucleic acid editing system of the present disclosure, includes all of Cas12f1, Cas12f1 variants, TnpB, and TnpB variants, which are found in nature, or engineered Cas12f1 or engineered TnpB. The endonuclease or nucleic acid editing protein may be, but is not limited to, Cas12f1, TnpB, or a modified nucleic acid editing protein thereof, for example, a protein obtained by deletion, addition, or substitution of one or more amino acids from the protein, a dead nucleic acid editing protein, or a nick nucleic acid editing protein.

The "Cas12f1 protein," which is a component of the present disclosure, is one of the effector proteins named Cas14 in Harrington et al., Science, 362, 839-842 (2018), and is also called Cas14a1 protein. The Cas12f1 protein may be a wild-type Cas12f1 protein (wild-type Cas14a1 protein) that exists in nature. Alternatively, the Cas12f1 protein may be a variant of the wild-type Cas12f1 protein, in which the variant is referred to as "Cas12f1 variant" or "Cas14a1 variant." The Cas12f1 variant may be a variant having the same function as the wild-type Cas12f1 protein, a variant of which some or all functions are modified as compared with the wild-type Cas12f1 protein, and/or a variant with additional functions as compared with the wild-type Cas12f1 protein.

In some embodiments, the Cas12f1, TnpB, or the variant protein thereof may comprise an amino acid sequence having at least 70% sequence identity with an amino acid sequence selected from the group consisting of SEQ ID NOS: 1 to 5.

In some embodiments, the TnpB or the variant protein thereof may be or comprise a sequence derived from the transposase accessory protein TnpB of the IS200/IS605 family which is similar in size to the Cas12f1 protein that belongs to the V-F subtype among Class 2, Type V CRISPR/nuclease proteins. The TnpB protein is a protein conventionally known as a transposase. To date, the TnpB protein is only known as a transposon-encoded nuclease, and it is not known whether the TnpB protein has Cas endonuclease activity. In addition, no guide RNA is known for the TnpB protein. The present disclosure has been completed, in part, by first discovering that a TnpB variant or engineered TnpB, which is based on the TnpB protein sequence, is similar in size to the Cas12f1 protein, which belongs to the group with the smallest molecular weight among nucleic acid degrading proteins, and has excellent endonuclease activity that targets a target nucleic acid or target gene and cleaves double-stranded DNA at a target region, and constructing an engineered guide RNA that exhibits excellent homology-directed repair induction efficiency when used together with TnpB or a variant protein thereof. TnpB or a variant protein thereof belongs to the group with the smallest molecular weight among the existing nucleic acid-degrading proteins, and has a great advantage in constructing a hypercompact nucleic acid editing system for introducing a desired sequence into a target gene in a cell from the viewpoint that it has an excellent effect of forming a complex with the engineered short guide RNA (gRNA) of the present disclosure to target a target nucleic acid or target gene and cleave a double strand, and is capable of inducing highly efficient homology-directed repair when a donor nucleic acid molecule is also present. In addition, unlike Cas9, which has 5'-NGG-3' as a PAM, the TnpB or the variant protein thereof has a T-rich PAM such as 5'-TTTA-3' or 5'-TTTG-3' as a PAM, which allows for selection of sequences rich in thymine (T) as target nucleic acids or target genes, thereby expanding the range of nucleic acid-degrading proteins available for genome editing.

In some embodiments, the Cas12f1, TnpB, or the variant protein thereof may comprise one selected from the following sequences: (i) the amino acid sequence of SEQ ID NO: 5; (ii) the amino acid sequence of SEQ ID NO: 1; (iii) an amino acid sequence having the amino acid sequence of SEQ ID NO: 1 in which 1 to 28 amino acids at the N-terminus have been removed or substituted; or (iv) an amino acid sequence having the amino acid sequence of SEQ ID NO: 1 in which 1 to 600 amino acids have been added to the N-terminus or C-terminus.

Specifically, according to an embodiment, the Cas12f1 or the variant protein thereof may be a protein that comprises or consists of the amino acid sequence of SEQ ID NO: 5, or a variant protein that comprises or consists of an amino acid sequence having at least 70% sequence identity with the amino acid sequence of SEQ ID NO: 5.

According to another embodiment, the TnpB or the variant protein thereof may be a protein that comprises or consists of the amino acid sequence of SEQ ID NO: 1, or a variant protein that comprises or consists of an amino acid sequence having at least 70% sequence identity with the amino acid sequence of SEQ ID NO: 1. In addition, the TnpB variant protein may be a TnpB variant protein that comprises or consists of an amino acid sequence having the amino acid sequence of SEQ ID NO: 1 in which 1 to 28 amino acids at the N-terminus have been removed or substituted. Here, the TnpB variant protein does not comprise the Cas12f1 protein consisting of the amino acid sequence of SEQ ID NO: 5. Specifically, the TnpB or the variant protein thereof may be a protein having at least 70% sequence identity with an amino acid sequence selected from the group consisting of SEQ ID NOS: 1 to 4. For example, the TnpB variant protein comprises a protein having at least 70% sequence identity with the amino acid sequence of SEQ ID NO: 1.

In yet another embodiment, the Cas12f1 variant protein may be a Cas12f1 protein that consists of the amino acid sequence of SEQ ID NO: 5 and further comprises one or more amino acids. In some examples, the Cas12f1 variant protein comprises a TnpB variant protein. For example, the Cas12f1 variant protein may comprise or consist of TnpB-v1 protein (SEQ ID NO: 2) that comprises N-terminal 26 aa of CasX at the N-terminus of the Cas12f1 protein, TnpB-v2 protein (SEQ ID NO: 3) that comprises a 28 aa random sequence at the N-terminus of the Cas12f1 protein, or TnpB-v3 protein (SEQ ID NO: 4) that comprises a 26 aa random sequence at the N-terminus of the Cas12f1 protein.

In some embodiments, the TnpB or the variant protein thereof may be or comprise a TnpB protein derived from another biological species or a variant derived therefrom. In other words, the TnpB protein includes TnpB homolog proteins that do not show significant sequence identity and perform the same function in other organisms. Specifically, the TnpB protein or the variant protein thereof may comprise or consist of any one amino acid sequence selected from the group consisting of SEQ ID NOS: 202 to 293. The TnpB protein (or TnpB homolog protein) refers to a protein that shares the same *in vivo* activity (that is, endonuclease activity) as the TnpB protein and has preserved, without loss, the characteristics derived from a common ancestor, regardless of their sequence similarity (or identity).

In still yet another embodiment, the TnpB or the variant protein thereof may be a protein comprising or consisting of one of amino acid sequences, each of which has the amino acid sequence of SEQ ID NO: 1 in which 1 to 600 amino acids have been added to the N-terminus or C-terminus. Here, there is no limitation on a sequence of the added 1 to 600 amino acids. As an example, the added 1 to 600 amino acids may be the amino acid sequence of SEQ ID NO: 294 or 295. An NLS or NES sequence may be further included between the added sequence and the TnpB variant protein.

In addition, the Cas12f1, TnpB, or the variant protein thereof may have a function similar to that of the wild-type Cas12f1 protein, or may have an altered function as compared with the wild-type Cas12f1 protein. More specifically, the alteration includes modification to all or part of the function, loss of all or part of the function, and/or addition of an additional function. The Cas12f1, TnpB, or the variant protein thereof may include any alteration without particular limitation, as long as it is an alteration that can be applied to a nucleic acid degrading protein of a hypercompact nucleic acid editing system by a person skilled in the art. For example, the Cas12f1 variant protein, or the TnpB or the variant protein thereof not only has activity of cleaving a DNA double strand, but also has activity of cleaving a single-stranded DNA or RNA, or a hybrid double strand of DNA and RNA, which may be for performing base editing and/or prime editing.

In some embodiments, the target nucleic acid editing system of the present disclosure cleaves a nucleic acid at a target region of a target nucleic acid or target gene, and thus may be characterized in that the target region is located in the nucleus of a cell. Therefore, the Cas12f1, TnpB, or the variant protein thereof used in the target nucleic acid editing system of the present disclosure may comprise one or two or more nuclear localization signal (NLS) sequences that localize the same into the nucleus. For example, one or more nuclear localization signal sequences may have a sufficient amount or intensity of activity to induce the Cas12f1, TnpB, or the variant protein thereof to be targeted to the nucleus in a detectable amount in the nucleus of a eukaryotic cell (including a mammalian cell). For example, differences in the intensity of activity may result from the number of NLS included in the Cas12f1, TnpB, or the variant protein thereof, the type of specific NLS('s) used, or a combination of these factors.

In still yet another embodiment, for the NLS included in the Cas12f1, TnpB, or the variant protein thereof, about 1, 2, 3, 4, 5, 6, 7, 8, 9 or 10 or more NLS's at or near the N-terminus, about 1, 2, 3, 4, 5, 6, 7, 8, 9 or 10 or more NLS's at or near the C-terminus, or combinations thereof may be variously selected. For example, the the Cas12f1, TnpB, or the variant protein thereof may comprise 0 or at least one NLS sequence at the N-terminus and/or 0 or at least one NLS sequence at the C-terminus. In a case where more than one NLS sequence is present, each NLS sequence may be selected independently of the others such that a single NLS may be present in more than one replicate and in combination with more than one other NLS present in more than one replicate.

In some embodiments, the NLS sequence is heterologous to the protein, exemplified by, but not limited to, the following NLS sequence. For example, the NLS may be NLS of an SV40 virus large T-antigen having the amino acid sequence 'PKKKRKV,' nucleoplasmin bipartite NLS having the sequence 'KRPAATKKAGQAKKKK,' which is an NLS sequence from a nucleoplasmin, or c-myc NLS having the amino acid sequence 'PAAKRVKLD' or 'RQRRNELKRSP.' In addition, the NLS may be an NLS sequence derived from the hRNPA1 M9 NLS sequence, the NLS sequence of the IBB domain from importin-alpha, the NLS sequence of the myoma T protein, the NLS sequence of human p53, the NLS sequence of mouse c-abl IV, the NLS sequence of influenza virus NS1, the NLS sequence of the hepatitis virus delta antigen, the NLS sequence of the mouse Mx1 protein, the NLS sequence of human poly(ADP-ribose) polymerase, or the NLS sequence of the steroid hormone receptor (human) glucocorticoid.

In addition, the Cas12f1, TnpB, or the variant protein thereof may be a fusion of various enzymes that may be involved in a gene expression process within cells. Here, the Cas12f1, TnpB, or the variant protein thereof to which the enzymes are fused may cause various quantitative and/or qualitative changes in gene expression in cells. For example, the various enzymes to be additionally bound may be DNMT, TET, KRAB, DHAC, LSD, p300, Moloney Murine Leukemia Virus (M-MLV) reverse transcriptase, or variants thereof. Here, the Cas12f1, TnpB, or the variant protein thereof to which the reverse transcriptase is fused may also function as a prime editor.

In some embodiments, the Cas12f1, TnpB, or the variant protein thereof may cause double-strand breaks in a target nucleic acid or target gene, and the double-strand breaks may be caused by cleavage of an outer portion of the target nucleic acid by the Cas12f1, TnpB, or the variant protein thereof. As described above, in a case where double-strand breaks occur in a target nucleic acid or target gene by the Cas12f1, TnpB, or the variant protein thereof, repair of the double-strand breaks may proceed with a donor nucleic acid molecule as a template by an intracellular mechanism. Such repair of the double-strand breaks allows a desired sequence to be introduced into the target nucleic acid or target gene.

### 2. PAM sequences of Cas12f1, TnpB, and variant protein thereof

In some embodiments, the following two conditions are required for the hypercompact nucleic acid editing system to be located at a target region in a target nucleic acid or target gene and to accurately cleave a nucleic acid at the target region.

First, there must be a nucleotide sequence of a certain length within the target nucleic acid or target gene which can be recognized by Cas12f1, TnpB, or a variant protein thereof. In addition, around the nucleotide sequence of a certain length, there must be a sequence that can bind complementarily to a guide sequence (spacer) included in a guide RNA (gRNA) for Cas12f1, TnpB, or a variant protein thereof. In other words, in a case where Cas12f1, TnpB, or a variant protein thereof recognizes the nucleotide sequence of a certain length, and a spacer sequence portion included in a guide RNA (gRNA) binds complementarily to a sequence portion around the nucleotide sequence of a certain length, it is possible to accurately cleave, edit, and/or repair a nucleic acid at a target region in a target nucleic acid or target gene. Here, the nucleotide sequence of a certain length recognized by Cas12f1, TnpB, or a variant protein thereof is called a protospacer adjacent motif (PAM) sequence. The PAM sequence is a unique sequence determined by the hypercompact gene editing protein Cas12f1, TnpB, or a variant protein thereof. This means that in a case of determining a target sequence of a complex of Cas12f1, TnpB, or a variant protein thereof with gRNA in a nucleic acid editing system, the target sequence must be determined within sequences adjacent to the PAM sequence.

The PAM sequence of Cas12f1, TnpB, or a variant protein thereof may be a T-rich sequence. More specifically, the PAM sequence of Cas12f1, TnpB, or a variant protein thereof may be 5'-TTTN-3'. Here, N is one of deoxythymidine (T), deoxyadenosine (A), deoxycytidine (C), or deoxyguanosine (G).

In some embodiments, the PAM sequence of Cas12f1, TnpB, or a variant protein thereof may be 5'-TTTA-3', 5'-TTTT-3', 5'-TTTC-3', or 5'-TTTG-3'. Preferably, the PAM sequence of Cas12f1, TnpB, or a variant protein thereof may be 5'-TTTA-3' or 5'-TTTG-3'.

In another embodiment, the PAM sequence ofCas12f1, TnpB, or a variant protein thereof may be different from the PAM sequence of a wild-type Cas12f1 protein.

### 3. Guide RNA for Cas12f1, TnpB, or variant protein thereof for homology-directed repair

### (1) Overview

Embodiments of the present disclosure were derived to overcome limitations of intracellular delivery using adeno-associated virus (AAV) which are caused by the conventional Cas9 system having a large protein molecular weight. Therefore, in addition to selecting Cas12f1, TnpB, or a variant protein thereof with a small molecular weight as a protein having cleavage activity for homology-directed repair included in the target nucleic acid editing system of the present disclosure, an engineered guide RNA (augment RNA) was constructed which achieves size minimization by artificial engineering that causes the guide RNA (gRNA) for the Cas12f1, TnpB, or the variant protein thereof to be much shorter than those found in nature, and, at the same time, exhibits increased efficiency of cleavage and/or homology-directed repair for a target.

Since no gRNA has been found in nature for TnpB or a variant thereof, which is the hypercompact endonuclease according to an embodiment of the present disclosure, an attempt was made to construct an optimal gRNA that exhibits highly efficient targeting and editing activity for the TnpB or the variant protein thereof. From this viewpoint, the gRNA that exists in nature for TnpB or a variant protein thereof may be a wild-type gRNA found in nature for Cas12f1 that is similar in size to TnpB or a variant protein thereof. That is, in the present disclosure, a "wild-type" gRNAfor Cas12f1, TnpB, or a variant protein thereof is used to mean "basic" or "canonical" gRNA. The wild-type gRNA may comprise tracrRNA (trans-activating CRISPR RNA) and crRNA (CRISPR RNA) which comprise (i) one or more stem regions, (ii) a tracrRNA-crRNA complementarity region, and optionally, (iii) a region containing three or more consecutive uracil (U) residues. Specifically, the wild-type gRNA may comprise tracrRNA and crRNA which sequentially comprise, from the 5'-end, a first stem region, a second stem region, a third stem region, a fourth stem region, and the tracrRNA-crRNA complementarity region. More specifically, the wild-type gRNA may comprise a wild-type tracrRNA having the nucleotide sequence of SEQ ID NO: 11, or may comprise a wild-type crRNA having the nucleotide sequence of SEQ ID NO: 12. In addition, the wild-type gRNA may be fused into a single guide RNA, thereby forming sgRNA having the nucleotide sequence of SEQ ID NO: 13.

In an embodiment, the gRNA for Cas12f1, TnpB, or a variant protein thereof is characterized by being an engineered gRNA obtained by adding a new configuration to a wild-type gRNA found in nature, removing and/or substituting the existing structure in the wild-type gRNA, or modifying a part of the structure in the wild-type gRNA.

In some embodiments, the engineered guide RNA comprises a sequence having a wild-type gRNA sequence in which one or more nucleotides have been substituted, deleted, inserted, or added, and a portion of the engineered guide RNA, excluding the guide sequence, has at least 50%, 50%, 55%, 60%, 65%, 70%, 75%, 80%, 85%, 90%, 91%, 92%, 93%, 94%, 95%, 96%, 97%, 98%, 99%, or 95% sequence identity with the wild-type Cas12f1 guide RNA. In the context of RNA, nucleic acid, or polypeptide, the term "sequence identity" refers to a value determined by comparing two sequences that are optimally aligned over a comparison window, in which a sequence portion of RNA, nucleic acid, or the like within the comparison window may comprise insertions or deletions (that is, gaps) relative to the reference sequence to achieve optimal alignment.

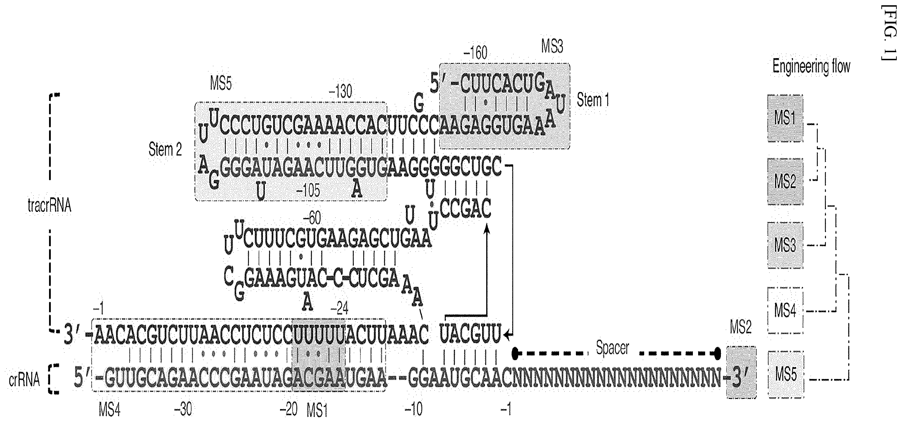

Hereinafter, the structure of wild-type and engineered gRNAs and modifications thereof will be described in detail for each of the five modification sites. The modification site is abbreviated as "MS (modification site)" throughout this specification, and the numbers following "modification site" or "MS" are sequentially assigned depending on engineering flow of each modification site according to an embodiment. However, this does not mean that engineering at the modification site with a later number necessarily includes engineering at the modification site with an earlier number. FIG. 1 illustrates MS1 to MS5, which are modification sites included in the engineered guide RNA (engineered gRNA) according to an embodiment of the present disclosure, on a wild-type guide RNA sequence.

### (2) Structure and definition of gRNA

The guide RNA (gRNA) of the present disclosure comprises crRNA. crRNA is a partial sequence present within crRNA which binds and/or interacts with tracrRNA and/or an effector protein. The crRNA may be a wild-type crRNA or engineered crRNA. Here, the crRNA may comprise a direct repeat sequence and a guide sequence (spacer sequence), and the direct repeat sequence may be located at the 5' end of the guide sequence. In addition, the crRNA may be located at the 3' end of tracrRNA.

In addition, the guide RNA comprises tracrRNA. The tracrRNA scaffold sequence is at least a part of the tracrRNA sequence that binds and/or interacts with crRNA and/or an effector protein.

The tracrRNA may be a wild-type tracrRNA or engineered tracrRNA. The engineered crRNA or tracrRNA may be a sequence obtained by artificial modification (substitution, deletion, or insertion) of a partial (nucleotide) sequence of the wild-type crRNA or tracrRNA, or a sequence modified to have a shorter length than the wild-type crRNA or tracrRNA sequence.

### (2-1) Scaffold sequence

In a case where a sequence of the wild-type or engineered guide RNA (gRNA) according to an embodiment of the present disclosure is functionally divided, it may be divided into a sequence portion that interacts with Cas12f1, TnpB, or a variant protein thereof to cause the gRNA and the protein to form a complex and a sequence portion that allows a complex of the gRNA with the protein to locate a target nucleic acid. Here, the sequence portion that interacts with the Cas12f1, TnpB, or the variant protein thereof to cause the gRNA and the protein (endonuclease) to form a complex may be referred to as a scaffold sequence. Specifically, the scaffold sequence may comprise sequences of two or more RNA molecules that are tracrRNA and crRNA.

In an embodiment, in a case where the engineered gRNA is a dual guide RNA, the scaffold sequence may comprise a tracrRNA sequence in the engineered gRNA sequence and a CRISPR RNA repeat sequence included in crRNA. As an example, the tracrRNA sequence may be a modified version obtained by modifying at least a part of the tracrRNA sequence found in nature. In addition, the CRISPR RNA repeat sequence may be a modified version obtained by modifying at least a part of the CRISPR RNA repeat sequence found in nature.

In another embodiment, in a case where the engineered guide RNA is a single guide RNA (sgRNA), the scaffold sequence may comprise an engineered tracrRNA sequence, a linker sequence, and a CRISPR RNA repeat sequence included in an engineered crRNA sequence. In an embodiment, the tracrRNA sequence may be a modified version obtained by modifying at least a part of the tracrRNA sequence found in nature.

In addition, in an embodiment, the scaffold sequence comprises portions of tracrRNA and crRNA, and does not necessarily refer to a single molecule of RNA. The scaffold sequence may comprise regions that are further subdivided into a first stem region, a second stem region, a third stem region, a fourth stem region, and a tracrRNA-crRNA complementarity region (which may be referred to as a fifth stem region). In the present disclosure, among the subdivided regions, the first stem region comprising modification site 3 (MS3), the second stem region comprising modification site 5 (MS5), and the tracrRNA-crRNA complementarity region comprising modification site 1 (MS1) and modification site 4 (MS4) may be defined as corresponding to or included in regions marked by single dotted line boxes in different shades of color in FIG. 1. In addition, the third stem region may be defined as corresponding to or included in the G(-90)-C(-74) sequence in FIG. 1, and the fourth stem region may be defined as corresponding to or included in the U(-68)-A(-35) sequence in FIG. 1.

Meanwhile, in the present disclosure, the regions, which are subdivided into the stem region, the tracrRNA-crRNA complementarity region, and the like, do not encompass all regions of the scaffold sequence, and the scaffold sequence may comprise other regions or sequences that do not correspond to the subdivided regions.

In another embodiment, the wild-type Cas12f1 gRNA comprises a scaffold sequence that may have the subdivided regions as described above, and is characterized by comprising a scaffold sequence that comprises (i) one or more stem regions, (ii) a tracrRNA-crRNA complementarity region, and optionally (iii) a region containing three or more consecutive uracil (U) residues in the tracrRNA-crRNA complementarity region. Specifically, the wild-type Cas12f1 gRNA will comprise tracrRNA comprising one or more stem regions, and tracrRNA and/or crRNA comprising a tracrRNA-crRNA complementarity region (or another stem region formed by complementary binding between tracrRNA and crRNA). For detailed information on the structure of a wild type V-F CRISPR/Cas gRNA, see Takeda et al., Structure of the miniature type VF CRISPR-Cas effector enzyme, Molecular Cell 81, 1-13 (2021). In an embodiment, the wild-type Cas12f1 guide RNA may comprise tracrRNA consisting of the nucleotide sequence of SEQ ID NO: 11 and crRNA consisting of the nucleotide sequence of SEQ ID NO: 12.

In the present disclosure, the engineered gRNA, which can be used in a target nucleic acid editing system for homology-directed repair, may comprise a modification caused by (a) deletion of at least a part of one or more stem regions, (b) deletion of at least a part of the tracrRNA-crRNA complementarity region, or (c) replacement of one or more uracil (U) residues in three or more consecutive U residues when the consecutive U residues are present. Details of the modification caused by each of the deletion and the replacement are described later.

Meanwhile, the engineered crRNA according to an embodiment of the present disclosure may further comprise, as a scaffold sequence, (d) a U-rich tail region having one or more uridine residues at the 3'-end of the crRNA. This region is added to the engineered scaffold region that may be introduced to enhance homology-directed repair efficiency of the target nucleic acid editing system of the present disclosure which comprise a complex of a guide RNA with Cas12f1, TnpB, or a variant protein thereof. Details about the U-rich tail region will be described later.

In an embodiment, the engineered scaffold sequence may comprise a combination of modifications in one or more of the following regions of a scaffold sequence found in nature: (i) the one or more stem regions, (ii) the tracrRNA-crRNA complementarity region, and (iii) the region containing three or more consecutive uracil (U) residues in the tracrRNA-crRNA complementarity region. Here, the engineered tracrRNA may be tracrRNA that has been modified to have a shorter length than a wild-type tracrRNA. In addition, the engineered tracrRNA may be tracrRNA that has been modified (modification at MS1) not to contain a sequence of four or five or more consecutive uridine residues. In addition, the engineered tracrRNA may be tracrRNA that has been modified not to contain a sequence of five or more consecutive uridine residues and to have a shorter length than a wild-type tracrRNA. In addition, the engineered tracrRNA may sequentially comprise, in a 5' to 3' direction, a first stem region, a second stem region, a third stem region, a fourth stem region, and a part of the tracrRNA-crRNA complementarity region. In addition, the engineered crRNA may sequentially comprise, in a 5' to 3' direction, a part of the tracrRNA-crRNA complementarity region and a spacer sequence that is a guide sequence. The tracrRNA-crRNA complementarity region of the tracrRNA may comprise any polynucleotide having sufficient complementarity to bind to a direct repeat sequence of the crRNA (that is, the tracrRNA-crRNA complementarity region of the crRNA).

In another embodiment, the engineered gRNA may be gRNA consisting of a sequence represented by Formula (I) or having at least 80%, at least 85%, at least 90%, or at least 95% sequence identity therewith [In Formula (I) as shown below and Formula (I) as described throughout this specification, the black solid line refers to a chemical bond (for example, phosphodiester bond) between nucleotides, and the gray thick line refers to a complementary bond between nucleotides].

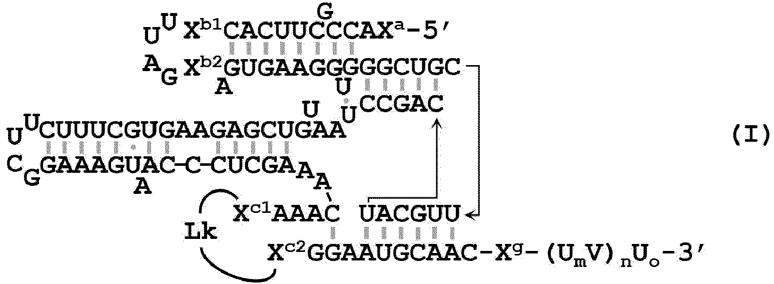

In Formula (I), X^{a}, X^{b1}, X^{b2}, X^{c1}, and X^{c2} each independently consists of 0 to 35 (poly)nucleotides, X^{g} is a guide sequence that consists of 10 to 30 nucleotides and is hybridizable with or complementary to a target sequence, Lk is a polynucleotide linker of 2 to 20 nucleotides or is absent, and (UₘV)ₙUₒ is present as a U-rich tail or absent, and when (UₘV)ₙUₒ is present, U is uridine, V is each independently A, C, or G, m and o are each independently an integer between 1 and 20, and n is an integer between 0 and 5.

Here, in a case where the X^{a}, X^{b1}, X^{b2}, X^{c1}, or X^{c2} consists of 0 nucleotides, it is interpreted to mean that the X^{a}, X^{b1}, X^{b2}, X^{c1}, or X^{c2} is absent.

In addition, in Formula (I), in a case where the X^{a}, X^{b1}, X^{b2}, X^{c1}, or X^{c2} consists of 0 nucleotides or is absent, it may be interpreted that if there are two or more nucleotides linked through the X^{a}, X^{b1}, X^{b2}, X^{c1}, or X^{c2}, these nucleotides are directly linked to each other in any way. For example, in Formula (I), in a case where X^{b1} consists of 0 nucleotides or is absent, the nucleotide directly connected to the 5'-end of X^{b1} and the nucleotide directly connected to the 3'-end of X^{b1} may be directly linked to each other, for example, through a phosphodiester bond.

In some embodiments, X^{a} may consist of 0 to 20 (poly)nucleotides, X^{b1} may consist of 0 to 13 (poly)nucleotides, X^{b2} may consist of 0 to 14 (poly)nucleotides, X^{c1} may consist of 0 to 28 (poly)nucleotides, or X^{c2} may consist of 0 to 27 (poly)nucleotides.

For the scaffold sequence, modification (i) may correspond to the polynucleotide indicated by X^{a}, X^{b1}, or X^{b2}, modification (ii) may correspond to the polynucleotide indicated by X^{c1} and X^{c2}, and modification (iii) may be present in the polynucleotide indicated by X^{c1}.

For details about modifications (i) to (iii) of the scaffold sequence, see the section "(4) Modification of gRNA to achieve highly efficient homology-directed repair."

### (2-2) Guide sequence

The wild-type or engineered guide RNA (gRNA) according to an embodiment of the present disclosure may comprise a sequence portion that enables it to locate a target nucleic acid, that is, at least one guide sequence that hybridizes with or forms a complementary bond with a target sequence in a gene.

The sequence referred to herein as "guide sequence" or "spacer sequence" is a sequence complementary to a target sequence in a target nucleic acid or target gene, and is connected to the 3'-end of the crRNA repeat sequence. The guide sequence is homologous to the protospacer sequence adjacent to the protospacer adjacent motif (PAM) sequence recognized by Cas12f1, TnpB, or a variant protein thereof, and has a sequence in which thymidine (T) in the protospacer sequence is replaced with uridine (U). Here, the target sequence and the protospacer sequence are determined within a sequence adjacent to the PAM sequence contained in the target nucleic acid, and the guide sequence is determined accordingly.

In an embodiment, the guide sequence portion of crRNA may bind complementarily to the target nucleic acid. In an embodiment, the guide sequence portion of crRNA may bind complementarily to a target sequence portion in the target nucleic acid. As an example, in a case where the target nucleic acid is double-stranded DNA, the guide sequence may be a sequence complementary to a target sequence contained in the target strand of the double-stranded DNA. Here, in a case where the target nucleic acid is double-stranded DNA, the guide sequence may comprise a sequence homologous to the protospacer sequence contained in the non-target strand of the double-stranded DNA. Specifically, the guide sequence may have the same nucleotide sequence as the protospacer sequence, except that each thymidine (T) contained in the nucleotide sequence is replaced with uridine (U). As an example, the guide sequence may comprise an RNA sequence corresponding to the DNA sequence of the protospacer. The guide sequence may comprise a combination of two guide sequences capable of hybridizing with one target sequence selected from the upstream and one target sequence selected from the downstream.

In an embodiment, the guide sequence may have a length of 10 to 50 nucleotides. Preferably, the guide sequence may have a length of 10 to 30 nucleotides. More preferably, the spacer sequence may have a length of 17 to 25 nucleotides.

The "target sequence" refers to a sequence present in a target nucleic acid or target gene, which is recognized by the guide RNA of the target nucleic acid editing system or TaRGET system of the present disclosure, or is an object to be modified by the target nucleic acid editing system or TaRGET system. Specifically, the target sequence refers to a sequence that is complementary to the guide sequence contained in the guide RNA or a sequence that binds complementarily to the guide sequence. The "target strand" refers to a strand containing a target sequence. In a case where the target nucleic acid or target gene is single-stranded, that strand may be a target strand. Alternatively, in a case where the target nucleic acid or target gene is double-stranded, one of the double strand may be a target strand, and there may be a strand complementary to the target strand. Here, the strand complementary to the target strand is referred to as a "non-target strand." The non-target strand comprises a protospacer adjacent motif (PAM) sequence and a protospacer sequence. The PAM sequence is a sequence recognized by Cas12f1, TnpB, or a variant protein thereof of the target nucleic acid editing system (or TaRGET system) of the present disclosure. The protospacer sequence is a sequence located at the 5'-end or 3'-end of the PAM sequence. The protospacer sequence is a sequence that is complementary to a target sequence or a sequence that binds complementarily to the target sequence. Correlation between the protospacer sequence and the target sequence is similar to correlation between the target sequence and the guide sequence. Due to such a characteristic, the guide sequence may typically be designed using a protospacer sequence. That is, in a case of designing a guide sequence that binds complementarily to the target sequence, the guide sequence may be designed as a nucleotide sequence having the same nucleotide sequence as the protospacer sequence. Here, T in the nucleotide sequence of the protospacer sequence is replaced with U to design the guide sequence.

The target sequence may be a sequence of 15 to 40 nucleotides. As an example, the target sequence may be a sequence of 15 to 20, 15 to 25, 15 to 30, 15 to 35, or 15 to 40 nucleotides. Alternatively, the target sequence may be a sequence of 20 to 25, 20 to 30, 20 to 35, or 20 to 40 nucleotides. Alternatively, the target sequence may be a sequence of 25 to 30, 25 to 35, or 25 to 40 nucleotides. Alternatively, the target sequence may be a sequence of 30 to 35 or 30 to 40 nucleotides. Alternatively, the target sequence may be a sequence of 35 to 40 nucleotides. As another example, the target sequence may be a sequence of 15, 16, 17, 18, 19, 20, 21, 22, 23, 24, 25, 26, 27, 28, 29, 30, 31, 32, 33, 34, 35, 36, 37, 38, 39, or 40 nucleotides.

In some embodiments, the guide sequence may be a sequence that binds complementarily to a target sequence. Here, the complementary bond may optionally include at least one mismatch bond. For example, the guide sequence is a sequence that hybridizes with or binds complementarily to the target sequence, in which the complementary bond may comprise 0 to 5 mismatches. Alternatively, the guide sequence may be a nucleotide sequence that is at least 70% complementary to the target sequence. Here, in a case where the target sequence is DNA, for adenosine (A) present in the target sequence, the guide sequence may comprise uridine (U) that is capable of forming a complementary bond to the adenosine (A).

In an embodiment, the guide sequence may be a sequence that is at least 70% to 75%, at least 70% to 80%, at least 70% to 85%, at least 70% to 90%, at least 70% to 95%, at least 70% to 100%, at least 75% to 80%, at least 75% to 85%, at least 75% to 90%, at least 75% to 95%, or at least 75% to 100% complementary to the target sequence. Alternatively, the guide sequence may be a sequence that is at least 80% to 85%, at least 80% to 90%, at least 80% to 95%, at least 80% to 100%, at least 85% to 90%, at least 85% to 95%, or at least 85% to 100% complementary to the target sequence. Alternatively, the guide sequence may be a sequence that is at least 90% to 95%, at least 90% to 100%, or at least 95% to 100% complementary to the target sequence. Alternatively, the guide sequence may be a sequence that is at least 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100% complementary to the target sequence.

The guide sequence may be the same as or similar to the protospacer sequence. Alternatively, the guide sequence may be a sequence having sequence identity or similarity to the protospacer sequence. Here, the sequence identity or similarity may be at least 70%. Here, for thymidine (T) present in the protospacer sequence, the guide sequence may comprise uridine (U) instead of thymidine (T).

In an embodiment, the guide sequence may be a sequence that is at least 70% to 75%, at least 70% to 80%, at least 70% to 85%, at least 70% to 90%, at least 70% to 95%, at least 70% to 100%, at least 75% to 80%, at least 75% to 85%, at least 75% to 90%, at least 75% to 95%, or at least 75% to 100% identical or similar to the protospacer sequence. Alternatively, the guide sequence may be a sequence that is at least 80% to 85%, at least 80% to 90%, at least 80% to 95%, at least 80% to 100%, at least 85% to 90%, at least 85% to 95%, or at least 85% to 100% identical or similar to the protospacer sequence. Alternatively, the guide sequence may be a sequence that is at least 90% to 95%, at least 90% to 100%, or at least 95% to 100% identical or similar to the protospacer sequence. Alternatively, the guide sequence may be a sequence that is at least 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100% identical or similar to the protospacer sequence.

In another embodiment, the guide sequence may be a sequence that is at least 70% to 75%, at least 70% to 80%, at least 70% to 85%, at least 70% to 90%, at least 70% to 95%, at least 70% to 100%, at least 75% to 80%, at least 75% to 85%, at least 75% to 90%, at least 75% to 95%, or at least 75% to 100% identical or similar to the protospacer sequence. Alternatively, the guide sequence may be a sequence that is at least 80% to 85%, at least 80% to 90%, at least 80% to 95%, at least 80% to 100%, at least 85% to 90%, at least 85% to 95%, or at least 85% to 100% identical or similar to the protospacer sequence. Alternatively, the guide sequence may be a sequence that is at least 90% to 95%, at least 90% to 100%, or at least 95% to 100% identical or similar to the protospacer sequence. Alternatively, the guide sequence may be a sequence that is at least 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100% identical or similar to the protospacer sequence.

### (3) Single guide RNA or dual guide RNA

The engineered guide RNA according to an embodiment of the present disclosure may be single guide RNA or dual guide RNA. The dual guide RNA means that the guide RNA consists of two RNA molecules: tracrRNA and crRNA. The single guide RNA (sgRNA) means that the 3'-end of an engineered tracrRNA and the 5'-end of an engineered crRNA are connected through a linker.

In an embodiment, the engineered single guide RNA (sgRNA) may further comprise a linker sequence, and the tracrRNA sequence and the crRNA sequence may be connected through the linker sequence. Preferably, this may include a case where the 3'-end of the tracrRNA-crRNA complementarity sequence in the tracrRNA and the 5'-end of the tracrRNA-crRNA complementarity sequence in the crRNA, which are contained in the engineered scaffold sequence, may be connected to each other through a linker. More preferably, the tracrRNA-crRNA complementarity regions of the tracrRNA and crRNA may be connected to each other, at the 3'-end of the tracrRNA and the 5'-end of the crRNA, by the linker 5'-GAAA-3'. For details about the linker, see details about Lk in Formula (I).

In an embodiment, a sequence of the single guide RNA is such that the tracrRNA sequence, the linker sequence, the crRNA sequence, and the U-rich tail sequence are sequentially linked in a 5' to 3' direction. A part of the tracrRNA sequence and at least a part of the CRISPR RNA repeat sequence contained in the crRNA sequence have sequences complementary to each other.

In addition, the engineered guide RNA according to an embodiment of the present disclosure may be dual guide RNA in which tracrRNA and crRNA form separate RNA molecules. Here, a part of the tracrRNA and a part of the crRNA may have sequences complementary to each other so that double-stranded RNA is formed. More specifically, in the dual guide RNA, a part containing the 3'-end of the tracrRNA and a part containing the CRISPR RNA repeat sequence of the crRNA may form a double strand. The engineered guide RNA may bind to Cas12f1, TnpB, or a variant protein thereof to form a complex of the guide RNA with the protein. This complex recognizes a target sequence complementary to the guide sequence contained in the crRNA sequence, which allows for editing of a target nucleic acid comprising the target sequence.

In an embodiment, a sequence of the tracrRNA may comprise a complementary sequence having 0 to 20 mismatches with the CRISPR RNA repeat sequence. Preferably, the sequence of the tracrRNA may comprise a complementary sequence having 0 to 8 or 8 to 12 mismatches with the CRISPR RNA repeat sequence.

### (4) Modification of gRNA to achieve highly efficient homology-directed repair

### (4-1) Overview

The modification applied to the engineered guide RNA(gRNA) of the present disclosure ultimately aims to achieve high homology-directed repair efficiency. That is, the modifications described in the present disclosure are made to provide highly efficient homology-directed repair (HDR) that was not achievable with conventional CRISPR/Cas systems. Such highly efficient homology-directed repair is accomplished by producing an engineered gRNAthat has a shorter length and exhibits equal or enhanced recognition/cleavage efficiency for a target nucleic acid as compared with longer wild-type gRNAs. By doing so, more space within the packaging limit (approximately 4.7 kb) of AAV delivery vehicles can be allocated for other essential components (for example, donor nucleic acid molecule) needed for homology-directed repair and/or additional components (for example, shRNAfor inhibition of a gene involved in non-homologous end joining).

Therefore, the engineered gRNA provided in the present disclosure basically comprises a sequence having the wild-type Cas12f1 gRNA sequence in which one or more nucleotides have been substituted, deleted, inserted, or added. Here, the engineered gRNA may have at least 50% sequence identity with the wild-type Cas12f1 gRNA, excluding the guide sequence.

As described above, the wild-type Cas12f1 guide RNA may comprise trans-activating CRISPR RNA (tracrRNA) and CRISPR RNA (crRNA) which comprise (i) one or more stem regions, (ii) a tracrRNA-crRNA complementarity region, and optionally (iii) a region containing three or more consecutive uracil (U) residues, and the engineered guide RNA of the present disclosure may comprise at least one modification selected from the group consisting of (a) deletion of at least a part of one or more stem regions; (b) deletion of at least a part of the tracrRNA-crRNA complementarity region; (c) replacement of one or more uracil (U) residues with A, G, or C in three or more consecutive U residues when the consecutive U resides are present; and (d) addition of one or more uridine residues to the 3'-end of the crRNA sequence.

Here, the tracrRNA included in the wild-type Cas12f1 gRNAmay consist of or comprise the nucleotide sequence of SEQ ID NO: 11. In addition, the crRNA included in the wild-type Cas12f1 gRNA may consist of or comprise the nucleotide sequence of SEQ ID NO: 12. In an embodiment, the wild-type Cas12f1 guide RNA may be gRNA comprising tracrRNA consisting of the nucleotide sequence of SEQ ID NO: 11 and crRNA consisting of the nucleotide sequence of SEQ ID NO: 12. Table 1 provides sequence information for tracrRNA and crRNA of the wild-type Cas12f1.

**[Table 1]**

| **Name** | **Nucleotide sequence** | **SEQ ID NO** |
|---|---|---|
| Wild-type tracrRNA | CUUCACUGAUAAAGUGGAGAACCGCUUCACCAAAAGCUG | 11 |
| | UCCCUUAGGGGAUUAGAACUUGAGUGAAGGUGGGCUGCU UGCAUCAGCCUAAUGUCGAGAAGUGCUUUCUUCGGAAAG UAACCCUCGAAACAAAUUCAUUUUCCUCUCCAAUUCUGC ACAA | |
| Wild-type crRNA | GUUGCAGAACCCGAAUAGACGAAUGAAGGAAUGCAAC | 12 |

In an embodiment, the wild-type Cas12f1 guide RNA may comprise tracrRNA and crRNA which sequentially comprise, from the 5'-end, a first stem region, a second stem region, a third stem region, a fourth stem region, and the tracrRNA-crRNA complementarity region, and the engineered guide RNA may comprise at least one modification selected from the group consisting of (a1) deletion of at least a part of the first stem region; (a2) deletion of at least a part of the second stem region; (b) deletion of at least a part of the tracrRNA-crRNA complementarity region; (c) replacement of one or more uracil (U) residues with A, G, or C in three or more consecutive U residues when the consecutive U residues are present in the tracrRNA-crRNA complementarity region; and (d1) addition of a U-rich tail to the 3'-end of the crRNA sequence (in which a sequence of the U-rich tail is represented by 5'-(UₘV)ₙUₒ-3', where V is each independently A, C, or G, m and o are integers between 1 and 20, and n is an integer between 0 and 5).

Here, the tracrRNA included in the wild-type Cas12f1 gRNAmay consist of or comprise the nucleotide sequence of SEQ ID NO: 11. In addition, the crRNA included in the wild-type Cas12f1 gRNA may consist of or comprise the nucleotide sequence of SEQ ID NO: 12. In an embodiment, the wild-type Cas12f1 guide RNA may be gRNA comprising tracrRNA consisting of the nucleotide sequence of SEQ ID NO: 11 and crRNA consisting of the nucleotide sequence of SEQ ID NO: 12.

Hereinafter, modifications at respective modification sites in the engineered gRNA will be described in detail.

### (4-2) Modification at modification site 1 (MS1)

This section describes a modification at MS1. In an embodiment, wild-type tracrRNA (for example, SEQ ID NO: 11), which may be naturally occurring guide RNA (gRNA), may have a sequence containing five consecutive uracil (U) residues therein. This poses a problem in that, in a case of attempting to express the wild-type tracrRNA in a cell using a vector or the like, such a sequence acts as a transcription termination signal under certain conditions, thereby causing unintended early termination of transcription. That is, in a case where the sequence containing five consecutive U residues acts as a transcription termination signal, normal or complete expression of the tracrRNA is inhibited, and formation of normal or complete gRNA is also inhibited, which consequently decreases cleavage or homology-directed repair efficiency of the target nucleic acid editing system of the present disclosure for a target nucleic acid or target gene.

Therefore, in order to solve the above-mentioned problem, the engineered gRNA may be such that at least one uracil (U) of three or more, four or more, or five or more consecutive U residues, preferably four or five U residues, which are contained in the wild-type tracrRNA (for example, SEQ ID NO: 11), is artificially modified into another nucleotide such as A, C, T, or G.

In an embodiment, an engineered gRNA is provided which comprises a modification in a region, referred to as MS1, containing three or more consecutive uracil (U) residues. The modification is such that at least one of the three or more consecutive uracil (U) residues is replaced with a different type of nucleotide. As an example, the three or more consecutive U residues may be present within the tracrRNA-crRNA complementarity region of tracrRNA, and this region may be modified by replacing at least one of the three or more consecutive U residues with A, G, or C so that a sequence with three or more consecutive U residues does not appear.

Here, it is preferable that the sequence within the tracrRNA-crRNA complementarity region of crRNA, which corresponds to the sequence to be modified, is also modified together. In an embodiment, in a case where there is the sequence 5'-ACGAA-3' within the tracrRNA-crRNA complementarity region of crRNA, which forms a partial complementary bond with the sequence 5'-UUUUU-3' within the tracrRNA-crRNA complementarity region of tracrRNA, this sequence may be replaced with 5'-NGNNN-3'. Here, N is each independently A, C, G, or U.

In another embodiment, the engineered gRNA may be gRNA consisting of a sequence represented by Formula (I) or having at least 80%, at least 85%, at least 90%, or at least 95% sequence identity therewith [In Formula (I) as shown below and Formula (I) as described throughout this specification, the black solid line refers to a chemical bond (for example, phosphodiester bond) between nucleotides or certain molecules, and the gray thick line refers to a complementary bond between nucleotides]. Here, MS1 may be present within the polynucleotide indicated by X^{c1} and X^{c2} in Formula (I).

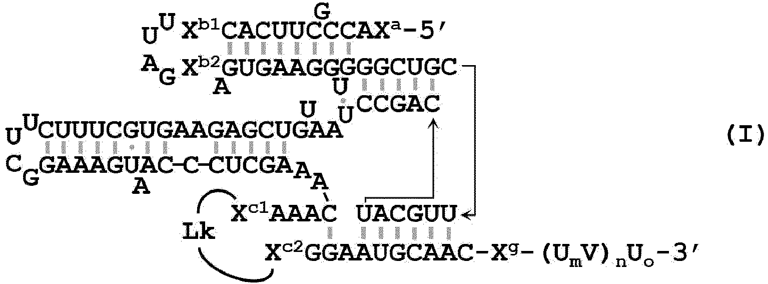

In an embodiment, in a case where in the engineered gRNA of Formula (I), three or more consecutive uracil (U) residues are present in a sequence of X^{c1}, the sequence may comprise a modification where at least one of the consecutive U residues is replaced with A, G, or C. As an example, in a case where the sequence 5'-UUUUU-3' exists in the sequence of X^{c1}, this sequence may be replaced with 5'-NNNCN-3'. Here, N is each independently A, C, G, or U. As a more specific example, the sequence 5'-UUUUU-3' in the sequence of X^{c1} may be replaced with any one nucleotide sequence selected from the group consisting of the following sequences; however, the nucleotide sequence is not limited to the following sequences as long as it prevents occurrence of a sequence containing three or more consecutive U residues: 5'-UUUCU-3', 5'-GUUCU-3', 5'-UCUCU-3', 5'-UUGCU-3', 5'-UUUCC-3', 5'-GCUCU-3', 5 '-GUUCC-3', 5'-UCGCU-3', 5'-UCUCC-3', 5'-UUGCC-3', 5'-GCGCU-3', 5'-GCUCC-3', 5'-GUGCC-3', 5'-UCGCC-3', 5'-GCGCC-3', and 5'-GUGCU-3'.

In another embodiment, the engineered gRNA of Formula (I) comprises a region (also referred to as a tracrRNA-crRNA complementarity region) where the sequence of X^{c2} forms at least a partial complementary bond with the sequence of X^{c1}. Here, the corresponding sequence within the sequence of X^{c2}, which forms at least one complementary bond with three or more consecutive U residues present in the sequence of X^{c1}, may also be modified. As an example, in a case where the sequence 5'-ACGAA-3' exists in the sequence of X^{c2}, this sequence may be replaced with 5'-NGNNN-3'. Here, N is each independently A, C, G, or U. As a more specific example, the sequence 5'-ACGAA-3' in the sequence of X^{c1} may be replaced with any one nucleotide sequence selected from, but not limited to, the group consisting of the following sequences: 5'-AGGAA-3', 5'-AGCAA-3 ', 5'-AGAAA-3', 5'-AGCAU-3', 5'-AGCAG-3', 5'-AGCAC-3', 5'-AGCUA-3', 5'-AGCGA-3', 5'-AGCCA-3', 5'-UGCAA-3', 5'-UGCUA-3', 5'-UGCGA-3', 5'-UGCCA-3', 5'-GGCAA-3', 5' -GGCUA-3', 5'-GGCGA-3', 5'-GGCCA-3', 5'-CGCAA-3', 5'-CGCUA-3', 5'-CGCGA-3', and 5'-CGCCA-3'.

In yet another embodiment, in a case where a sequence containing three or more consecutive U residues in the sequence of X^{c1} is modified into another sequence, it is preferable that the nucleotide, which corresponds thereto (that is, forms at least a partial complementary bond therewith), in the sequence of X^{c2} is modified so that it can form a complementary bond with the nucleotide to be modified. For example, in a case where the sequence 5'-UUUUU-3' in the sequence of X^{c1} is modified into 5'-GUGCU-3', it is preferable that the sequence 5'-ACGAA-3' in the sequence of X^{c2} is modified into 5'-AGCAA-3'; however, a complementary bond is not necessarily required.

### (4-3) Modification at modification site 2 (MS2)

This section describes a modification at MS2. In an embodiment, the engineered guide RNA (gRNA) may be obtained by adding a new configuration to the gRNA found in nature, and may be such that one or more uridine residues are added to the 3'-end of the crRNA sequence. Here, the 3'-end of the crRNA sequence may be the 3'-end of the guide sequence (spacer). In the present disclosure, one or more uridine residues added to the 3'-end are also referred to as a U-rich tail. The engineered gRNA, which contains one or more uridine residues or a U-rich tail added to the 3'-end, serves to increase efficiency of nucleic acid cleavage or homology-directed repair of the hypercompact CRISPR/Cas12 system for a target nucleic acid or target gene.

The term "U-rich tail" as used herein may refer not only to an RNA sequence itself that is rich in uridine (U), but also to a DNA sequence encoding the same. This term is appropriately interpreted depending on the context. The present inventors have experimentally elucidated the structure and effects of the U-rich tail sequence in detail. The U-rich tail sequence will be described in more detail with specific embodiments.

In an embodiment, the U-rich tail sequence may be represented by Ux. Here, x may be 1, 2, 3, 4, 5, 6, 7, 8, 9, 10, 11, 12, 13, 14, 15, 16, 17, 18, 19, or 20. As an example, x may be an integer within a range of two numerical values selected from the numerical values listed above. For example, x may be an integer between 1 and 6. As another example, x may be an integer between 1 and 20. In an embodiment, x may be an integer of 20 or higher.

In another embodiment, the U-rich tail sequence is represented by 5'-(UₘV)ₙUₒ-3', where V is each independently A, C, or G, m and o are integers between 1 and 20, and n is an integer between 0 and 5. As an example, n may be 0, 1, or 2. For example, m and o may each independently be 1, 2, 3, 4, 5, 6, 7, 8, 9, or 10.

In yet another embodiment, the engineered gRNAmay be gRNAconsisting of a sequence represented by Formula (I) or having at least 80%, at least 85%, at least 90%, or at least 95% sequence identity therewith. Here, MS2 is a region corresponding to (UₘV)ₙUₒ in Formula (I), where U is uridine, and V, m, o and n are as defined above.

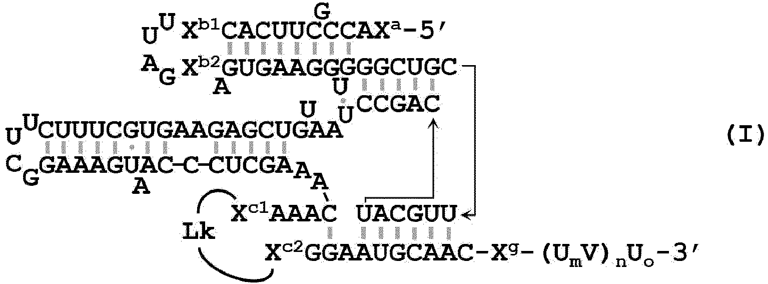

Preferably, in the engineered gRNA represented by Formula (I), (UₘV)ₙUₒ may be a U-rich tail where (i) n is 0, o is an integer between 1 and 6, or (ii) V is A or G, m and o are each independently an integer between 3 and 6, and n is an integer between 1 and 3. In a specific example, (UₘV)ₙUₒ in Formula (I) may be a U-rich tail that consists of any one sequence selected from the group consisting of 5'-U-3', 5'-UU-3', 5'-UUU-3', 5'-UUUU-3', 5'-UUUUU-3', 5'-UUUUUU-3', 5 '-UUURUUU-3', 5'-UUURUUURUUU-3', 5'-UUUURU-3', 5'-UUUURUU-3', 5'-UUUURUUU-3', 5'-UUUURUUUU-3', 5'-UUUURUUUUU-3', and 5'-UUUURUUUUUU-3', where R is A or G.

In still yet another embodiment, the U-rich tail sequence may comprise a modified uridine repeat sequence that contains a non-uridine ribonucleoside (A, C, or G) for every 1 to 5 repetitions of uridine. The modified consecutive uridine sequence is particularly useful in a case of designing a vector that expresses an engineered crRNA. In an embodiment, the U-rich tail sequence may comprise a sequence in which UV, UUV, UUUV, UUUUV, and/or UUUUUV are repeated one or more times. Here, V is one of A, C, and G.

In addition, the U-rich tail sequence may be in a form of a combination of the sequence represented by Ux and the sequence represented by (UaV)n. In an embodiment, the U-rich tail sequence may be represented by (U)n1-V1-(U)n2-V2-Ux. Here, V1 and V2 are each one of adenine (A), cytidine (C), and guanine (G). Here, n1 and n2 may each be an integer between 1 and 4. Here, x may be an integer between 1 and 20. In addition, the U-rich tail sequence may have a length of 1 nt, 2 nts, 3 nts, 4 nts, 5 nts, 6 nts, 7 nts, 8 nts, 9 nts, 10 nts, 11 nts, 12 nts, 13 nts, 14 nts, 15 nts, 16 nts, 17 nts, 18 nts, 19 nts, or 20 nts. In an embodiment, the U-rich tail sequence may have a length of 20 nts or more.

In still yet another embodiment, in a case where the engineered gRNA is expressed in a cell, the U-rich tail may exist in multiple forms due to premature termination of transcription. For example, according to an embodiment, in a case where gRNA intended to contain a U-rich tail sequence of 5'-UUUUAUUUUUU-3' is transcribed in a cell, 4 or more or 5 or more T residues may act as a termination sequence, and thus gRNAs containing a U-rich tail such as 5'-UUUUAUUUU-3', 5'-UUUUAUUUUU-3', or 5'-UUUUAUUUUUU-3' may be produced simultaneously. Therefore, in the present disclosure, a U-rich tail containing four or more U residues may be understood to also include a U-rich tail sequence having a shorter length than the intended length.

In still yet another embodiment, the U-rich tail sequence may comprise additional nucleotides other than uridine, depending on the environment where the CRISPR/Cas12 system for homology-directed repair of the present disclosure is actually used and the expression environment, such as the internal environment of a eukaryotic or prokaryotic cell.

### (4-4) Modification at modification site 3 (MS3)

This section describes a modification at MS3. As described above, MS3 refers to a region (which may be referred to as the first stem region) that comprises at least a part of the nucleotides forming a stem structure within a complex of gRNA with an effector protein. MS3 may comprise a region that does not interact with the effector protein when the gRNA and the effector protein form a complex. The modification at MS3 involves removal of at least a part of the first stem region near the 5'-end of tracrRNA.

In an embodiment, the engineered gRNA comprises a modification where at least a part of the first stem region is deleted.

In another embodiment, the engineered gRNA comprises a modification where at least a part of the first stem region on tracrRNA is deleted, in which the at least a part of the first stem region to be deleted may consist of 1 to 20 nucleotides. Specifically, the at least a part of the first stem region may consist of 2 to 20, 3 to 20, 4 to 20, 5 to 20, 6 to 20, 7 to 20, 8 to 20, 9 to 20, 10 to 20, 11 to 20, 12 to 20, 13 to 20, 14 to 20, 15 to 20, 16 to 20, 17 to 20, 18 to 20, or 19 or 20 nucleotides.

In yet another embodiment, the engineered gRNAmay be gRNA consisting of a sequence represented by Formula (I) or having at least 80%, at least 85%, at least 90%, or at least 95% sequence identity therewith. Here, MS3 or the first stem region is a region corresponding to the polynucleotide indicated by X^{a} in Formula (I), in which due to the modification where at least the part of the first stem region is deleted, X^{a} may consist of 0 to 35 (poly)nucleotides, preferably 0 to 20, 0 to 19, 0 to 18, 0 to 17, 0 to 16, 0 to 15, 0 to 14, 0 to 13, 0 to 12, 0 to 11, 0 to 10, 0 to 9, 0 to 8, 0 to 7, 0 to 6, 0 to 5, 0 to 4, 0 to 3, 0 to 2, 1 or 0 (poly)nucleotides.

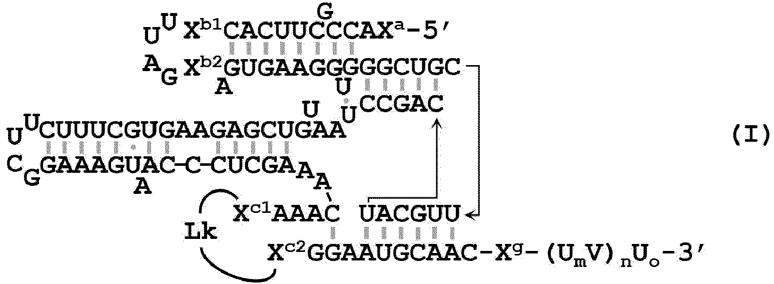

In an embodiment, in the engineered gRNA of Formula (I), X^{a} may comprise the nucleotide sequence of SEQ ID NO: 14 or a nucleotide sequence having the sequence of SEQ ID NO: 14 of which a part of or all nucleotides, preferably 1 to 20 nucleotides, are deleted. As an example, the nucleotide deletion may involve random deletion of at least 1, 2, 3, 5, 6, 7, 8, 9, 10, 11, 12, 13, 14, 15, 16, 17, 18, 19, or 20 nucleotides from the sequence of SEQ ID NO: 14. As a preferred example, the nucleotide deletion may involve sequential deletion of at least 1, 2, 3, 5, 6, 7, 8, 9, 10, 11, 12, 13, 14, 15, 16, 17, 18, 19 or 20 nucleotides from the 5'-end of the sequence of SEQ ID NO: 14. More specifically, X^{a} may comprise or consist of 5'-CUUCACUGAUAAAGUGGAGA-3' (SEQ ID NO: 14), 5'-UUCACUGAUAAAGUGGAGA-3' (SEQ ID NO: 15), 5'-UCACUGAUAAAGUGGAGA-3' (SEQ ID NO: 16), 5'-CACUGAUAAAGUGGAGA-3' (SEQ ID NO: 17), 5'-ACUGAUAAAGUGGAGA-3' (SEQ ID NO: 18), 5'-CUGAUAAAGUGGAGA-3' (SEQ ID NO: 19), 5'-UGAUAAAGUGGAGA-3' (SEQ ID NO: 20), 5'-GAUAAAGUGGAGA-3' (SEQ ID NO: 21), 5'-AUAAAGUGGAGA-3' (SEQ ID NO: 22), 5'-UAAAGUGGAGA-3' (SEQ ID NO: 23), 5'-AAAGUGGAGA-3' (SEQ ID NO: 24), 5'-AAGUGGAGA-3', 5'-AGUGGAGA-3', 5'-GUGGAGA-3', 5'-UGGAGA-3', 5'-GGAGA-3', 5'-GAGA-3', 5'-AGA-3', 5'-GA-3', or 5'-A-3', or X^{a} may be absent.

### (4-5) Modification at modification site 4 (MS4)

This section describes a modification at MS4. MS4 refers to a region spanning the 3'-end of tracrRNA and the 5'-end of crRNA, or, in a case of a single guide RNA form, a region where the sequence corresponding to tracrRNA and the sequence corresponding to crRNA form at least partial complementary bonding. MS4 may comprise at least a part of the sequence referred to as the tracrRNA-crRNA complementarity region (which may also be referred to as the fifth stem region). In the present disclosure, the tracrRNA-crRNA complementarity region may comprise both modification site 1 (MS1) and modification site 4 (MS4). The modification at MS4 comprises deletion of at least a part of the tracrRNA-crRNA complementarity region. The tracrRNA-crRNA complementarity region may comprise a part of tracrRNA and a part of crRNA. In this regard, the tracrRNA-crRNA complementarity region may comprise nucleotides such that partial nucleotides contained in tracrRNA can form complementary bonds with partial nucleotides contained in crRNA within a complex of gRNA with the nucleic acid degrading protein, and may comprise nucleotides adjacent thereto. The tracrRNA-crRNA complementarity region of tracrRNA may comprise a region that does not interact with the nucleic acid degrading protein within a complex of gRNA with the nucleic acid degrading protein.

In some embodiments, the engineered gRNA comprises deletion of at least a part of the tracrRNA-crRNA complementarity region in tracrRNA, deletion of at least a part of the tracrRNA-crRNA complementarity region in crRNA, or deletion of at least a part of the tracrRNA-crRNA complementarity region in both the tracrRNA and the crRNA.

In another embodiment, the engineered gRNA comprises a modification where a part of the tracrRNA-crRNA complementarity region is deleted, wherein the part of the complementary region to be deleted may consist of 1 to 54 nucleotides.

In yet another embodiment, the engineered gRNA comprises a modification where the entire tracrRNA-crRNA complementarity region is deleted, wherein the entire complementary region to be deleted may consist of 55 nucleotides.

Specifically, at least the part of the tracrRNA-crRNA complementarity region may consist of 3 to 55, 5 to 55, 7 to 55, 9 to 55, 11 to 55, 13 to 55, 15 to 55, 17 to 55, 19 to 55, 21 to 55, 23 to 55, 25 to 55, 27 to 55, 29 to 55, 31 to 55, 33 to 55, 35 to 55, 37 to 55, 39 to 55, or 41 to 55 nucleotides, preferably 42 to 55, 43 to 55, 44 to 55, 45 to 55, 46 to 55, 47 to 55, 48 to 55, 49 to 55, 50 to 55, 51 to 55, 52 to 55, 53 to 55, or 54 or 55 nucleotides.

In another embodiment, the engineered gRNA may be gRNA consisting of a sequence represented by Formula (I) or having at least 80%, at least 85%, at least 90%, or at least 95% sequence identity therewith. Here, MS4 or the tracrRNA-crRNA complementarity region is a region corresponding to the polynucleotide indicated by X^{c1} and X^{c2} in Formula (I), in which due to the modification where at least a part of the tracrRNA-crRNA complementarity region is deleted, X^{c1} and X^{c2} may each independently consist of 0 to 35 (poly)nucleotides.

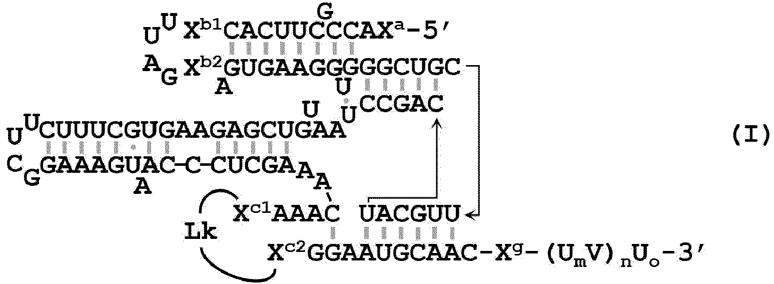

Preferably, X^{c1} may consist of 0 to 28, 0 to 27, 0 to 26, 0 to 25, 0 to 24, 0 to 23, 0 to 22, 0 to 21, 0 to 20, 0 to 19, 0 to 18, 0 to 17, 0 to 16, 0 to 15, 0 to 14, 0 to 13, 0 to 12, 0 to 11, 0 to 10, 0 to 9, 0 to 8, 0 to 7, 0 to 6, 0 to 5, 0 to 4, 0 to 3, 0 to 2, or 1 or 0 (poly)nucleotides. In addition, preferably, X^{c2} may consist of 0 to 27, 0 to 26, 0 to 25, 0 to 24, 0 to 23, 0 to 22, 0 to 21, 0 to 20, 0 to 19, 0 to 18, 0 to 17, 0 to 16, 0 to 15, 0 to 14, 0 to 13, 0 to 12, 0 to 11, 0 to 10, 0 to 9, 0 to 8, 0 to 7, 0 to 6, 0 to 5, 0 to 4, 0 to 3, 0 to 2, 1 or 0 (poly)nucleotides.

In an embodiment, in the engineered gRNA of Formula (I), X^{c1} may comprise the nucleotide sequence of SEQ ID NO: 39 or a nucleotide sequence having the sequence of SEQ ID NO: 39 from which 1 to 28 nucleotides are deleted. Preferably, the nucleotide deletion may involve sequential removal of at least 1, 2, 3, 5, 6, 7, 8, 9, 10, 11, 12, 13, 14, 15, 16, 17, 18, 19, 20, 21, 22, 23, 24, 25, 26, 27, or 28 nucleotides from the 5'-end of the sequence of SEQ ID NO: 39. More specifically, X^{c1} may comprise or consist of UUCAUUUUUCCUCUCCAAUUCUGCACAA-3' (SEQ ID NO: 39), 5'-UUCAUUUUUCCUCUCCAAUUCUGCACA-3' (SEQ ID NO: 40), 5'-UUCAUUUUUCCUCUCCAAUUCUGCAC-3' (SEQ ID NO: 41), 5'-UUCAUUUUUCCUCUCCAAUUCUGCA-3' (SEQ ID NO: 42), 5'-UUCAUUUUUCCUCUCCAAUUCUGC-3' (SEQ ID NO: 43), 5'-UUCAUUUUUCCUCUCCAAUUCUG-3' (SEQ ID NO: 44), 5'-UUCAUUUUUCCUCUCCAAUUCU-3' (SEQ ID NO: 45), 5'-UUCAUUUUUCCUCUCCAAUUC-3' (SEQ ID NO: 46), 5'-UUCAUUUUUCCUCUCCAAUU-3' (SEQ ID NO: 47), 5'-UUCAUUUUUCCUCUCCAAU-3' (SEQ ID NO: 48), 5'-UUCAUUUUUCCUCUCCAA-3' (SEQ ID NO: 49), 5'-UUCAUUUUUCCUCUCCA-3' (SEQ ID NO: 50), 5'-UUCAUUUUUCCUCUCC-3' (SEQ ID NO: 51), 5'-UUCAUUUUUCCUCUC-3' (SEQ ID NO: 52), 5'-UUCAUUUUUCCUCU-3' (SEQ ID NO: 53), 5'-UUCAUUUUUCCUC-3' (SEQ ID NO: 54), 5'-UUCAUUUUUCCU-3' (SEQ ID NO: 55), 5'-UUCAUUUUUCC-3' (SEQ ID NO: 56), 5'-UUCAUUUUUC-3' (SEQ ID NO: 57), 5'-UUCAUUUUU-3', 5'-UUCAUUUU-3', 5'-UUCAUUU-3', 5'-UUCAUU-3', 5'-UUCAU-3', 5'-UUCA-3', 5'-UUC-3', 5'-UU-3', or 5'-U-3', or X^{c1} may be absent.

Here, in a case where there is a region containing 3, 4, or 5 or more uracil (U) residues in the sequence of X^{c1} from which some nucleotides have been removed, the modification at MS1 as described above may also apply. For details about MS1, see the section "(4-2) Modification at modification site 1 (MS1)."

In yet another embodiment, in the engineered gRNA of Formula (I), X^{c2} may comprise the nucleotide sequence of SEQ ID NO: 58 or a nucleotide sequence having the sequence of SEQ ID NO: 58 from which 1 to 27 nucleotides are deleted. Preferably, the nucleotide deletion may involve sequential removal of at least 1, 2, 3, 5, 6, 7, 8, 9, 10, 11, 12, 13, 14, 15, 16, 17, 18, 19, 20, 21, 22, 23, 24, 25, 26, or 27 nucleotides from the 5'-end of the sequence of SEQ ID NO: 58. More specifically, X^{c2} may comprise or consist of GUUGCAGAACCCGAAUAGACGAAUGAA-3' (SEQ ID NO: 58), 5'-UUGCAGAACCCGAAUAGACGAAUGAA-3' (SEQ ID NO: 59), 5'-UGCAGAACCCGAAUAGACGAAUGAA-3' (SEQ ID NO: 60), 5'-GCAGAACCCGAAUAGACGAAUGAA-3' (SEQ ID NO: 61), 5'-CAGAACCCGAAUAGACGAAUGAA-3' (SEQ ID NO: 62), 5'-AGAACCCGAAUAGACGAAUGAA-3' (SEQ ID NO: 63), 5'-GAACCCGAAUAGACGAAUGAA-3' (SEQ ID NO: 64), 5'-AACCCGAAUAGACGAAUGAA-3' (SEQ ID NO: 65), 5'-ACCCGAAUAGACGAAUGAA-3' (SEQ ID NO: 66), 5'-CCCGAAUAGACGAAUGAA-3' (SEQ ID NO: 67), 5'-CCGAAUAGACGAAUGAA-3' (SEQ ID NO: 68), 5'-CGAAUAGACGAAUGAA-3' (SEQ ID NO: 69), 5'-GAAUAGACGAAUGAA-3' (SEQ ID NO: 70), 5'-AAUAGACGAAUGAA-3' (SEQ ID NO: 71), 5'-AUAGACGAAUGAA-3' (SEQ ID NO: 72), 5'-UAGACGAAUGAA-3' (SEQ ID NO: 73), 5'-AGACGAAUGAA-3' (SEQ ID NO: 74), 5'-GACGAAUGAA-3' (SEQ ID NO: 75), 5'-ACGAAUGAA-3', 5'-CGAAUGAA-3', 5'-GAAUGAA-3', 5'-AAUGAA-3', 5'-AUGAA-3', 5'-UGAA-3', 5'-GAA-3', 5'-AA-3', or 5'-A-3', or X^{c2} may be absent.

Here, in a case where there is a sequence corresponding a sequence containing 3 or more, or 3, 4, or 5 or more uracil (U) residues in the sequence of X^{c2} from which some nucleotides have been removed, the modification at MS1 as described above may also apply. For details about MS1, see the section "(4-2) Modification at modification site 1 (MS1)."

In the engineered gRNA of Formula (I) as described above, the regions corresponding to X^{c1} and X^{c2} may be each independently modified. However, MS4 or the tracrRNA-crRNA complementarity region is a region where tracrRNA and crRNA form complementary bonds. For the tracrRNA and the crRNA to function as a dual guide RNA, it is preferable that the position and number of nucleotides to be deleted in each of X^{c1} and X^{c2} be identical or similar to each other. That is, in order to preserve complementarity, in a case of sequentially deleting nucleotides from the 3'-end of tracrRNA in MS4 (tracrRNA-crRNA complementarity region), it is preferable to sequentially delete nucleotides from the 5'-end of crRNA.

In some embodiments, in the engineered gRNA of Formula (I), the 3'-end of X^{c1} and the 5'-end of X^{c2} may be connected by a linker (Lk) so that the gRNA is modified into a single guide RNA (sgRNA) form. Lk is a sequence that physically or chemically connects tracrRNA and crRNA, and may be a polynucleotide sequence having a length of 1 to 30 nucleotides. In an embodiment, Lk may be a sequence of 1 to 5, 5 to 10, 10 to 15, 2 to 20, 15 to 20, 20 to 25, or 25 to 30 nucleotides. For example, Lk may be 5'-GAAA-3', but is not limited thereto. As another example, Lk may a linker that comprises or consists of 5'-UUAG-3', 5'-UGAAAA-3', 5'-UUGAAAAA-3', 5'-UUCGAAAGAA-3' (SEQ ID NO: 76), 5'-UUCAGAAAUGAA-3' (SEQ ID NO: 77), 5'-UUCAUGAAAAUGAA-3' (SEQ ID NO: 78), or 5'-UUCAUUGAAAAAUGAA-3' (SEQ ID NO: 79).

On the other hand, while it is possible to use a linker (Lk) to make sgRNA, it is also possible to directly connect the 3' end of tracrRNA, of which a partial sequence has been removed, to the 5' end of crRNA of which a partial sequence has been removed.

In another embodiment, in the engineered gRNA of Formula (I), a case where X^{c1}and X^{c2} are connected by a linker may be indicated by 5'-X^{c1}-Lk-X^{c2} as in Formula (I), and this may be, but is not limited to, any one nucleotide sequence selected from the group consisting of SEQ ID NOS: 80 to 86.

### (4-6) Modification at modification site 5 (MS5)

This section describes a modification at MS5. As described above, MS5 corresponds to a region located toward the 3'-end of tracrRNA, which is referred to as the second stem region. The second stem region may comprise nucleotides that form a stem structure within a complex of the guide RNA (gRNA) with the nucleic acid editing protein, and may comprise nucleotides adjacent thereto. Here, the stem structure is distinct from the stem included in the above-described first stem region.

In an embodiment, the engineered gRNA comprises a modification where at least a part of the second stem region is deleted.

In another embodiment, the engineered gRNA comprises deletion of at least a part of the second stem region, wherein the at least a part of the second stem region to be deleted may consist of 1 to 27 nucleotides. Specifically, the at least a part of the second stem region may consist of 2 to 27, 3 to 27, 4 to 27, 5 to 27, 6 to 27, 7 to 27, 8 to 27, 9 to 27, 10 to 27, 11 to 27, 12 to 27, 13 to 27, 14 to 27, 15 to 27, 16 to 27, 17 to 27, 18 to 27, 19 to 27, 20 to 27, 21 to 27, 22 to 27, 23 to 27, 24 to 27, 25 to 27, or 26 or 27 nucleotides.

In yet another embodiment, the engineered gRNA may be gRNAconsisting of a sequence represented by Formula (I) or having at least 80%, at least 85%, at least 90%, or at least 95% sequence identity therewith. Here, MS5 or the second stem region is a region comprising a (poly)nucleotide (comprising a loop of 5'-UUAG-3') that is adjacent to the polynucleotide indicated by X^{b1} and X^{b2} in Formula (I), in which due to the modification where at least the part of the second stem region is deleted, X^{b1} and X^{b2} may each independently consist of 0 to 35 (poly)nucleotides.

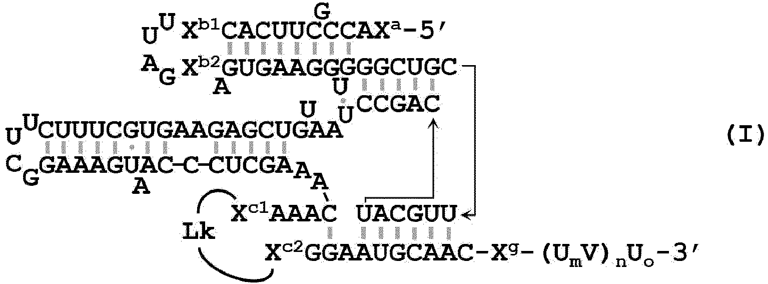

Preferably, X^{b1} may consist of 0 to 13, 0 to 12, 0 to 11, 0 to 10, 0 to 9, 0 to 8, 0 to 7, 0 to 6, 0 to 5, 0 to 4, 0 to 3, 0 to 2, or 1 or 0 (poly)nucleotides. In addition, preferably, X^{b2} may consist of 0 to 14, 0 to 13, 0 to 12, 0 to 11, 0 to 10, 0 to 9, 0 to 8, 0 to 7, 0 to 6, 0 to 5, 0 to 4, 0 to 3, 0 to 2, or 1 or 0 (poly)nucleotides.

In an embodiment, in the engineered gRNA of Formula (I), X^{b1} may comprise the nucleotide sequence of SEQ ID NO: 25 or a nucleotide sequence having the sequence of SEQ ID NO: 25 from which 1 to 13 nucleotides are deleted. Preferably, the nucleotide deletion may involve sequential removal of at least 1, 2, 3, 5, 6, 7, 8, 9, 10, 11, 12, or 13 nucleotides from the 5'-end of the sequence of SEQ ID NO: 25. More specifically, X^{b1} may comprise or consist of 5'-CAAAAGCUGUCCC-3' (SEQ ID NO: 25), 5'-CAAAAGCUGUCC-3' (SEQ ID NO: 26), 5'-CAAAAGCUGUC-3' (SEQ ID NO: 27), 5'-CAAAAGCUGU-3' (SEQ ID NO: 28), 5'-CAAAAGCUG-3', 5'-CAAAAGCU-3', 5'-CAAAAGC-3', 5'-CAAAAG-3', 5'-CAAAA-3', 5'-CAAA-3', 5'-CAA-3', 5'-CA-3', or 5'-C-3', or X^{b1} may be absent.

In another embodiment, in the engineered gRNA of Formula (I), X^{b2} may comprise the nucleotide sequence of SEQ ID NO: 29 or a nucleotide sequence having the sequence of SEQ ID NO: 29 from which 1 to 14 nucleotides are deleted. Preferably, the nucleotide deletion may involve sequential removal of at least 1, 2, 3, 5, 6, 7, 8, 9, 10, 11, 12, 13, or 14 nucleotides from the 5'-end of the sequence of SEQ ID NO: 29. More specifically, X^{b2} may comprise or consist of 5'-GGGAUUAGAACUUG-3' (SEQ ID NO: 29), 5'-GGAUUAGAACUUG-3' (SEQ ID NO: 30), 5'-GAUUAGAACUUG-3' (SEQ ID NO: 31), 5'-AUUAGAACUUG-3' (SEQ ID NO: 32), 5'-UUAGAACUUG-3' (SEQ ID NO: 33), 5'-UAGAACUUG-3', 5'-AGAACUUG-3', 5'-GAACUUG-3', 5'-AACUUG-3', 5'-ACUUG-3', 5'-CUUG-3', 5'-UUG-3', 5'-UG-3', or 5'-G-3', or X^{b1}may be absent.

In the engineered gRNA of Formula (I) as described above, the regions corresponding to X^{b1} and X^{b2} may be each independently modified. However, for normal preservation of the stem-loop structure, it is preferable that the position and number of nucleotides to be deleted in each of X^{b1} and X^{b2} be identical or similar to each other. For example, in a case of sequentially deleting nucleotides from the 5'-end direction in X^{b1}, it is preferable to sequentially delete nucleotides from the 3'-end direction in X^{b2}.

In yet another embodiment, a sequence of the loop portion connecting X^{b1} and X^{b2} in the engineered gRNA of Formula (I) is indicated by 5'-UUAG-3', and this may be replaced with another sequence such as 5'-NNNN-3' and '5-NNN-3', if necessary. Here, N is each independently A, C, G, or U. For example, the 5'-NNNN-3' may be 5'-GAAA-3', and the 5'-NNN-3' may be 5'-CGA-3'.

As an example, a sequence of the loop portion connecting X^{b1} and X^{b2} in the engineered gRNA of Formula (I) is 5'-UUAG-3', and the sequence 5'-X^{b1}UUAGX^{b2}-3' in Formula (I) may comprise or consist of any one nucleotide sequence selected from the group consisting of SEQ ID NOS: 34 to 38.

### (5) Additional sequence

The engineered tracrRNA of the present disclosure may optionally further comprise an additional sequence. The additional sequence may be located at the 3'-end of the engineered tracrRNA. In addition, the additional sequence may also be located at the 5'-end of the engineered tracrRNA. For example, the additional sequence may be located at the 5'-end of the first stem region.

The additional sequence may be 1 to 40 nucleotides. In an embodiment, the additional sequence may be any nucleotide sequence or a randomly arranged nucleotide sequence. For example, the additional sequence may be 5'-AUAAAGGUGA-3' (SEQ ID NO: 187).

In addition, the additional sequence may be a known nucleotide sequence. As an example, the additional sequence may be a hammerhead ribozyme nucleotide sequence. Here, the hammerhead ribozyme nucleotide sequence may be 5'-CUGAUGAGUCCGUGAGGACGAAACGAGUAAGCUCGUC-3' (SEQ ID NO: 188) or 5'-CUGCUCGAAUGAGCAAAGCAGGAGUGCCUGAGUAGUC-3' (SEQ ID NO: 189). The sequences listed above are merely examples, and the additional sequence is not limited thereto.

### (5) Examples of gRNA to which modifications at modification sites 1 to 5 have been applied

The engineered guide RNA (gRNA) included in the target nucleic acid editing system of the present disclosure may comprise modifications at two or more modification sites among the above-mentioned modification site 1 (MS 1) to modification site 5 (MS5).

In some embodiments, the engineered guide RNA may comprise at least one modification selected from the group consisting of (a1) deletion of at least a part of the first stem region; (a2) deletion of at least a part of the second stem region; (b) deletion of at least a part of the tracrRNA-crRNA complementarity region; (c) replacement of one or more uracil (U) residues with A, G, or C in three or more consecutive U residues when the consecutive U residues are present in the tracrRNA-crRNA complementarity region; and (d1) addition of a U-rich tail to the 3'-end of the crRNA sequence. The U-rich tail sequence may be represented by 5'-(UₘV)ₙUₒ-3', where V is each independently A, C, or G, m and o are integers between 1 and 20, and n is an integer between 0 and 5.

As an example, the engineered guide RNA may comprise (d1) addition of a U-rich tail to the 3'-end of the crRNA sequence and (c) replacement of one or more uracil (U) residues with A, G, or C in three or more consecutive U residues when the consecutive U residues are present in the tracrRNA-crRNA complementarity region.

As another example, the engineered guide RNA may comprise (d1) addition of a U-rich tail to the 3'-end of the crRNA sequence, (c) replacement of one or more uracil (U) residues with A, G, or C in three or more consecutive U residues when the consecutive U residues are present in the tracrRNA-crRNA complementarity region, and (a1) deletion of at least a part of the first stem region.

As yet another example, the engineered guide RNA may comprise (d1) addition of a U-rich tail to the 3'-end of the crRNA sequence, (c) replacement of one or more uracil (U) residues with A, G, or C in three or more consecutive U residues when the consecutive U residues are present in the tracrRNA-crRNA complementarity region, and (a1) deletion of at least a part of the first stem region.

As still yet another example, the engineered guide RNA may comprise (d1) addition of a U-rich tail to the 3'-end of the crRNA sequence, (a1) deletion of at least a part of the first stem region, and (b) deletion of at least a part of the tracrRNA-crRNA complementarity region, wherein the engineered guide RNA may further comprise replacement of one or more uracil (U) residues with A, G, or C in three or more consecutive U residues when the consecutive U residues are present in the tracrRNA-crRNA complementarity region containing partial deletion.

As still yet another example, the engineered guide RNA may comprise (d1) addition of a U-rich tail to the 3'-end of the crRNA sequence, (a1) deletion of at least a part of the first stem region, (b) deletion of at least a part of the tracrRNA-crRNA complementarity region, and (a2) deletion of at least a part of the second stem region wherein the engineered guide RNA may further comprise replacement of one or more uracil (U) residues with A, G, or C in three or more consecutive U residues when the consecutive U residues are present in the tracrRNA-crRNA complementarity region containing partial deletion.

As an example of tracrRNA to which modifications at the plurality of modification sites (MS) as described above have been applied, there is provided an engineered tracrRNA comprising the nucleotide sequence of any one of SEQ ID NOS: 87 to 132.

Specifically, the engineered tracrRNA of the present disclosure may comprise or consist of the nucleotide sequence of SEQ ID NO: 87 (MS1), SEQ ID NO: 88 (MS1/MS3-1), SEQ ID NO: 89 (MS1/MS3-2), SEQ ID NO: 90 (MS1/MS3-3), SEQ ID NO: 91 (MS1/MS4*-1), SEQ ID NO: 92 (MS1/MS4*-2), SEQ ID NO: 93 (MS1/MS4*-3), SEQ ID NO: 94 (MS1/MS5-1), SEQ ID NO: 95 (MS1/MS5-2), SEQ ID NO: 96 (MS1/MS5-3), SEQ ID NO: 97 (MS1/MS3-3/MS4*-1), SEQ ID NO: 98 (MS1/MS3-3/MS4*-2), SEQ ID NO: 99 (MS1/MS3-3/MS4*-3), SEQ ID NO: 100 (MS1/MS4*-2/MSS-1), SEQ ID NO: 101 (MS1/MS4*-2/MSS-2), SEQ ID NO: 102 (MS1/MS4*-2/MSS-3), SEQ ID NO: 103 (MS1/MS3-3/MS5-1), SEQ ID NO: 104 (MS1/MS3-3/MS5-2), SEQ ID NO: 105 (MS1/MS3-3/MS5-3), SEQ ID NO: 106 (MS1/MS3-3/MS4*-2/MS5-3), SEQ ID NO: 107 (mature form, MF), SEQ ID NO: 108 (MF/MS3-1), SEQ ID NO: 109 (MF/MS3-2), SEQ ID NO: 110 (MF/MS3-3), SEQ ID NO: 111 (MF/MS4-1), SEQ ID NO: 112 (MF/MS4-2), SEQ ID NO: 113 (MF/MS4-3), SEQ ID NO: 114 (MF/MS5-1), SEQ ID NO: 115 (MF/MS5-2), SEQ ID NO: 116 (MF/MS5-3), SEQ ID NO: 117 (MF/MS5), SEQ ID NO: 118 (MF/MS3-3/MS4-1), SEQ ID NO: 119 (MF/MS3-3/MS4-2), SEQ ID NO: 120 (MF/MS3-3/MS4-3), SEQ ID NO: 121 (MF/MS4-3/MS5-1), SEQ ID NO: 122 (MF/MS4-3/MS5-2), SEQ ID NO: 123 (MF/MS4-3/MS5-3), SEQ ID NO: 124 (MF/MS4-3/MS5-F), SEQ ID NO: 125 (MF/MS3-3/MS5-1), SEQ ID NO: 126 (MF/MS3-3/MS5-2), SEQ ID NO: 127 (MF/MS3-3/MS5-3), SEQ ID NO: 128 (MF/MS3-3/MS5), SEQ ID NO: 129 (MF/MS3-3/MS4-3/MS5-3), SEQ ID NO: 130 (MF/MS3-3/MS4-1/MS5), SEQ ID NO: 131 (MF/MS3-3/MS4-2/MS5), or SEQ ID NO: 132 (MF/MS3-3/MS4-3/MS5).

In some embodiments, exemplary sequences of the engineered tracrRNA, which has at least one modification at any one or more modification sites selected from MS1, MS3, MS4, and MS5, are shown in Table 2.

**[Table 2]**

| **tracrRNA** | **Nucleotide sequence** | **SEQ ID NO** |
|---|---|---|
| MS1 | CUUCACUGAUAAAGUGGAGAACCGCUUCACCAAAAGC UGUCCCUUAGGGGAUUAGAACUUGAGUGAAGGUGGGC UGCUUGCAUCAGCCUAAUGUCGAGAAGUGCUUUCUUC GGAAAGUAACCCUCGAAACAAAUUCAGUGCUCCUCUC CAAUUCUGCACAA | 87 |
| MS1/MS3-1 | GAUAAAGUGGAGAACCGCUUCACCAAAAGCUGUCCCU UAGGGGAUUAGAACUUGAGUGAAGGUGGGCUGCUUGC AUCAGCCUAAUGUCGAGAAGUGCUUUCUUCGGAAAGU AACCCUCGAAACAAAUUCAGUGCUCCUCUCCAAUUCU GCACAA | 88 |
| MS1/MS3-2 | UGGAGAACCGCUUCACCAAAAGCUGUCCCUUAGGGGA UUAGAACUUGAGUGAAGGUGGGCUGCUUGCAUCAGCC UAAUGUCGAGAAGUGCUUUCUUCGGAAAGUAACCCUC GAAACAAAUUCAGUGCUCCUCUCCAAUUCUGCACAA | 89 |
| MS1/MS3-3 | ACCGCUUCACCAAAAGCUGUCCCUUAGGGGAUUAGAA CUUGAGUGAAGGUGGGCUGCUUGCAUCAGCCUAAUGU CGAGAAGUGCUUUCUUCGGAAAGUAACCCUCGAAACA AAUUCAGUGCUCCUCUCCAAUUCUGCACAA | 90 |
| MS1/MS4^{*}-1 | CUUCACUGAUAAAGUGGAGAACCGCUUCACCAAAAGC UGUCCCUUAGGGGAUUAGAACUUGAGUGAAGGUGGGC UGCUUGCAUCAGCCUAAUGUCGAGAAGUGCUUUCUUC | 91 |
| | GGAAAGUAACCCUCGAAACAAAUUCAGUGCUCCUCUC CAAUUC | |
| MS1/MS4^{*}-2 | CUUCACUGAUAAAGUGGAGAACCGCUUCACCAAAAGC UGUCCCUUAGGGGAUUAGAACUUGAGUGAAGGUGGGC UGCUUGCAUCAGCCUAAUGUCGAGAAGUGCUUUCUUC GGAAAGUAACCCUCGAAACAAAUUCAGUGCUCCUCUC | 92 |
| MS1/MS4^{*}-3 | CUUCACUGAUAAAGUGGAGAACCGCUUCACCAAAAGC UGUCCCUUAGGGGAUUAGAACUUGAGUGAAGGUGGGC UGCUUGCAUCAGCCUAAUGUCGAGAAGUGCUUUCUUC GGAAAGUAACCCUCGAAACAAAUUCAGUGCU | 93 |
| MS1/MS5-1 | CUUCACUGAUAAAGUGGAGAACCGCUUCACCAAAAGC UGUUUAGAUUAGAACUUGAGUGAAGGUGGGCUGCUU GCAUCAGCCUAAUGUCGAGAAGUGCUUUCUUCGGAAA GUAACCCUCGAAACAAAUUCAGUGCUCCUCUCCAAUU CUGCACAA | 94 |
| MS1/MS5-2 | CUUCACUGAUAAAGUGGAGAACCGCUUCACCAAAAGC UUAGGAACUUGAGUGAAGGUGGGCUGCUUGCAUCAGC CUAAUGUCGAGAAGUGCUUUCUUCGGAAAGUAACCCU CGAAACAAAUUCAGUGCUCCUCUCCAAUUCUGCACAA | 95 |
| MS1/MS5-3 | CUUCACUGAUAAAGUGGAGAACCGCUUCACCAAUUAG UUGAGUGAAGGUGGGCUGCUUGCAUCAGCCUAAUGUC GAGAAGUGCUUUCUUCGGAAAGUAACCCUCGAAACAA AUUCAGUGCUCCUCUCCAAUUCUGCACAA | 96 |
| MS1/MS3-3/MS4^{*}-1 | ACCGCUUCACCAAAAGCUGUCCCUUAGGGGAUUAGAA CUUGAGUGAAGGUGGGCUGCUUGCAUCAGCCUAAUGU CGAGAAGUGCUUUCUUCGGAAAGUAACCCUCGAAACA AAUUCAGUGCUCCUCUCCAAUUC | 97 |
| MS1/MS3-3/MS4^{*}-2 | ACCGCUUCACCAAAAGCUGUCCCUUAGGGGAUUAGAA CUUGAGUGAAGGUGGGCUGCUUGCAUCAGCCUAAUGU CGAGAAGUGCUUUCUUCGGAAAGUAACCCUCGAAACA | 98 |
| | AAUUCAGUGCUCCUCUC | |
| MS1/MS3-3/MS4^{*}-3 | ACCGCUUCACCAAAAGCUGUCCCUUAGGGGAUUAGAA CUUGAGUGAAGGUGGGCUGCUUGCAUCAGCCUAAUGU CGAGAAGUGCUUUCUUCGGAAAGUAACCCUCGAAACA AAUUCAGUGCU | 99 |
| MS1/MS4^{*}-2/MS5-1 | CUUCACUGAUAAAGUGGAGAACCGCUUCACCAAAAGC UGUUUAGAUUAGAACUUGAGUGAAGGUGGGCUGCUU GCAUCAGCCUAAUGUCGAGAAGUGCUUUCUUCGGAAA GUAACCCUCGAAACAAAUUCAGUGCUCCUCUC | 100 |
| MS1/MS4^{*}-2/MS5-2 | CUUCACUGAUAAAGUGGAGAACCGCUUCACCAAAAGC UUAGGAACUUGAGUGAAGGUGGGCUGCUUGCAUCAGC CUAAUGUCGAGAAGUGCUUUCUUCGGAAAGUAACCCU CGAAACAAAUUCAGUGCUCCUCUC | 101 |
| MS1/MS4^{*}-2/MS5-3 | CUUCACUGAUAAAGUGGAGAACCGCUUCACCAAUUAG UUGAGUGAAGGUGGGCUGCUUGCAUCAGCCUAAUGUC GAGAAGUGCUUUCUUCGGAAAGUAACCCUCGAAACAA AUUCAGUGCUCCUCUC | 102 |
| MS1/MS3-3/MS5-1 | ACCGCUUCACCAAAAGCUGUUUAGAUUAGAACUUGAG UGAAGGUGGGCUGCUUGCAUCAGCCUAAUGUCGAGAA GUGCUUUCUUCGGAAAGUAACCCUCGAAACAAAUUCA GUGCUCCUCUCCAAUUCUGCACAA | 103 |
| MS1/MS3-3/MS5-2 | ACCGCUUCACCAAAAGCUUAGGAACUUGAGUGAAGGU GGGCUGCUUGCAUCAGCCUAAUGUCGAGAAGUGCUUU CUUCGGAAAGUAACCCUCGAAACAAAUUCAGUGCUCC UCUCCAAUUCUGCACAA | 104 |
| MS1/MS3-3/MS5-3 | ACCGCUUCACCAAUUAGUUGAGUGAAGGUGGGCUGCU UGCAUCAGCCUAAUGUCGAGAAGUGCUUUCUUCGGAA AGUAACCCUCGAAACAAAUUCAGUGCUCCUCUCCAAU UCUGCACAA | 105 |
| MS1/MS3-3/MS4^{*}-2/MS5-3 | ACCGCUUCACCAAUUAGUUGAGUGAAGGUGGGCUGCU UGCAUCAGCCUAAUGUCGAGAAGUGCUUUCUUCGGAA AGUAACCCUCGAAACAAAUUCAGUGCUCCUCUC | 106 |
| Mature Form(MF) | CUUCACUGAUAAAGUGGAGAACCGCUUCACCAAAAGC UGUCCCUUAGGGGAUUAGAACUUGAGUGAAGGUGGGC UGCUUGCAUCAGCCUAAUGUCGAGAAGUGCUUUCUUC GGAAAGUAACCCUCGAAACAAAUUCAUUU | 107 |
| MF/MS3-1 | GAUAAAGUGGAGAACCGCUUCACCAAAAGCUGUCCCU UAGGGGAUUAGAACUUGAGUGAAGGUGGGCUGCUUGC AUCAGCCUAAUGUCGAGAAGUGCUUUCUUCGGAAAGU AACCCUCGAAACAAAUUCAUUU | 108 |
| MF/MS3-2 | UGGAGAACCGCUUCACCAAAAGCUGUCCCUUAGGGGA UUAGAACUUGAGUGAAGGUGGGCUGCUUGCAUCAGCC UAAUGUCGAGAAGUGCUUUCUUCGGAAAGUAACCCUC GAAACAAAUUCAUUU | 109 |
| MF/MS3-3 | ACCGCUUCACCAAAAGCUGUCCCUUAGGGGAUUAGAA CUUGAGUGAAGGUGGGCUGCUUGCAUCAGCCUAAUGU CGAGAAGUGCUUUCUUCGGAAAGUAACCCUCGAAACA AAUUCAUUU | 110 |
| MF/MS4-1 | CUUCACUGAUAAAGUGGAGAACCGCUUCACCAAAAGC UGUCCCUUAGGGGAUUAGAACUUGAGUGAAGGUGGGC UGCUUGCAUCAGCCUAAUGUCGAGAAGUGCUUUCUUC GGAAAGUAACCCUCGAAACAAAUUCAU | 111 |
| MF/MS4-2 | CUUCACUGAUAAAGUGGAGAACCGCUUCACCAAAAGC UGUCCCUUAGGGGAUUAGAACUUGAGUGAAGGUGGGC UGCUUGCAUCAGCCUAAUGUCGAGAAGUGCUUUCUUC GGAAAGUAACCCUCGAAACAAAUUC | 112 |
| MF/MS4-3 | CUUCACUGAUAAAGUGGAGAACCGCUUCACCAAAAGC UGUCCCUUAGGGGAUUAGAACUUGAGUGAAGGUGGGC UGCUUGCAUCAGCCUAAUGUCGAGAAGUGCUUUCUUC | 113 |
| | GGAAAGUAACCCUCGAAACAAA | |
| MF/MS5-1 | CUUCACUGAUAAAGUGGAGAACCGCUUCACCAAAAGC UGUUUAGAUUAGAACUUGAGUGAAGGUGGGCUGCUU GCAUCAGCCUAAUGUCGAGAAGUGCUUUCUUCGGAAA GUAACCCUCGAAACAAAUUCAUUU | 114 |
| MF/MS5-2 | CUUCACUGAUAAAGUGGAGAACCGCUUCACCAAAAGC UUUAGAGAACUUGAGUGAAGGUGGGCUGCUUGCAUCA GCCUAAUGUCGAGAAGUGCUUUCUUCGGAAAGUAACC CUCGAAACAAAUUCAUUU | 115 |
| MF/MS5-3 | CUUCACUGAUAAAGUGGAGAACCGCUUCACCAAUUAG UUGAGUGAAGGUGGGCUGCUUGCAUCAGCCUAAUGUC GAGAAGUGCUUUCUUCGGAAAGUAACCCUCGAAACAA AUUCAUUU | 116 |
| MF/MS5 | CUUCACUGAUAAAGUGGAGAACCGCUUCACUUAGAGU GAAGGUGGGCUGCUUGCAUCAGCCUAAUGUCGAGAAG UGCUUUCUUCGGAAAGUAACCCUCGAAACAAAUUCAU UU | 117 |
| MF/MS3-3/MS4-1 | ACCGCUUCACCAAAAGCUGUCCCUUAGGGGAUUAGAA CUUGAGUGAAGGUGGGCUGCUUGCAUCAGCCUAAUGU CGAGAAGUGCUUUCUUCGGAAAGUAACCCUCGAAACA AAUUCAU | 118 |
| MF/MS3-3/MS4-2 | ACCGCUUCACCAAAAGCUGUCCCUUAGGGGAUUAGAA CUUGAGUGAAGGUGGGCUGCUUGCAUCAGCCUAAUGU CGAGAAGUGCUUUCUUCGGAAAGUAACCCUCGAAACA AAUUC | 119 |
| MF/MS3-3/MS4-3 | ACCGCUUCACCAAAAGCUGUCCCUUAGGGGAUUAGAA CUUGAGUGAAGGUGGGCUGCUUGCAUCAGCCUAAUGU CGAGAAGUGCUUUCUUCGGAAAGUAACCCUCGAAACA AA | 120 |
| MF/MS4-3/MS5-1 | CUUCACUGAUAAAGUGGAGAACCGCUUCACCAAAAGC UGUUUAGAUUAGAACUUGAGUGAAGGUGGGCUGCUU GCAUCAGCCUAAUGUCGAGAAGUGCUUUCUUCGGAAA GUAACCCUCGAAACAAA | 121 |
| MF/MS4-3/MS5-2 | CUUCACUGAUAAAGUGGAGAACCGCUUCACCAAAAGC UUUAGAGAACUUGAGUGAAGGUGGGCUGCUUGCAUCA GCCUAAUGUCGAGAAGUGCUUUCUUCGGAAAGUAACC CUCGAAACAAA | 122 |
| MF/MS4-3/MS5-3 | CUUCACUGAUAAAGUGGAGAACCGCUUCACCAAUUAG UUGAGUGAAGGUGGGCUGCUUGCAUCAGCCUAAUGUC GAGAAGUGCUUUCUUCGGAAAGUAACCCUCGAAACAA A | 123 |
| MF/MS4-3/MS5 | CUUCACUGAUAAAGUGGAGAACCGCUUCACUUAGAGU GAAGGUGGGCUGCUUGCAUCAGCCUAAUGUCGAGAAG UGCUUUCUUCGGAAAGUAACCCUCGAAACAAA | 124 |
| MF/MS3-3/MS5-1 | ACCGCUUCACCAAAAGCUGUUUAGAUUAGAACUUGAG UGAAGGUGGGCUGCUUGCAUCAGCCUAAUGUCGAGAA GUGCUUUCUUCGGAAAGUAACCCUCGAAACAAAUUCA UUU | 125 |
| MF/MS3-3/MS5-2 | ACCGCUUCACCAAAAGCUUUAGAGAACUUGAGUGAAG GUGGGCUGCUUGCAUCAGCCUAAUGUCGAGAAGUGCU UUCUUCGGAAAGUAACCCUCGAAACAAAUUCAUUU | 126 |
| MF/MS3-3/MS5-3 | ACCGCUUCACCAAUUAGUUGAGUGAAGGUGGGCUGCU UGCAUCAGCCUAAUGUCGAGAAGUGCUUUCUUCGGAA AGUAACCCUCGAAACAAAUUCAUUU | 127 |
| MF/MS3-3/MS5 | ACCGCUUCACUUAGAGUGAAGGUGGGCUGCUUGCAUC AGCCUAAUGUCGAGAAGUGCUUUCUUCGGAAAGUAAC CCUCGAAACAAAUUCAUUU | 128 |
| MF/MS3-3/MS4-3/MS5-3 | ACCGCUUCACCAAUUAGUUGAGUGAAGGUGGGCUGCU | 129 |
| | UGCAUCAGCCUAAUGUCGAGAAGUGCUUUCUUCGGAA AGUAACCCUCGAAACAAA | |
| MF/MS3-3/MS4-1/MS5 | ACCGCUUCACUUAGAGUGAAGGUGGGCUGCUUGCAUC AGCCUAAUGUCGAGAAGUGCUUUCUUCGGAAAGUAAC CCUCGAAACAAAUUCAU | 130 |
| MF/MS3-3/MS4-2/MS5 | ACCGCUUCACUUAGAGUGAAGGUGGGCUGCUUGCAUC AGCCUAAUGUCGAGAAGUGCUUUCUUCGGAAAGUAAC CCUCGAAACAAAUUC | 131 |
| MF/MS3-3/MS4-3/MS5 | ACCGCUUCACUUAGAGUGAAGGUGGGCUGCUUGCAUC AGCCUAAUGUCGAGAAGUGCUUUCUUCGGAAAGUAAC CCUCGAAACAAA | 132 |

In addition, as an example of crRNA to which modifications at the plurality of modification sites (MS) as described above have been applied, there is provided an engineered crRNA comprising the nucleotide sequence of any one of SEQ ID NOS: 133 to 148.

Specifically, the engineered crRNA of the present disclosure may comprise or consist of the nucleotide sequence of SEQ ID NO: 133 (MS1), SEQ ID NO: 134 (MS1/MS4*-1), SEQ ID NO: 135 (MS1/MS4*-2), SEQ ID NO: 136 (MS1/MS4^{*}-3), SEQ ID NO: 137 (mature form; MF), SEQ ID NO: 138 (MF/MS4-1), SEQ ID NO: 139 (MF/MS4-2), SEQ ID NO: 140 (MF/MS4-3), SEQ ID NO: 141 (MS1/MS2), SEQ ID NO: 142 (MS1/MS2/MS4*-1), SEQ ID NO: 143 (MS1/MS2/MS4*-2), SEQ ID NO: 144 (MS1/MS2/MS4*-3), SEQ ID NO: 145 (MF/MS2), SEQ ID NO: 146 (MF/MS2/MS4-1), SEQ ID NO: 147 (MF/MS2/MS4-2), or SEQ ID NO: 148 (MF/MS2/MS4-3).

In some embodiments, exemplary sequences of the engineered crRNA, which has at least one modification at any one or more modification sites selected from MS1, MS2, and MS4, are shown in Table 3.

**[Table 3]**

| **crRNA** | **Nucleotide sequence** | **SEQ ID NO** |
|---|---|---|
| MS1 | GUUGCAGAACCCGAAUAGAGCAAUGAAGGAAUGCAAC | 133 |
| MS1/MS4^{*}-1 | GAACCCGAAUAGAGCAAUGAAGGAAUGCAAC | 134 |
| MS1/MS4^{*}-2 | GAAUAGAGCAAUGAAGGAAUGCAAC | 135 |
| MS1/MS4^{*}-3 | AGCAAUGAAGGAAUGCAAC | 136 |
| MF | GAAUGAAGGAAUGCAAC | 137 |
| MF/MS4-1 | AUGAAGGAAUGCAAC | 138 |
| MF/MS4-2 | GAAGGAAUGCAAC | 139 |
| MF/MS4-3 | GGAAUGCAAC | 140 |
| MS1/MS2 | GUUGCAGAACCCGAAUAGAGCAAUGAAGGAAUGCAAC NNNNNNNNNNNNNNNNNNNNUUUUAUUUUUU | 141 |
| MS1/MS2/MS4^{*} -1 | GAACCCGAAUAGAGCAAUGAAGGAAUGCAACNNNNNN NNNNNNNNNNNNNNUUUUAUUUUUU | 142 |
| MS1/MS2/MS4^{*} -2 | GAAUAGAGCAAUGAAGGAAUGCAACNNNNNNNNNNN NNNNNNNNNUUUUAUUUUUU | 143 |
| MS1/MS2/MS4^{*} -3 | AGCAAUGAAGGAAUGCAACNNNNNNNNNNNNNNNNN NNNUUUUAUUUUUU | 144 |
| MF/MS2 | GAAUGAAGGAAUGCAACNNNNNNNNNNNNNNNNNNNN NUUUUAUUUUUU | 145 |
| MF/MS2/MS4-1 | AUGAAGGAAUGCAACNNNNNNNNNNNNNNNNNU UUUAUUUUU | 146 |
| MF/MS2/MS4-2 | GAAGGAAUGCAACNNNNNNNNNNNNNNNNNNNNUUU UAUUUUUU | 147 |
| MF/MS2/MS4-3 | GGAAUGCAACNNNNNNNNNNNNNNNNNNNNNNUUUUAU UUUUU | 148 |

In Table 3, indication of a guide sequence (spacer) is omitted from all crRNA sequences unless necessary, and the sequence indicated by 'NNNNNNNNNNNNNNNNNNNN' refers to any guide sequence (spacer) that can hybridize with a target sequence in a target gene. The guide sequence may be appropriately designed by a person skilled in the art depending on a desired target gene and/or a target sequence in the target gene, as described above, and thus is not limited to a specific sequence of a particular length.

In another embodiment, the engineered gRNA may comprise tracrRNA comprising or consisting of any one nucleotide sequence selected from the group consisting of SEQ ID NOS: 87 to 132; and crRNA comprising or consisting of any one nucleotide sequence selected from the group consisting of SEQ ID NOS: 133 to 148.

In yet another embodiment, in a case where the engineered gRNA of the present disclosure is in a form of a single guide RNA (sgRNA), the engineered sgRNA may be sgRNA comprising or consisting of any one nucleotide sequence selected from the group consisting of SEQ ID NOS: 149 to 186.

Specifically, the engineered sgRNA may be sgRNA of SEQ ID NO: 149 comprising a modification at MS1, sgRNA of SEQ ID NO: 150 comprising modifications at MS1/MS2, sgRNA of SEQ ID NO: 151 comprising modifications at MS1/MS2/MS3, sgRNA of SEQ ID NO: 152 comprising modifications at MS2/MS3/MS4, or sgRNA of SEQ ID NO: 153 comprising modifications at MS2/MS3/MS4/MS5.

In still yet another embodiment, the engineered sgRNA may be sgRNA comprising or consisting of the nucleotide sequence of SEQ ID NO: 154 (MS1/MS3-1), SEQ ID NO: 155 (MS1/MS3-2), SEQ ID NO: 156 (MS1/MS3-3), SEQ ID NO: 157 (MS1/MS4*-1), SEQ ID NO: 158 (MS1/MS4*-2), SEQ ID NO: 159 (MS1/MS4*-3), SEQ ID NO: 160 (MS1/MS5-1), SEQ ID NO: 161 (MS1/MS5-2), SEQ ID NO: 162 (MS1/MS5-3), SEQ ID NO: 163 (MS1/MS2/MS4*-2), SEQ ID NO: 164 (MS1/MS3-3/MS4*-2), SEQ ID NO: 165 (MS1/MS2/MS5-3), SEQ ID NO: 166 (MS1/MS3-3/MS5-3), SEQ ID NO: 167 (MS1/MS4^{*}-2/MS5-3), SEQ ID NO: 168 (MS1/MS2/MS3-3/MS4*-2), SEQ ID NO: 169 (MS1/MS2/MS3-3/MS5-3), SEQ ID NO: 170 (MS1/MS2/MS4*-2/MSS-3), SEQ ID NO: 171 (MS1/MS3-3/MS4*-2/MS5-3), or SEQ ID NO: 172 (MS1/MS2/MS3-3/MS4*-2/MSS-3).

In addition, the sgRNA may be sgRNA comprising or consisting of the nucleotide sequence of SEQ ID NO: 173, which is a mature form (abbreviated as MF).

In still yet another embodiment, there is provided exemplary sgRNA obtained by partial modification of the nucleotide sequence of the MF sgRNA. Specifically, the MF sgRNA may be sgRNA comprising or consisting of the nucleotide sequence of SEQ ID NO: 174 (MS3-1), SEQ ID NO: 175 (MS3-2), SEQ ID NO: 176 (MS3-3), SEQ ID NO: 177 (MS4-1), SEQ ID NO: 178 (MS4-2), SEQ ID NO: 179 (MS4-3), SEQ ID NO: 180 (MS5-1), SEQ ID NO: 181 (MS5-2), SEQ ID NO: 182 (MS5-3), SEQ ID NO: 183 (MS3-3/MS4-3), SEQ ID NO: 184 (MS3-3/MS5-3), SEQ ID NO: 185 (MS4-3/MS5-3), or SEQ ID NO: 186 (MS3-3/MS4-3/MS5-3).

In a preferred embodiment, the engineered sgRNA may consist of the nucleotide sequence of SEQ ID NO: 150 (ge3.0), SEQ ID NO: 151 (ge4.0), or SEQ ID NO: 152 (ge4.1).

### (6) Examples of additional modification

According to another aspect of the present disclosure, additional modifications may be applied to gRNA in addition to the modifications at MS1 to MS5 as described above.

In some embodiments, the engineered guide RNA may consist of a sequence represented by Formula (II) or may have at least 90% sequence identity therewith.

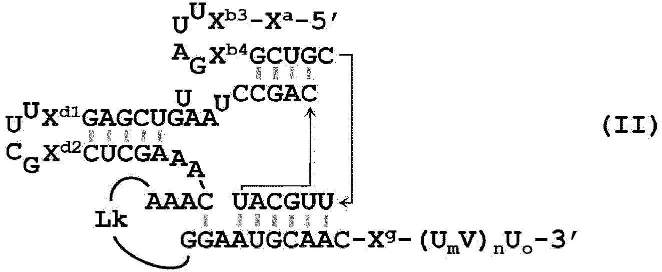

In Formula (II), X^{a}, X^{b3}, X^{b4}, X^{d1}, and X^{d2} each independently consists of 0 to 35 (poly)nucleotides, X^{g} is a guide sequence that consists of 10 to 30 nucleotides and is hybridizable with or complementary to a target sequence, Lk is a polynucleotide linker of 2 to 20 nucleotides or is absent, and (UₘV)ₙUₒ is present as a U-rich tail or absent, and when (UₘV)ₙUₒ is present, U is uridine, V is each independently A, C, or G, m and o are each independently an integer between 1 and 20, and n is an integer between 0 and 5.

In some embodiments, X^{b3} may comprise or consist of the sequence 5'-ACCGCUUCAC-3'. In addition, X^{b3} may comprise or consist of a sequence having the above sequence from which any 1 to 9 nucleotides are deleted, or X^{b3} may be absent.

In another embodiment, X^{b4} may comprise or consist of the sequence 5'-AGUGAAGGUGG-3'. In addition, X^{b4} may comprise or consist of a sequence having the above sequence from which any 1 to 10 nucleotides are deleted, or X^{b4} may be absent.

In yet another embodiment, X^{d1} may comprise or consist of the sequence 5'-AAGUGCUUUC-3'. In addition, X^{d1} may be a sequence having the above sequence from which any 1 to 9 nucleotides are deleted, or X^{d1} may be absent.

In still yet another embodiment, X^{d2} may comprise or consist of the sequence 5'-GAAAGUAACC-3'. In addition, X^{d2} may be a sequence having the above sequence from which any 1 to 9 nucleotides are deleted, or X^{d2} may be absent.

For details about X^{a}, X^{g}, Lk, and (UₘV)ₙUₒ, see the description above.

### (7) Chemical modification

In some embodiments, the engineered tracrRNA or engineered crRNA included in the engineered gRNA may have chemical modification in one or more nucleotides, if necessary. Here, the chemical modification may be a modification in various covalent bonds that may occur in a nucleotide base and/or sugar portion.

As an example, the chemical modification may be methylation, halogenation, acetylation, phosphorylation, phosphorothioate (PS) linkage, locked nucleic acid (LNA), 2'-O-methyl 3'phosphorothioate (MS), or 2'-O-methyl 3'thioPACE (MSP). The above example is a simple example and the modification is not limited thereto.

In a case of using the hypercompact nucleic acid editing system of the present disclosure that comprises a complex of the engineered gRNA with the Cas12f1, TnpB, or a variant protein thereof, efficiency of cleavage and/or homology-directed repair for a target nucleic acid or target gene in a cell is significantly improved, as compared with a case of using the guide RNA found in nature.

Above all, the engineered gRNA may involve optimized length for high efficiency and resulting cost reduction in gRNA synthesis, creation of additional space or capacity in a case of being inserted into a viral vector, normal expression of tracrRNA, increased expression of operable gRNA, increased gRNA stability, increased stability of complex of gRNA with nucleic acid editing protein, induction of formation of complex of gRNA with nucleic acid editing protein at high efficiency, increased cleavage efficiency of target nucleic acid by hypercompact target nucleic acid editing system comprising complex of gRNA with nucleic acid editing protein, and increased efficiency of homology-directed repair for target nucleic acid by such a system. Accordingly, in a case of using the above-described engineered gRNA for Cas12f1, TnpB, or a variant protein thereof, it is possible to overcome the limitations of the above-mentioned prior art, thereby cleaving or editing a gene with high efficiency in a cell.

In addition, the engineered gRNAhas a shorter length as compared with the gRNAfound in nature, and thus has high applicability in the field of gene editing technology. Using the engineered gRNA, the hypercompact nucleic acid editing system comprising a complex of the gRNA with the nucleic acid editing protein has advantages of being very small in size and having excellent editing efficiency, which allows the system to be utilized in various gene editing technologies.

### 3. Donor nucleic acid molecule

The donor nucleic acid molecule is a sequence used as a template in homology-directed repair. The donor nucleic acid molecule comprises at least one donor sequence. In some embodiments, a desired sequence in the donor nucleic acid molecule may correspond to an endogenous or native chromosomal sequence. The desired sequence may be essentially identical to a target region (target sequence) or a region adjacent thereto, and may comprise at least one nucleotide change. Accordingly, the desired sequence may comprise a modified version of the wild-type sequence at a targeted region such that upon being integrated into or exchanged with a native sequence, the sequence comprises at least one nucleotide change at the targeted region. For example, the change may be insertion of one or more nucleotides, deletion of one or more nucleotides, substitution of one or more nucleotides, or a combination thereof. As a result of integration of the modified sequence, the cell or embryo/animal can produce a modified gene product from the targeted sequence.

In another embodiment, the desired sequence in the donor nucleic acid molecule corresponds to an exogenous sequence. As used in the embodiments of the present disclosure, the "exogenous" sequence refers to a sequence that is not native to the cell or embryo, or to a sequence whose location in the cell's genome is different from its native location. For example, the exogenous sequence may comprise a protein-coding sequence that can be operably linked to an exogenous promoter control sequence such that upon being integrated into the genome, the cell can express the protein encoded by the integrated sequence. Alternatively, the exogenous sequence may be integrated into a chromosomal sequence such that expression of the exogenous sequence is regulated by an endogenous promoter control sequence. In other iterations, the exogenous sequence may be a transcription control sequence, another expression control sequence, an RNA coding sequence, or the like. Integration of the exogenous sequence into a chromosomal sequence is called "knock-in."

As will be appreciated by those skilled in the art, the donor nucleic acid molecule or desired sequence may and will vary in length. For example, the donor nucleic acid molecule or desired sequence may vary in length from several nucleotides to hundreds of nucleotides to hundreds of thousands of nucleotides. For example, the donor nucleic acid molecule or desired sequence may be at least 1 bp, 10 bp, 100 bp, or 200 bp or more in length. For example, the donor nucleic acid molecule may be 1 bp to 20 kb, 100 bp to 50 kb, 100 bp to 30 kb, 100 bp to 10 kb, 100 bp to 10 kb, 200 bp to 50 kb, 200 bp to 30 kb, or 200 bp to 10 kb in length. In an embodiment, the donor nucleic acid molecule or desired sequence may be 1 bp to 20 kb, 1 bp to 10 kb, 1 bp to 8 kb, 1 bp to 6 kb, or 1 bp to 4 kb in length.

The donor nucleic acid molecule may comprise upstream and downstream sequences. In some embodiments, the desired sequence in the donor nucleic acid molecule is flanked by upstream and downstream sequences that have substantial sequence identity with sequences located upstream and downstream of a targeted region, respectively, within the chromosomal sequence. Due to such sequence similarity, the sequences upstream and downstream of the donor nucleic acid molecule allow homologous recombination between the donor nucleic acid molecule and the targeted chromosomal sequence such that the desired sequence can be integrated into (or exchanged with) the chromosomal sequence.

The upstream sequence, as used herein, refers to a nucleotide sequence that shares substantial sequence identity with a chromosomal sequence upstream of the targeted region. Similarly, the downstream sequence refers to a nucleotide sequence that shares substantial sequence identity with a chromosomal sequence downstream of the targeted region. As used herein, the phrase "substantial sequence identity" refers to a sequence having at least about 75% sequence identity. Therefore, the upstream and downstream sequences in the donor nucleic acid molecule may have about 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity with the sequences upstream or downstream of the targeted region. In exemplary embodiments, the upstream and downstream sequences in the donor nucleic acid molecule may have about 95% or 100% sequence identity with the chromosomal sequences upstream or downstream of the targeted region. In an embodiment, the upstream sequence shares substantial sequence identity with a chromosomal sequence located immediately upstream of the targeted region (that is, adjacent to the targeted region). In another embodiment, the upstream sequence shares substantial sequence identity with a chromosomal sequence located within about 100 nucleotides upstream from the targeted region. Thus, by way of example, the upstream sequence may share substantial sequence identity with a chromosomal sequence located within about 1 to about 20, about 21 to about 40, about 41 to about 60, about 61 to about 80, or about 81 to about 100 nucleotides upstream from the targeted region. In an embodiment, the downstream sequence shares substantial sequence identity with a chromosomal sequence located immediately downstream of the targeted region (that is, adjacent to the targeted region). In another embodiment, the downstream sequence shares substantial sequence identity with a chromosomal sequence located within about 100 nucleotides downstream from the targeted region. Thus, by way of example, the downstream sequence may share substantial sequence identity with a chromosomal sequence located within about 1 to about 20, about 21 to about 40, about 41 to about 60, about 61 to about 80, or about 81 to about 100 nucleotides downstream from the targeted region.

Each upstream or downstream sequence may vary in length from about 20 nucleotides to about 5000 nucleotides. In some embodiments, the upstream and downstream sequences may comprise about 50, 100, 200, 300, 400, 500, 600, 700, 800, 900, 1000, 1100, 1200, 1300, 1400, 1500, 1600, 1700, 1800, 1900, 2000, 2100, 2200, 2300, 2400, 2500, 2600, 2800, 3000, 3200, 3400, 3600, 3800, 4000, 4200, 4400, 4600, 4800, or 5000 nucleotides. In exemplary embodiments, the upstream and downstream sequences may vary in length from about 50 to about 1500 nucleotides.

The donor nucleic acid molecule, which comprises upstream and downstream sequences having sequence similarity to a targeted chromosomal sequence, may be linear or circular. In embodiments where the donor nucleic acid molecule is circular, it may be part of a vector. For example, the vector may be a plasmid vector.

The donor nucleic acid molecule may comprise a targeted cleavage site. In another embodiment, the donor nucleic acid molecule may additionally comprise at least one targeted cleavage site that is recognized by an RNA-guided endonuclease. The targeted cleavage site added to the donor nucleic acid molecule may be placed upstream or downstream or both upstream and downstream of the desired sequence. For example, the desired sequence, upon cleavage by an RNA-guided endonuclease, may be flanked by targeted cleavage sites such that the desired sequence is flanked by overhangs that are compatible with those in the chromosomal sequence generated upon cleavage by the RNA-guided endonuclease. Accordingly, the desired sequence may be ligated with the cleaved chromosomal sequence during repair of double stranded breaks by a nonhomologous repair process. Typically, the donor nucleic acid molecule comprising the targeted cleavage site will be circular (for example, may be part of a plasmid vector).

The donor nucleic acid molecule may comprise a short desired sequence with optional overhangs. In another alternative embodiment, the donor nucleic acid molecule may be a linear molecule comprising a short desired sequence with optional short overhangs that are compatible with the overhangs generated by an RNA-guided endonuclease. In such embodiments, the desired sequence may be ligated directly with the cleaved chromosomal sequence during repair of the double-stranded breaks. In some cases, the desired sequence may be less than about 1,000, less than about 500, less than about 250, or less than about 100 nucleotides. In certain cases, the donor nucleic acid molecule may be a linear molecule comprising a short donor sequence with blunt ends. In other iterations, the donor nucleic acid molecule may be a linear molecule comprising a short desired sequence with 5' and/or 3' overhangs. The overhangs may comprise 1, 2, 3, 4, or 5 nucleotides.

Typically, the donor nucleic acid molecule will be DNA. The DNA may be single-stranded or double-stranded and/or linear or circular. The donor nucleic acid molecule may be a DNA plasmid, a bacterial artificial chromosome (BAC), a yeast artificial chromosome (YAC), a viral vector, a linear piece of DNA, a PCR fragment, a naked nucleic acid, or a nucleic acid complexed with a delivery vehicle such as a liposome or poloxamer. In an embodiment, the donor nucleic acid molecule comprising the desired sequence may be part of a plasmid vector. In addition, in an embodiment, the donor nucleic acid molecule comprising the desired sequence may further comprise at least one additional sequence.

### 4. Molecule that inhibits non-homologous end joining

In addition to the nucleic acid editing protein (endonuclease), a guide RNA, and a donor nucleic acid molecule as described above, the target nucleic acid editing system of the present disclosure may further comprise various types of molecules to improve homology-directed repair efficiency.

In some embodiments, the target nucleic acid editing system may further comprise a molecule that inhibits expression of a gene involved in non-homologous end joining (NHEJ). Specifically, the system may further comprise a factor that can decrease activity of a gene or protein involved in non-homologous end joining, for example, a molecule that inhibits expression of a gene involved in non-homologous end joining. Without wishing to be bound by any particular theory, for example, decreased non-homologous end joining activity may result in promotion of a homologous repair-mediated pathway. The inhibitor may be used to decrease non-homologous end joining activity or to increase or decrease homologous directed repair activity. Such an inhibitor may include, for example, small molecules or inhibitory nucleic acids such as short interfering nucleic acids (for example, short interfering RNA (siRNA), double-stranded RNA (dsRNA), micro-RNA (miRNA), and short hairpin RNA (shRNA) specific for carcass) or an antisense oligonucleotide. The inhibitor may be targeted to enzymes involved in non-homologous end joining or homology-directed repair, or upstream regulation thereof, by post translational modification, for example, through phosphorylation, ubiquitination, and sumoylation.

In an embodiment, the gene involved in non-homologous end joining may be any one or more selected from the group consisting of ATM1, XRCC4, XLF, XRCC6, LIG4, and DCLRE1C.

According to an embodiment of the present disclosure, shRNAs for DCLRE1C, LIG4, XRCC4, XRCC6, XLF, and ATM genes were included in the nucleic acid editing system of the present disclosure to inhibit non-homologous end joining. As a result, significantly increased homology-directed repair efficiency was observed (see Example 3.1).

### 5. System/composition for editing target nucleic acid

According to another aspect of the present disclosure, there is provided a gene editing composition comprising the above-described target nucleic acid editing system. In addition, there is provided a vector system as described later or a gene editing composition comprising both the target nucleic acid editing system and the vector system.

In an embodiment, the gene editing composition of the present disclosure comprises an endonuclease comprising Cas12f1, TnpB, or a variant protein thereof, or a nucleic acid encoding the endonuclease; an engineered guide RNAcomprising a guide sequence or a nucleic acid encoding the guide RNA; and a donor nucleic acid molecule or a nucleic acid encoding the donor nucleic acid molecule.

For details about the "Cas12f1, TnpB, or a variant protein thereof, "engineered guide RNA," and "donor nucleic acid molecule", see the description above.

In another embodiment, the endonuclease comprising Cas12f1, TnpB, or a variant protein thereof, and the guide RNA, which are included in the target nucleic acid editing system or gene editing composition, may be included in a form of a ribonucleoprotein particle (RNP).

Meanwhile, it is obvious that the gene editing composition of the present disclosure may further comprise appropriate substances required for gene editing in addition to the respective components of the hypercompact nucleic acid editing system according to the present disclosure.

### III. Nucleic acids encoding components of target nucleic acid editing system for homology-directed repair

Each component of the target nucleic acid editing system provided by the present disclosure is intended to be expressed in a cell. Thus, according to another aspect of the present disclosure, there is provided a nucleic acid or polynucleotide encoding each component of the target nucleic acid editing system.

Specifically, the nucleic acid or polynucleotide comprises a nucleotide sequence that encodes a nucleic acid editing protein, a guide RNA, and/or a donor nucleic acid molecule included in the target nucleic acid editing system to be expressed. Here, a sequence of the nucleic acid or polynucleotide may comprise not only a nucleotide sequence encoding a wild-type gene editing protein and a wild-type guide RNA, but also a nucleotide sequence encoding an engineered guide RNA and/or a codon-optimized nucleic acid editing protein, a nucleotide sequence encoding an engineered nucleic acid editing protein, or a nucleotide sequence encoding a gene editing protein having lost or decreased DNA double-strand cleavage activity, depending on the intended purpose.

In the present disclosure, the nucleic acid or polynucleotide may comprise a sequence configured to express Cas12f1, TnpB, or a variant protein thereof which is a hypercompact nucleic acid editing protein. Here, the Cas12f1, TnpB, or the variant protein thereof may be a protein that has activity of cleaving double-stranded or single-stranded DNA.

In an embodiment, the nucleic acid or polynucleotide may comprise a sequence configured to express Cas12f1, TnpB, or a variant protein thereof. Here, the Cas12f1, TnpB, or the variant protein thereof may be a protein comprising an amino acid sequence having 70% or more sequence identity with an amino acid sequence selected from the group consisting of SEQ ID NOS: 1 to 5. For details on this, see the description above.

In addition, the nucleic acid or polynucleotide may comprise a sequence encoding Cas12f1, TnpB, or a variant protein thereof. Preferably, the nucleic acid or polynucleotide may comprise a human codon-optimized nucleotide sequence encoding Cas12f1, TnpB, or a variant protein thereof. "Codon optimization" refers to a process of modifying a native nucleic acid sequence for enhanced expression in a cell of interest by replacing at least one codon in the native sequence with a codon, which is used more frequently or most frequently in a gene of the target cell, while maintaining its native amino acid sequence. Different species have specific biases for specific codons of specific amino acids, and codon bias (differences in codon usage between organisms) is often correlated with translation efficiency of an mRNA, which is considered to be dependent on the nature of codons being translated and availability of specific tRNA molecules. Predominance of tRNA selected in a cell generally reflects the most frequently used codon in peptide synthesis. Thus, genes may be tailored for optimal gene expression in a given organism based on codon optimization.

In an embodiment, the nucleic acid encoding the TnpB protein may be a nucleic acid encoding a human codon-optimized TnpB protein. For example, the nucleic acid encoding the human codon-optimized TnpB protein may comprise the nucleotide sequence of SEQ ID NO: 6. As another example, the nucleic acid encoding the human codon-optimized Cas12f1 protein may comprise the nucleotide sequence of SEQ ID NO: 10. As yet another example, the nucleic acid encoding the variant protein of the human codon-optimized Cas12f1 or TnpB may comprise the nucleotide sequence of SEQ ID NO: 7 (TnpB-v1), SEQ ID NO: 8 (TnpB-v2), or SEQ ID NO: 9 (TnpB-v3).

In addition, the nucleic acid or polynucleotide may comprise a sequence encoding a modified version of Cas12f1, TnpB, or a variant protein thereof or a fusion protein of the Cas12f1, TnpB, or the variant protein thereof. In an embodiment, the nucleic acid or polynucleotide may comprise a sequence configured to express Cas12f1, TnpB, or a variant protein thereof that is altered to cleave only one strand of a double strand of a target nucleic acid. As an example, the modified version of Cas12f1, TnpB, or a variant protein thereof may be altered to cleave only one strand of the double strand of the target nucleic acid and to perform base editing or prime editing on a uncleaved strand. Alternatively, the nucleic acid or polynucleotide may comprise a sequence encoding a variant protein that has been altered to perform base editing, prime editing, or a gene expression regulatory function for a target nucleic acid.

In addition, the nucleic acid or polynucleotide may comprise a sequence configured to express a guide RNA (augment RNA) engineered to have the optimal targeting efficiency for Cas12f1, TnpB, or a variant thereof or one or more different engineered guide RNAs. As an example, the engineered guide RNA sequence may comprise a scaffold sequence, a spacer sequence, and a U-rich tail sequence. Specifically, the engineered gRNA sequence may comprise an altered tracrRNA sequence and/or an altered crRNA sequence, and may also comprise a U-rich tail sequence. For details about the engineered gRNA comprising a U-rich tail and modifications thereof, see the description above.

In addition, the nucleic acid or polynucleotide may comprise a donor nucleic acid molecule for use as a template in homology-directed repair. For details about the donor nucleic acid molecule, see the description above.

### IV. Vector system for expression of target nucleic acid editing system

According to still yet another aspect of the present disclosure, there is provided a vector system comprising at least one vector that comprises a first nucleic acid construct to which a nucleotide sequence encoding an endonuclease is operably linked, the endonuclease comprising Cas12f1, TnpB, or a variant protein thereof; a second nucleic acid construct to which a nucleotide sequence encoding an engineered guide RNA is operably linked, the engineered guide RNA comprising a guide sequence that binds complementarily to a target nucleic acid; and a third nucleic acid construct comprising a donor nucleic acid molecule.

In order to use the target nucleic acid editing system provided by the present disclosure for gene editing involving homology-directed repair, a method may be used in which a vector that comprises sequences encoding respective components of the target nucleic acid editing system is introduced directly or via a vehicle such as a virus into a target cell, and the respective components of the gene editing system are allowed to be expressed in the target cell.

In addition, in order to achieve excellent homology-directed repair efficiency using the nucleic acid editing system of the present disclosure for editing a target nucleic acid or target gene, it is preferable that respective components of the complex of the guide RNA with the Cas12f1, TnpB, or the variant protein thereof are operably linked to each other and incorporated into one vector. Here, the nucleic acid degrading protein or the guide molecule may be linked to an effector protein, if necessary, to make a protein in a fused form.

As an example, the protein in a fused form may comprise an orthogonal RNA-binding protein or adapter protein present within bacteriophage coat proteins. Here, the coat protein may include MS2, Qβ, F2, GA, fr, JP501, M12, R17, BZ13, JP34, JP500, KU1, M11, MX1, TW18, VK, SP, FI, ID2, NL95, TW19, AP205, ΦCb5, ΦCb8r, ΦCb12r, ΦCb23r, 7s, PRR1, and the like. In addition, the protein in a fused form may be delivered through one or more lipid nanoparticles.

In an embodiment, the hypercompact nucleic acid editing protein Cas12f1, TnpB, or a variant protein thereof and one or more guide RNAs, which correspond to the components of the target nucleic acid editing system of the present disclosure, may be delivered to a cell as one or more mRNA molecules encoding the same. Here, the mRNA molecule may be delivered through one or more lipid nanoparticles.

In addition, the components of the target nucleic acid editing system of the present disclosure may be in a form of one or more DNA molecules. Here, the one or more DNA molecules may comprise one or more regulatory elements operably configured to express a gene editing protein or guide molecule. The one or more regulatory elements may comprise an inducible promoter, if necessary.

In an embodiment, the nucleic acid constructs included in the vector system may be located in the same vector or different vectors.

The DNA molecules constituting the target nucleic acid editing system may be contained in one or more adeno-associated virus (AAV) vectors and delivered into a cell. Preferably, all of the DNA molecules may be contained in one adeno-associated virus (AAV) vector so that the DNA molecules constituting the target nucleic acid editing system within the AAV virus can be delivered into a cell in a packed form.

More specifically, the vector components, which allow the hypercompact gene editing system of the present disclosure to be expressed in a cell, include the following.

### 1. Nucleic acid constructs encoding components of target nucleic acid editing system

The final purpose of the vector system is to allow respective components of the target nucleic acid editing system of the present disclosure to be expressed in a cell. Thus, the sequence included in the vector system should necessarily comprise at least one of the nucleotide sequences that encode the respective components of the target nucleic acid editing system.

In an embodiment, the vector system may comprise a first nucleic acid construct to which a nucleotide sequence encoding an endonuclease is operably linked, the endonuclease comprising Cas12f1, TnpB, or a variant protein thereof; a second nucleic acid construct to which a nucleotide sequence encoding an engineered guide RNA is operably linked, the engineered guide RNA comprising a guide sequence that binds complementarily to a target nucleic acid; and a third nucleic acid construct comprising a donor nucleic acid molecule. Here, the first nucleic acid construct, the second nucleic acid construct, and/or the third nucleic acid construct may be located on the same vector or on different/separate vectors in the vector system. Here, the linkage may be made directly or through a linker.

In an embodiment, the nucleic acid construct may comprise a nucleic acid encoding an engineered guide RNA (gRNA). Here, the engineered gRNA may comprise an engineered tracrRNA and/or an engineered crRNA. Here, the engineered guide RNA may have the same configuration as the embodiment of the engineered guide RNA as described above.

In addition, in the nucleic acid construct, the nucleic acid editing protein Cas12f1, TnpB, or a variant protein thereof may be a protein that comprises an amino acid sequence having at least 70%, at least 75%, at least 80%, at least 85%, at least 90%, or at least 95% sequence identity with an amino acid sequence selected from the group consisting of SEQ ID NOS: 1 to 5. The nucleic acid construct may comprise a nucleic acid encoding the protein or a codon-optimized nucleic acid of the protein. As an example, the hypercompact nucleic acid editing protein may be a nucleic acid editing protein characterized by having at least 70%, at least 75%, at least 80%, at least 85%, at least 90%, or at least 95% sequence identity with an amino acid sequence selected from the group consisting of SEQ ID NOS: 1 to 5; and the codon-optimized nucleic acid encoding the same may be a human codon-optimized nucleic acid and may consist of any one nucleotide sequence selected from SEQ ID NOS: 6 to 10.

In addition, the nucleic acid construct may comprise at least one nuclear localization signal (NLS) or nuclear export signal (NES) sequence at the N-terminus or C-terminus. The NLS sequence refers to a peptide of a certain length or a sequence thereof that is attached to a substance to be transported into the cell nucleus by nuclear transport and acts as a type of "tag." The NES sequence refers to a peptide of a certain length or a sequence thereof that is attached to a substance to be transported outside the cell nucleus by nuclear transport and acts as a type of "tag." For example, the NLS may be, but is not limited to, an NLS sequence derived from the NLS of an SV40 virus large T-antigen; the c-myc NLS; the hRNPA1 M9 NLS; the NLS from a nucleoplasmin; the sequence of an IBB domain from importin alpha; the sequences of myoma T protein; the sequence of human p53; the sequence of mouse c-abI IV; the sequences of influenza virus NS 1; the sequence of hepatitis virus delta antigen; the sequence of mouse Mx1 protein; the sequence of human poly(ADP-ribose) polymerase; or the sequence of steroid hormone receptor (human) glucocorticoid.

The vector system comprises a nucleotide sequence encoding a guide RNA and/or a nucleic acid editing protein included in the nucleic acid editing system to be expressed. For details about the nucleotide sequence, see the description above.

The vector may be configured to express two or more different engineered guide RNAs. In an embodiment, the vector may be configured to express an engineered first guide RNA and an engineered second guide RNA. In an embodiment, the engineered first guide RNA sequence may comprise a first scaffold sequence, a first spacer sequence, and a first U-rich tail sequence, and the engineered second guide RNA sequence may comprise a second scaffold sequence, a second spacer sequence, and a second U-rich tail sequence.

In addition, besides the components of the target nucleic acid editing system as described above, the vector system may comprise a nucleotide sequence encoding an additional expression element that a person skilled in the art intends to express as needed.

As an example, the additional expression element may be a tag. Specifically, the additional expression element may be a herbicide resistance gene such as glyphosate, glufosinate ammonium or phosphinothricin, or an antibiotic resistance gene such as ampicillin, kanamycin, G418, bleomycin, hygromycin, or chloramphenicol.

In another embodiment, the vector system may further comprise at least one nucleic acid construct to which a nucleotide sequence encoding a molecule is operably linked, the molecule inhibiting expression of a gene involved in non-homologous end joining. Here, the gene involved in non-homologous end joining may be any one or more selected from the group consisting of ATM1, XRCC4, XLF, XRCC6, LIG4, and DCLRE1C. In addition, the molecule may be shRNA, siRNA, miRNA, or antisense oligonucleotide. For the molecule, reference is made to the section "4. Molecule that inhibits non-homologous end joining." For details about the "non-homologous end joining", see the description below.

### 2. Regulatory and/or control component

The vector system must comprise at least one regulatory and/or control component so that it is directly expressed in a cell. Specifically, the regulatory and/or control component may include, but is not limited to, a promoter, an enhancer, an intron, a polyadenylation signal, a Kozak consensus sequence, an internal ribosome entry site (IRES), a splice acceptor, a 2A sequence, and/or a replication origin. Here, the replication origin may be, but is not limited to, an f1 origin of replication, an SV40 origin of replication, a pMB 1 origin of replication, an adeno origin of replication, an AAV origin of replication, and/or a BBV origin of replication.

### 3. Promoter

In order to express, in a cell, the nucleotide sequences encoding the nucleic acid editing system of the present disclosure included in the vector system, a promoter sequence is operably linked to the sequence encoding each component so that an RNA transcription factor can be activated in the cell. The promoter sequence may be designed differently depending on the corresponding RNA transcription factor or expression environment, and is not limited as long as it can properly express the component of the nucleic acid editing system (TaRGET system) of the present disclosure in a cell.

As an example, the promoter sequence may be a promoter that promotes transcription of RNA polymerase RNA Pol I, Pol II, or Pol III. Specifically, the promoter may be one of U6 promoter, EFS promoter, EF1-α promoter, H1 promoter, 7SK promoter, CMV promoter, LTR promoter, Ad MLP promoter, HSV promoter, SV40 promoter, CBA promoter, or RSV promoter.

### 4. Termination signal

In a case where a sequence of the vector comprises the promoter sequence, transcription of a sequence operably linked to the promoter is induced by an RNA transcription factor. The vector may comprise a termination signal that induces termination of transcription of the RNA transcription factor. The termination signal may vary depending on the type of promoter sequence. Specifically, in a case where the promoter is a U6 or H1 promoter, the promoter recognizes TTTTT(T5) or TTTTTT(T6) sequence, which is a thymidine (T) repeat sequence, as a termination signal.

The sequence of the engineered guide RNA provided in the present disclosure comprises a U-rich tail sequence at its 3'-end. Accordingly, the sequence encoding the engineered guide RNA comprise a T-rich sequence corresponding to the U-rich tail sequence at its 3'-end. As described above, some promoter sequences recognize a thymidine (T) repeat sequence, for example, a sequence consisting of 5 or more consecutive thymidine (T) residues, as a termination signal. Thus, in some cases, the T-rich sequence may be recognized as a termination signal.

In other words, in a case where the vector sequence provided herein comprises the sequence encoding the engineered guide RNA, the sequence encoding the U-rich tail sequence included in the engineered gRNA sequence may be used as a termination signal.

In an embodiment, in a case where the vector sequence comprises a U6 or H1 promoter sequence and a sequence encoding the engineered guide RNA operably linked thereto, the sequence portion that encodes the U-rich tail included in the augment RNA sequence may be recognized as a termination signal. Here, the U-rich tail sequence comprises a sequence consisting of five or more consecutive uridine (U) residues.

### 5. Additional expression element

The vector may be configured to express an additional component such as NLS, NES, and/or tag protein, if necessary.

In an embodiment, the additional component may be expressed independently of the engineered guide RNA (gRNA) for the Cas12f1, TnpB, or the variant protein thereof.

In another embodiment, the additional component may be expressed in a form where it is connected directly or via a linker to the engineered guide RNA (gRNA) for the Cas12f1, TnpB, or the variant protein.

As an example, the nucleic acid construct encoding the component of the hypercompact gene editing system according to the present disclosure may be characterized by comprising at least one nuclear localization sequence (NLS) at its N-terminus or C-terminus. Here, the additional component may be a component that is generally expressed in a case of expressing the nucleic acid editing system of the present disclosure, and reference may be made to the known techniques widely recognized by those skilled in the art.

In addition, in an embodiment of the present disclosure, there is provided a nucleic acid included in a vector or the like to express the engineered guide RNA (gRNA) or a nucleic acid encoding the same and/or the component of the target nucleic acid editing system according to the present disclosure. Here, the nucleic acid may be DNA or RNA that exists in nature, or a modified nucleic acid in which a part of or all of the nucleic acid is chemically modified. For example, the nucleic acid may be one in which one or more nucleotides are chemically modified. Here, the chemical modification includes any of modifications of a nucleic acid known to those of ordinary skill in the art.

### 6. Type and form of expression vector

The vector according to the present disclosure may be a viral vector. More specifically, the viral vector may be at least one selected from the group consisting of a retroviral vector, a lentiviral vector, an adenoviral vector, an adeno-associated viral vector, a vaccinia viral vector, a poxviral vector, a herpes simplex viral vector, and a phagemid vector. In an embodiment, the viral vector may be an adeno-associated viral vector.

In addition, the vector according to the present disclosure may be a non-viral vector. More specifically, the non-viral vector may be, but is not limited to, at least one selected from the group consisting of a plasmid, naked DNA, a DNA complex, mRNA (transcript), and amplicon. In an embodiment, the plasmid may be selected from the group consisting of pcDNA series, pSC101, pGV1106, pACYC177, ColE1, pKT230, pME290, pBR322, pUC8/9, pUC6, pBD9, pHC79, pIJ61, pLAFR1, pHV14, pGEX series, pET series, and pUC19.

The term "naked DNA" refers to DNA (for example, histone-free DNA) that encodes a protein, such as Cas12f1, TnpB, or a variant thereof of the present disclosure, cloned into a suitable expression vector (for example, plasmid) in an appropriate orientation for expression. Viral vectors that can be used include, but are not limited to, SIN lentiviral vectors, retroviral vectors, foamy viral vectors, adenoviral vectors, adeno-associated virus (AAV) vectors, hybrid vectors, and/or plasmid transposons (for example, Sleeping Beauty transposons), or integrase-based vector systems.

The term "amplicon", when used in respect to a nucleic acid, means a product of copying the nucleic acid, wherein the product has a nucleotide sequence that is the same as or complementary to at least a portion of the nucleotide sequence of the nucleic acid. The amplicon may be produced by any of a variety of amplification methods that use a nucleic acid, or an amplicon thereof, as a template including, for example, polymerase extension, polymerase chain reaction (PCR), rolling circle amplification (RCA), multi-displacement amplification (MDA), ligation extension, or ligation chain reaction. The amplicon may be a nucleic acid molecule having a single copy of a particular nucleotide sequence (for example, a PCR product) or multiple copies of the nucleotide sequence (for example, a concatameric product of RCA).

The vector of the present disclosure may be designed in a form of a linear or circular vector. When the vector is a linear vector, RNA transcription is terminated at the 3' end thereof even if a sequence of the linear vector does not separately comprise a termination signal. In contrast, when the vector is a circular vector, RNA transcription is not terminated unless a sequence of the circular vector separately comprises a termination signal. Therefore, when the vector is used in a form of a circular vector, a termination signal corresponding to a transcription factor related to each promoter sequence has to be included in order for the vector to express an intended target.

In an embodiment, the viral vector or non-viral vector may be delivered by a delivery system such as liposomes, polymeric nanoparticles (for example, lipid nanoparticles), oil-in-water nanoemulsions, or combinations thereof, or in the form of a virus.

### V. Virus expressing target nucleic acid editing system

According to still yet another aspect of the present disclosure, there is provided a virus or viral particle produced by a viral vector system comprising at least one vector that comprises a first nucleic acid construct to which a nucleotide sequence encoding an endonuclease is operably linked, the endonuclease comprising Cas12f1, TnpB, or a variant protein thereof; a second nucleic acid construct to which a nucleotide sequence encoding an engineered guide RNA is operably linked, the engineered guide RNA comprising a guide sequence that binds complementarily to a target nucleic acid; and a third nucleic acid construct comprising a donor nucleic acid molecule.

In an embodiment, the viral vector may be, for example, at least one viral vector selected from the group consisting of a retroviral (retrovirus) vector, a lentiviral (lentivirus) vector, an adenoviral (adenovirus vector), an adeno-associated viral (adeno-associated virus; AAV) vector, a vaccinia viral (vaccinia virus) vector, a poxviral (poxvirus) vector, a herpes simplex viral (herpes simplex virus) vector, and a phagemid vector. Preferably, the viral vector may be an adeno-associated viral vector.

In another embodiment, the virus may be selected from the group consisting of retrovirus, lentivirus, adenovirus, adeno-associated virus, vaccinia virus, poxvirus, herpes simplex virus, and phage.

In yet another embodiment, the phage may be selected from the group consisting of λgt4λB, λ-charon, λΔz1, and M13.

For details about "Cas12f1, TnpB, or a variant protein thereof, "engineered guide RNA," and "donor nucleic acid molecule", see the description above.

In order to efficiently deliver the target nucleic acid editing system of the present disclosure into a target cell or target region through a virus, in particular adeno-associated virus (AAV), it is important to design a size of the nucleotide sequence encoding all the components within 4.7 kb that is a packaging limit of AAV. This has an advantage in that in a case of comprising the hypercompact nucleic acid editing protein and engineered gRNA of the present disclosure, their very small size allows sufficient packaging by AAV even if a longer donor nucleic acid molecule and an additional regulatory molecule are further included.

According to an embodiment of the present disclosure, vectors with a size within 4.7 kb were constructed depending on the type of promoter, the length of donor nucleic acid, and the presence or absence of a molecule for inhibiting expression of a gene that regulates non-homologous end joining (see FIG. 10A). It was identified that all four of these vectors exhibited high homology-directed repair efficiency in all three target genes, which proves that viruses, such as AAV, expressed by the viral vector system according to the present disclosure can be normally produced so that the nucleic acid editing system of the present disclosure is delivered into a cell and expressed therein (see Example 4, FIGS. 10A and 10B).

The viral vector may optionally further comprise a regulation/control component, a promoter, and/or an additional expression element. For details about the regulation/control component, see the description above.

### VI. Method for inducing homology-directed repair using target nucleic acid editing system

According to still yet another aspect of the present disclosure, there is provided a method for introducing a desired sequence into a target region on a double-stranded nucleic acid in a cell, comprising bringing, into contact with the cell, the system or composition, or the vector system according to the present disclosure, or expressing the same in the cell; and allowing the desired sequence to be introduced in a target nucleic acid or a region adjacent thereto by repair of double-strand breaks using the donor nucleic acid molecule as a template.

In an embodiment, the method for introducing the desired sequence comprises delivering, into a target cell containing a target nucleic acid or target gene, an engineered guide RNA (augment RNA) for Cas12f1, TnpB, or a variant thereof, Cas12f1, TnpB, or a variant protein, and a donor nucleic acid molecule, or a nucleic acid encoding each of these. As a result, a complex of a guide RNA, including the engineered guide RNA, with Cas12f1, TnpB, or a variant protein thereof is introduced into the target cell, or formation of a complex of the guide RNA with the Cas12f1, TnpB, or the variant protein thereof is induced therein, so that a target gene is cleaved, edited, and/or repaired by the complex of the guide RNA with the Cas12f1, TnpB, or the variant protein thereof. Gene editing involves nucleic acid cleavage of double-stranded DNA, single-stranded DNA, or DNA-RNA hybrid double strand having a target sequence in a target gene or target nucleic acid. Preferably, the gene editing involves nucleic acid cleavage of double-stranded DNA. Here, the Cas12f1 or TnpB variant protein may be a wild-type Cas12f1 variant protein, an engineered Caf12f1 variant protein, a modified Cas12f1 variant protein, or a homolog protein of the Cas12f1 variant.

Repair of DNA breakage (for example, double-strand breaks) in cells is primarily accomplished through two DNArepair pathways, that is, a non-homologous end joining (NHEJ, for example C-NHEJ) repair pathway and a homology-directed repair (HDR) pathway. During non-homologous end joining, the Ku70/80 heterodimer binds to the DNA terminus and recruits DNA protein kinase (DNA-PK) (see Cannan & Pederson (2015) J Cell Physiol 231:3-14). Upon binding of molecules associated with NHEJ, DNA-PK activates its own catalytic subunit (DNA-PKcs) and further engages the endonuclease Artemis (also known as SNM1c) in the process. In the subunit of double-strand breaks, Artemis removes excess single-stranded DNA (ssDNA) and produces a substrate to be ligated by DNA Ligase IV DNA repair by non-homologous end joining involves a blunt-end ligation mechanism independent of sequence homology through the DNA-PKcs/Ku70/80 complex. During the cell cycle, non-homologous end joining occurs predominantly in G0/G1 and G2 (see Chiruvella et al., (2013) Cold Spring Harb Perspect Biol 5:a012757). Current studies have shown that non-homologous end joining is the only DSB repair pathway active in G0 and G1, whereas homology-directed repair functions primarily in the S and G2 phases, playing a major role in the repair of replication-associated double-strand breaks (see Karanam et al., (2012) Mol Cell 47:320-329; Li and Xu (2016) Acta Biochim Biophys Sin 48(7):641-646). Non-homologous end joining, unlike homology-directed repair, is active in both dividing and non-dividing cells, as well as dividing cells. During DNA repair by homology-directed repair, the ends of the gene with double-strand breaks are mainly excised by the MRE11-RAD50-NBS1 (MRN) complex to expose the 3'-ssDNA tail (see Heyer et al., (2010) Annu Rev Genet 44: 113-139). Under physiological conditions, adjacent chromosomes will be used as repair templates providing homologous sequences.

The third repair mechanism is microhomologous-mediated end joining (MMEJ), also referred to as "alternative NHEJ (A-NHEJ)", where the genetic consequences are similar to NHEJ in that minor deletions and insertions can occur at a cleavage site. MMEJ uses homologous sequences of a small number of nucleotides flanking the DNA break site leading to more desirable DNA terminal linkage repair results, and recent reports further explain the molecular mechanism of this process (see Cho and Greenberg, (2015) Nature 518:174-176; Mateos-Gomez et al., (2015) Nature 518, 254-257; Ceccaldi et al., (2015) Nature 528, 258-262).

In mammalian cells, the "canonical" or "classical" NHEJ pathway (C-NHEJ) requires several factors, including DNA-PK, Ku70-80, Artemis, ligase IV (Lig4), XRCC4, CLF, and Pol Mu to repair double-strand breaks in a nucleic acid or gene (see Kasparek & Humphrey (2011) Seminars in Cell & Dev. Biol. 22:886-897).

Accordingly, in some of the systems, the compositions, or the methods disclosed herein, cells may be modified to decrease or eliminate expression or activity of a factor involved in C-NHEJ. For example, some systems or methods may further comprise a factor that can decrease or eliminate expression or activity of MRE11, RAD50, NBS1, DNA-PK, CtIP, Ku70, Ku80, Artemis (DCLRE1C), Ligase IV (Lig4), PNKP, XRCC4, XRCC4-like factor (XLF), ATM Serine/Threonine Kinase (ATM), CHK1/CHK2, CURLY LEAF (CLF), and/or Pol Mu (POLM).

In addition, in some of the systems, the compositions, or the methods disclosed herein, cells may be modified to decrease or eliminate expression or activity of a factor involved in A-NHEJ. For example, some systems, compositions, or methods may further comprise a factor that can decrease or eliminate expression or activity of XRCC1, PARP (for example, PARP1), Lig1, and/or Lig3.

DNA-repair via homology-directed repair involves multiple classes of proteins that include nucleases or helicases to process free DNA ends, and protein binding domains to act as nucleation sites for supplementary homology-directed repair factors. In an embodiment, the protein for homology-directed repair may be selected from the group consisting of nucleases and/or helicases that promote DNA strand cleavage, for example, MRE11, EXO1, DNA2, CtIP, TREX2, and Apollo; binding factors/nucleation proteins that recruit specific factors or catalyze strand invasion, for example, BRCA1, BRCA2, PALB2, RAD50 or NBS1, RAD51, RAD52, RAD54, SRCAP, FANCI, FANCD2, BRIP1, SLX4, FANCA, FANCE, and FANCL (including truncated, mutated, modified, or optimized versions of these factors).

In an embodiment, to enhance homology-directed repair, the full-length or truncated version of any of the proteins for homology-directed repair described herein may bind to an endonuclease (for example, Cas12f1, TnpB, or a variant thereof). Accordingly, in the present disclosure, there is further provided a fusion protein comprising Cas12f1, TnpB, or a variant protein thereof. The Cas12f1, TnpB, or the variant thereof and the protein for homology-directed repair may be linked to each other through any sequence of 1 to 100, 1 to 50, 1 to 30, or 1 to 20 amino acids.

In some embodiments, the method provides a method for introducing a desired sequence into a target region on a double-stranded nucleic acid in a cell, comprising bringing, into contact with the cell, the system, the composition, or the vector system according to the present disclosure, or expressing the same in the cell; and inducing double-strand breaks at a target region under conditions sufficient to allow a donor nucleic acid molecule to be inserted into a double-strand break site and the double-strand breaks to be repaired, thereby causing the donor nucleic acid molecule to be introduced in a target nucleic acid or a region adjacent thereto. Here, the repair of double-strand breaks may be by homology-directed repair mechanism.

The bringing, into contact with the cell, may comprise delivery or introduction of the target nucleic acid editing system into the cell. Treating the cell with the target nucleic acid editing system may be performed using electroporation, gene gun, sonoporation, magnetofection, nanoparticles, and/or transient cell compression or squeezing. Alternatively, treating the eukaryotic cell with the composition may be performed using cationic liposome, lithium acetate-DMSO, lipid-mediated transfection, calcium phosphate precipitation, lipofection, polyethyleneimine (PEI)-mediated transfection, DEAE-dextran-mediated transfection, and/or nanoparticle-mediated nucleic acid delivery (see Panyam et al., Adv Drug Deliv Rev. 2012 Sep. 13.doi: 10.1016/j.addr.2012.09.023). Treating the cell with the system, or delivering or introducing the system into the cell may be performed *in vitro*, *in vivo,* or *ex vivo.*

The vector system may be such that it is introduced into a packaging virus selected from the group consisting of retrovirus, lentivirus, adenovirus, adeno-associated virus, vaccinia virus, poxvirus, herpes simplex virus, and phage, and is delivered into a prokaryotic or eukaryotic cell in a form of a virus produced by the packaging virus.

The cell may be a plant cell, non-human animal cell, or human cell. In addition, the cell may be a eukaryotic or prokaryotic cell.

For details about "Cas12f1, TnpB, or a variant protein thereof, "engineered guide RNA," and "donor nucleic acid molecule", see the description above.

Representative embodiments or combinations of embodiments are as follows.

### [Embodiment 1]

A target nucleic acid editing system, comprising:
an endonuclease comprising Cas12f1, TnpB, or a variant protein thereof, or a nucleic acid encoding the endonuclease;
an engineered guide RNA comprising a guide sequence or a nucleic acid encoding the guide RNA; and
a donor nucleic acid molecule or a nucleic acid encoding the donor nucleic acid molecule.

### [Embodiment 2]

The system of the above-described embodiment, wherein
the system causes double-strand breaks in the target nucleic acid.

### [Embodiment 3]

The system of any one of the above-described embodiments, wherein
the system is such that a desired sequence is introduced in the target nucleic acid or a region adjacent thereto by homology-directed repair of double-strand breaks using the donor nucleic acid molecule as a template.

### [Embodiment 4]

A gene editing composition for a target nucleic acid, comprising:
an endonuclease comprising Cas12f1, TnpB, or a variant protein thereof, or a nucleic acid encoding the endonuclease;
an engineered guide RNA comprising a guide sequence or a nucleic acid encoding the guide RNA; and
a donor nucleic acid molecule or a nucleic acid encoding the donor nucleic acid molecule.

### [Embodiment 5]

The system or composition of any one of the above-described embodiments, wherein
the Cas12f1, TnpB, or the variant protein thereof comprises an amino acid sequence having at least 70% sequence identity with an amino acid sequence selected from the group consisting of SEQ ID NOS: 1 to 5.

### [Embodiment 6]

The system or composition of any one of the above-described embodiments, wherein
the TnpB protein comprises an amino acid sequence having at least 70% sequence identity with an amino acid sequence selected from the group consisting of SEQ ID NOS: 202 to 293.

### [Embodiment 7]

The system or composition of any one of the above-described embodiments, wherein
the Cas12f1, TnpB, or the variant protein thereof comprises one selected from the following sequences:
(i) the amino acid sequence of SEQ ID NO: 5;
(ii) the amino acid sequence of SEQ ID NO: 1;
(iii) an amino acid sequence having the amino acid sequence of SEQ ID NO: 1 in which 1 to 28 amino acids at the N-terminus have been removed or substituted; or
(iv) an amino acid sequence having the amino acid sequence of SEQ ID NO: 1 in which 1 to 600 amino acids have been added to the N-terminus or C-terminus.

### [Embodiment 8]

The system or composition of any one of the above-described embodiments, wherein
the added 1 to 600 amino acids are the amino acid sequence of SEQ ID NO: 294 or 295.

### [Embodiment 9]

The system or composition of any one of the above-described embodiments, wherein
the Cas12f1, TnpB, or the variant protein thereof has at least 70% sequence identity with an amino acid sequence selected from the group consisting of SEQ ID NOS: 1 to 4.

### [Embodiment 10]

The system or composition of any one of the above-described embodiments, wherein
the Cas12f1, TnpB, or the variant protein thereof has at least 70% sequence identity with the amino acid sequence of SEQ ID NO: 1.

### [Embodiment 11]

The system or composition of any one of the above-described embodiments, wherein
the engineered guide RNA comprises substitution, deletion, insertion, or addition of one or more nucleotides in reference with a wild-type Cas12f1 guide RNA sequence, and a portion of the engineered guide RNA, excluding the guide sequence, has at least 50% sequence identity with the wild-type Cas12f1 guide RNA.

### [Embodiment 12]

The system or composition of any one of the above-described embodiments, wherein
the wild-type Cas12f1 guide RNA comprises trans-activating CRISPR RNA (tracrRNA) and CRISPR RNA (crRNA) comprising (i) one or more stem regions, (ii) a tracrRNA-crRNA complementarity region, and optionally (iii) a region containing three or more consecutive uracil (U) residues, and
the engineered guide RNA comprises at least one modification selected from the group consisting of (a) to (d):
   (a) deletion of at least a part of one or more stem regions;
   (b) deletion of at least a part of the tracrRNA-crRNA complementarity region;
   (c) replacement of one or more uracil (U) residues with A, G, or C in three or more consecutive U residues when the consecutive U residues are present in the tracrRNA-crRNA complementarity region; and
   (d) addition of one or more uridine residues to the 3'-end of the crRNA sequence.

### [Embodiment 13]

The system or composition of any one of the above-described embodiments, wherein
the wild-type Cas12f1 guide RNA comprises tracrRNA consisting of the nucleotide sequence of SEQ ID NO: 11 and crRNA consisting of the nucleotide sequence of SEQ ID NO: 12.

### [Embodiment 14]

The system or composition of any one of the above-described embodiments, wherein
the wild-type Cas12f1 guide RNA comprises tracrRNA and crRNA which sequentially comprise, from the 5'-end, a first stem region, a second stem region, a third stem region, a fourth stem region, and the tracrRNA-crRNA complementarity region, and
the engineered guide RNA comprises at least one modification selected from the group consisting of:
   (a1) deletion of at least a part of the first stem region
   (a2) deletion of at least a part of the second stem region;
   (b) deletion of at least a part of the tracrRNA-crRNA complementarity region;
   (c) replacement of one or more uracil (U) residues with A, G, or C in three or more consecutive U residues when the consecutive U residues are present in the tracrRNA-crRNA complementarity region; and
   (d1) addition of a U-rich tail to the 3'-end of the crRNA sequence (in which a sequence of the U-rich tail is represented by 5'-(UₘV)ₙUₒ-3', where V is each independently A, C, or G, m and o are integers between 1 and 20, and n is an integer between 0 and 5).

### [Embodiment 15]

The system or composition of any one of the above-described embodiments, wherein
the engineered guide RNA comprises (d1) addition of a U-rich tail to the 3'-end of the crRNA sequence or (c) replacement of one or more uracil (U) residues with A, G, or C in three or more consecutive U residues when the consecutive U residues are present in the tracrRNA-crRNA complementarity region, or both modifications.

### [Embodiment 16]

The system or composition of any one of the above-described embodiments, wherein
the engineered guide RNA comprises at least one modification selected from the group consisting of (a1) deletion of at least a part of the first stem region; (a2) deletion of at least a part of the second stem region; and (b) deletion of at least a part of the tracrRNA-crRNA complementarity region.

### [Embodiment 17]

The system or composition of any one of the above-described embodiments, wherein
the engineered guide RNA comprises (b 1) deletion of a part of the tracrRNA-crRNA complementarity region, and the part of the complementary region consists of 1 to 54 nucleotides.

### [Embodiment 18]

The system or composition of any one of the above-described embodiments, wherein
the engineered guide RNA comprises (b2) deletion of the entire tracrRNA-crRNA complementarity region, and the entire complementary region consists of 55 nucleotides.

### [Embodiment 19]

The system or composition of any one of the above-described embodiments, wherein
the engineered guide RNA comprises (a1) deletion of at least a part of the first stem region, and the at least a part of the stem region consists of 1 to 20 nucleotides.

### [Embodiment 20]

The system or composition of any one of the above-described embodiments, wherein
the engineered guide RNA comprises (a2) deletion of at least a part of the second stem region, and the at least a part of the stem region consists of 1 to 27 nucleotides.

### [Embodiment 21]

The system or composition of any one of the above-described embodiments, wherein
the engineered guide RNA comprises (a1) deletion of at least a part of the first stem region; (d1) addition of a U-rich tail to the 3'-end of the crRNA sequence; or both modifications.

### [Embodiment 22]

The system or composition of any one of the above-described embodiments, wherein
the engineered guide RNA consists of a sequence represented by Formula (I) or has at least 80% sequence identity therewith: 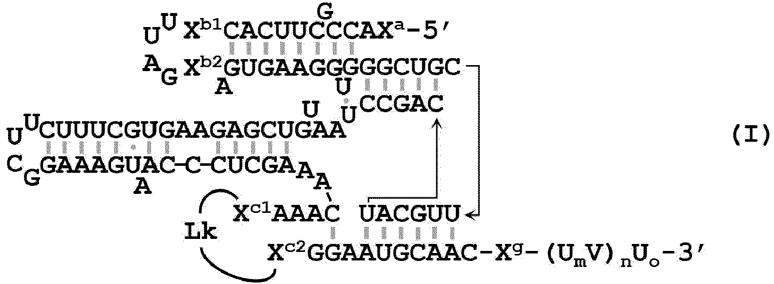 in Formula (I),
X^{a}, X^{b1}, X^{b2}, X^{c1}, and X^{c2} each independently consists of 0 to 35 (poly)nucleotides,
X^{g} is a guide sequence that consists of 10 to 30 nucleotides and is hybridizable with or complementary to a target sequence,
Lk is a polynucleotide linker of 2 to 20 nucleotides or is absent, and
(UₘV)ₙUₒ is present as a U-rich tail or absent, and when (UₘV)ₙUₒ is present, U is uridine, V is each independently A, C, or G, m and o are each independently an integer between 1 and 20, and n is an integer between 0 and 5.

### [Embodiment 23]

The system or composition of any one of the above-described embodiments, wherein
X^{a} comprises the nucleotide sequence of SEQ ID NO: 14 or a nucleotide sequence having the sequence of SEQ ID NO: 14 from which 1 to 20 nucleotides are deleted.

### [Embodiment 24]

The system or composition of any one of the above-described embodiments, wherein
X^{b1} comprises the nucleotide sequence of SEQ ID NO: 25 or a nucleotide sequence having the sequence of SEQ ID NO: 25 from which 1 to 13 nucleotides are deleted.

### [Embodiment 25]

The system or composition of any one of the above-described embodiments, wherein
X^{b2} comprises the nucleotide sequence of SEQ ID NO: 29 or a nucleotide sequence having the sequence of SEQ ID NO: 29 from which 1 to 14 nucleotides are deleted.

### [Embodiment 26]

The system or composition of any one of the above-described embodiments, wherein
the sequence 5'-X^{b1}UUAGX^{b2}-3' in Formula (I) is any one nucleotide sequence selected from the group consisting of SEQ ID NOS: 34 to 38.

### [Embodiment 27]

The system or composition of any one of the above-described embodiments, wherein
X^{c1} comprises the nucleotide sequence of SEQ ID NO: 39 or a nucleotide sequence having the sequence of SEQ ID NO: 39 from which 1 to 28 nucleotides are deleted.

### [Embodiment 28]

The system or composition of any one of the above-described embodiments, wherein
in a case where three or more consecutive uracil (U) residues are present in a sequence of X^{c1}, the sequence of X^{c1} comprises a modification in which at least one uracil residue thereof is replaced with A, G, or C.

### [Embodiment 29]

The system or composition of any one of the above-described embodiments, wherein
X^{c2} comprises the nucleotide sequence of SEQ ID NO: 58 or a nucleotide sequence having the sequence of SEQ ID NO: 58 from which 1 to 27 nucleotides are deleted.

### [Embodiment 30]

The system or composition of any one of the above-described embodiments, wherein
in a case where the sequence 5'-ACGAA-3' is present in X^{c2}, the sequence is replaced with 5'-NGNNN-3', and N is each independently A, C, G, or U.

### [Embodiment 31]

The system or composition of any one of the above-described embodiments, wherein
the sequence 5'-X^{c1}-Lk-X^{c2}-3' in Formula (I) is any one nucleotide sequence selected from the group consisting of SEQ ID NOS: 80 to 86.

### [Embodiment 32]

The system or composition of any one of the above-described embodiments, wherein
Lk is any one nucleotide sequence selected from the group consisting of 5'-GAAA-3', 5'-UUAG-3', 5'-UGAAAA-3', 5'-UUGAAAAA-3', 5'-UUCGAAAGAA-3' (SEQ ID NO: 76), 5'-UUCAGAAAUGAA-3' (SEQ ID NO: 77), 5'-UUCAUGAAAAUGAA-3' (SEQ ID NO: 78), and 5'-UUCAUUGAAAAAUGAA-3' (SEQ ID NO: 79).

### [Embodiment 33]

The system or composition of any one of the above-described embodiments, wherein
(UₘV)ₙUₒ is such that (i) n is 0 and o is an integer between 1 and 6, or (ii) V is A or G, m and o are each independently an integer between 3 and 6, and n is an integer between 1 and 3.

### [Embodiment 34]

The system or composition of any one of the above-described embodiments, wherein
the engineered guide RNA comprises an engineered tracrRNA consisting of any one nucleotide sequence selected from the group consisting of SEQ ID NOS: 87 to 132.

### [Embodiment 35]

The system or composition of any one of the above-described embodiments, wherein
the engineered guide RNA comprises an engineered crRNA consisting of any one nucleotide sequence selected from the group consisting of SEQ ID NOS: 133 to 148.

### [Embodiment 36]

The system or composition of any one of the above-described embodiments, wherein
the engineered guide RNA is a dual guide RNA or a single guide RNA.

### [Embodiment 37]

The system or composition of any one of the above-described embodiments, wherein
the engineered single guide RNA consists of any one nucleotide sequence selected from the group consisting of SEQ ID NOS: 149 to 186.

### [Embodiment 38]

The system or composition of any one of the above-described embodiments, wherein
the endonuclease comprising Cas12f1, TnpB, or a variant protein thereof; and the guide RNA are included in a form of a ribonucleoprotein (RNP).

### [Embodiment 39]

The system or composition of any one of the above-described embodiments, wherein
the donor nucleic acid molecule is a sequence used as a template in homology-directed repair and has a length of 1 bp to 20 kb.

### [Embodiment 40]

The system or composition of any one of the above-described embodiments, wherein
the system or composition further comprises a molecule that inhibits expression of a gene involved in non-homologous end joining (NHEJ).

### [Embodiment 41]

The system or composition of any one of the above-described embodiments, wherein
the gene involved in non-homologous end joining is at least one selected from the group consisting of ATM1, XRCC4, XLF, XRCC6, LIG4, and DCLRE1C.

### [Embodiment 42]

The system or composition of any one of the above-described embodiments, wherein
the molecule is shRNA, siRNA, miRNA, or antisense oligonucleotide.

### [Embodiment 43]

A vector system, comprising:
a first nucleic acid construct to which a nucleotide sequence encoding an endonuclease is operably linked, the endonuclease comprising Cas12f1, TnpB, or a variant protein thereof;
a second nucleic acid construct to which a nucleotide sequence encoding an engineered guide RNA is operably linked, the engineered guide RNA comprising a guide sequence that binds complementarily to a target nucleic acid; and
a third nucleic acid construct comprising a donor nucleic acid molecule.

### [Embodiment 44]

The vector system of any one of the above-described embodiments, wherein
the nucleic acid constructs included in the vector system are located in the same or different vectors.

### [Embodiment 45]

The vector system of any one of the above-described embodiments,
further comprising at least one nucleic acid construct to which a nucleotide sequence encoding a molecule is operably linked, the molecule inhibiting expression of a gene involved in non-homologous end joining (NHEJ).

### [Embodiment 46]

The vector system of any one of the above-described embodiments, wherein
the gene involved in non-homologous end joining is at least one selected from the group consisting of ATM1, XRCC4, XLF, XRCC6, LIG4, and DCLRE1C.

### [Embodiment 47]

The vector system of any one of the above-described embodiments, wherein
the molecule is shRNA, siRNA, miRNA, or antisense oligonucleotide.

### [Embodiment 48]

The vector system of any one of the above-described embodiments, wherein
respective components in the vector are included in one vector.

### [Embodiment 49]

The vector system of any one of the above-described embodiments, wherein
the donor nucleic acid molecule has a length of 1 bp to 20 kb.

### [Embodiment 50]

The vector system of any one of the above-described embodiments, wherein
the vector further comprises a promoter or an enhancer.

### [Embodiment 51]

The vector system of any one of the above-described embodiments, wherein
the promoter is U6 promoter, EFS promoter, EF1-α promoter, H1 promoter, 7SK promoter, CMV promoter, LTR promoter, Ad MLP promoter, HSV promoter, SV40 promoter, CBA promoter, or RSV promoter.

### [Embodiment 52]

The vector system of any one of the above-described embodiments, wherein
the vector is at least one viral vector selected from the group consisting of a retroviral (retrovirus) vector, a lentiviral (lentivirus) vector, an adenoviral (adenovirus vector), an adeno-associated viral vector. (adeno-associated virus; AAV) vector, a vaccinia viral (vaccinia virus) vector, a poxviral (poxvirus) vector, a herpes simplex viral (herpes simplex virus) vector, and a phagemid vector.

### [Embodiment 53]

The vector system of any one of the above-described embodiments, wherein
the vector is an adeno-associated viral vector, and the adeno-associated viral vector is such that all components within the vector can be included in one vector.

### [Embodiment 54]

The vector system of any one of the above-described embodiments, wherein
the vector is at least one non-viral vector selected from the group consisting of plasmid, naked DNA, DNA complex, mRNA (transcript), and amplicon.

### [Embodiment 55]

The vector system of any one of the above-described embodiments, wherein
the plasmid is at least one selected from the group consisting of pcDNA series, pSC101, pGV1106, pACYC177, ColE1, pKT230, pME290, pBR322, pUC8/9, pUC6, pBD9, pHC79, pIJ61, pLAFR1, pHV14, pGEX series, pET series, and pUC19.

### [Embodiment 56]

The vector system of any one of the above-described embodiments, wherein
the Cas12f1, TnpB, or the variant protein thereof comprises an amino acid sequence having at least 70% sequence identity with an amino acid sequence selected from the group consisting of SEQ ID NOS: 1 to 5.

### [Embodiment 57]

The vector system of any one of the above-described embodiments, wherein
the TnpB protein comprises an amino acid sequence having at least 70% sequence identity with an amino acid sequence selected from the group consisting of SEQ ID NOS: 202 to 293.

### [Embodiment 58]

The vector system of any one of the above-described embodiments, wherein
the Cas12f1, TnpB, or the variant protein thereof comprises one selected from the following sequences:
(i) the amino acid sequence of SEQ ID NO: 5;
(ii) the amino acid sequence of SEQ ID NO: 1;
(iii) an amino acid sequence having the amino acid sequence of SEQ ID NO: 1 in which 1 to 28 amino acids at the N-terminus have been removed or substituted; or
(iv) an amino acid sequence having the amino acid sequence of SEQ ID NO: 1 in which 1 to 600 amino acids have been added to the N-terminus or C-terminus.

### [Embodiment 59]

The vector system of any one of the above-described embodiments, wherein
the added 1 to 600 amino acids are the amino acid sequence of SEQ ID NO: 294 or 295.

### [Embodiment 60]

The vector system of any one of the above-described embodiments, wherein
the Cas12f1, TnpB, or the variant protein thereof has at least 70% sequence identity with an amino acid sequence selected from the group consisting of SEQ ID NOS: 1 to 4.

### [Embodiment 61]

The vector system of any one of the above-described embodiments, wherein
the Cas12f1, TnpB, or the variant protein thereof has at least 70% sequence identity with the amino acid sequence of SEQ ID NO: 1.

### [Embodiment 62]

The vector system of any one of the above-described embodiments, wherein
the engineered guide RNA comprises substitution, deletion, insertion, or addition of one or more nucleotides in reference with a wild-type Cas12f1 guide RNA sequence, and a portion of the engineered guide RNA, excluding the guide sequence, has at least 50% sequence identity with the wild-type Cas12f1 guide RNA.

### [Embodiment 63]

The vector system of any one of the above-described embodiments, wherein
the wild-type Cas12f1 guide RNA comprises trans-activating CRISPR RNA (tracrRNA) and CRISPR RNA (crRNA) comprising (i) one or more stem regions, (ii) a tracrRNA-crRNA complementarity region, and optionally (iii) a region containing three or more consecutive uracil (U) residues, and
the engineered guide RNA comprises at least one modification selected from the group consisting of (a) to (d):
   (a) deletion of at least a part of one or more stem regions;
   (b) deletion of at least a part of the tracrRNA-crRNA complementarity region;
   (c) replacement of one or more uracil (U) residues with A, G, or C in three or more consecutive U residues when the consecutive U residues are present in the tracrRNA-crRNA complementarity region; and
   (d) addition of one or more uridine residues to the 3'-end of the crRNA sequence.

### [Embodiment 64]

The vector system of any one of the above-described embodiments, wherein
the wild-type Cas12f1 guide RNA comprises tracrRNA consisting of the nucleotide sequence of SEQ ID NO: 11 and crRNA consisting of the nucleotide sequence of SEQ ID NO: 12.

### [Embodiment 65]

The vector system of any one of the above-described embodiments, wherein
the wild-type Cas12f1 guide RNA comprises tracrRNA and crRNA which sequentially comprise, from the 5'-end, a first stem region, a second stem region, a third stem region, a fourth stem region, and the tracrRNA-crRNA complementarity region, and
the engineered guide RNA comprises at least one modification selected from the group consisting of:
   (a1) deletion of at least a part of the first stem region;
   (a2) deletion of at least a part of the second stem region;
   (b) deletion of at least a part of the tracrRNA-crRNA complementarity region;
   (c) replacement of one or more uracil (U) residues with A, G, or C in three or more consecutive U residues when the consecutive U residues are present in the tracrRNA-crRNA complementarity region; and
   (d1) addition of a U-rich tail to the 3'-end of the crRNA sequence (in which a sequence of the U-rich tail is represented by 5'-(UₘV)ₙUₒ-3', where V is each independently A, C, or G, m and o are integers between 1 and 20, and n is an integer between 0 and 5).

### [Embodiment 66]

The vector system of any one of the above-described embodiments, wherein
the engineered guide RNA comprises (d1) addition of a U-rich tail to the 3'-end of the crRNA sequence or (c) replacement of one or more uracil (U) residues with A, G, or C in three or more consecutive U residues when the consecutive U residues are present in the tracrRNA-crRNA complementarity region, or both modifications.

### [Embodiment 67]

The vector system of any one of the above-described embodiments, wherein
the engineered guide RNA comprises at least one modification selected from the group consisting of (a1) deletion of at least a part of the first stem region; (a2) deletion of at least a part of the second stem region; and (b) deletion of at least a part of the tracrRNA-crRNA complementarity region.

### [Embodiment 68]

The vector system of any one of the above-described embodiments, wherein
the engineered guide RNA comprises (b1) deletion of a part of the tracrRNA-crRNA complementarity region, and the part of the complementary region consists of 1 to 54 nucleotides.

### [Embodiment 69]

The system or composition of any one of the above-described embodiments, wherein
the engineered guide RNA comprises (b2) deletion of the entire tracrRNA-crRNA complementarity region, and the entire complementary region consists of 55 nucleotides.

### [Embodiment 70]

The vector system of any one of the above-described embodiments, wherein
the engineered guide RNA comprises (a1) deletion of at least a part of the first stem region, and the at least a part of the stem region consists of 1 to 20 nucleotides.

### [Embodiment 71]

The vector system of any one of the above-described embodiments, wherein
the engineered guide RNA comprises (a2) deletion of at least a part of the second stem region, and the at least a part of the stem region consists of 1 to 27 nucleotides.

### [Embodiment 72]

The vector system of any one of the above-described embodiments, wherein
the engineered guide RNA comprises (a1) deletion of at least a part of the first stem region; (d1) addition of a U-rich tail to the 3'-end of the crRNA sequence; or both modifications.

### [Embodiment 73]

The system or composition of any one of the above-described embodiments, wherein
the engineered guide RNA consists of a sequence represented by Formula (I) or has at least 80% sequence identity therewith: 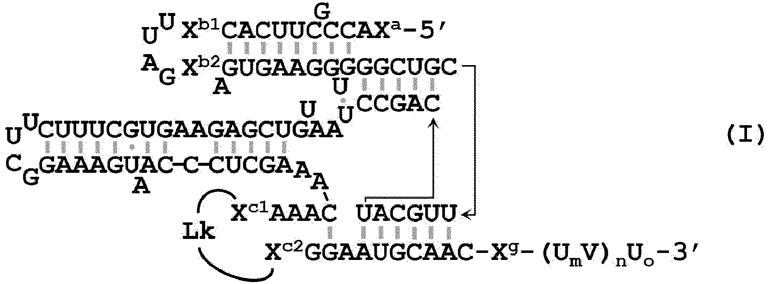 in Formula (I),
X^{a}, X^{b1}, X^{b2}, X^{c1}, and X^{c2} each independently consists of 0 to 35 (poly)nucleotides,
X^{g} is a guide sequence that consists of 10 to 30 nucleotides and is hybridizable with or complementary to a target sequence,
Lk is a polynucleotide linker of 2 to 20 nucleotides or is absent, and
(UₘV)ₙUₒ is present as a U-rich tail or absent, and when (UₘV)ₙUₒ is present, U is uridine, V is each independently A, C, or G, m and o are each independently an integer between 1 and 20, and n is an integer between 0 and 5.

### [Embodiment 74]

The vector system of any one of the above-described embodiments, wherein
X^{a} comprises the nucleotide sequence of SEQ ID NO: 14 or a nucleotide sequence having the sequence of SEQ ID NO: 14 from which 1 to 20 nucleotides are deleted.

### [Embodiment 75]

The vector system of any one of the above-described embodiments, wherein
X^{b1} comprises the nucleotide sequence of SEQ ID NO: 25 or a nucleotide sequence having the sequence of SEQ ID NO: 25 from which 1 to 13 nucleotides are deleted.

### [Embodiment 76]

The vector system of any one of the above-described embodiments, wherein
X^{b2} comprises the nucleotide sequence of SEQ ID NO: 29 or a nucleotide sequence having the sequence of SEQ ID NO: 29 from which 1 to 14 nucleotides are deleted.

### [Embodiment 77]

The vector system of any one of the above-described embodiments, wherein
the sequence 5'-X^{b1}UUAGX^{b2}-3' in Formula (I) is any one nucleotide sequence selected from the group consisting of SEQ ID NOS: 34 to 38.

### [Embodiment 78]

The vector system of any one of the above-described embodiments, wherein
X^{c1} comprises the nucleotide sequence of SEQ ID NO: 39 or a nucleotide sequence having the sequence of SEQ ID NO: 39 from which 1 to 28 nucleotides are deleted.

### [Embodiment 79]

The vector system of any one of the above-described embodiments, wherein
in a case where three or more consecutive uracil (U) residues are present in a sequence of X^{c1}, the sequence of X^{c1} comprises a modification in which at least one uracil residue thereof is replaced with A, G, or C.

### [Embodiment 80]

The vector system of any one of the above-described embodiments, wherein
X^{c2} comprises the nucleotide sequence of SEQ ID NO: 58 or a nucleotide sequence having the sequence of SEQ ID NO: 58 from which 1 to 27 nucleotides are deleted.

### [Embodiment 81]

The vector system of any one of the above-described embodiments, wherein
in a case where the sequence 5'-ACGAA-3' is present in X^{c2}, the sequence is replaced with 5'-NGNNN-3', and N is each independently A, C, G, or U.

### [Embodiment 82]

The vector system of any one of the above-described embodiments, wherein
the sequence 5'-X^{c1}-Lk-X^{c2}-3' in Formula (I) is any one nucleotide sequence selected from the group consisting of SEQ ID NOS: 80 to 86.

### [Embodiment 83]

The vector system of any one of the above-described embodiments, wherein
Lk is any one nucleotide sequence selected from the group consisting of 5'-GAAA-3', 5'-UUAG-3', 5'-UGAAAA-3', 5'-UUGAAAAA-3', 5'-UUCGAAAGAA-3' (SEQ ID NO: 76), 5'-UUCAGAAAUGAA-3' (SEQ ID NO: 77), 5'-UUCAUGAAAAUGAA-3' (SEQ ID NO: 78), and 5'-UUCAUUGAAAAAUGAA-3' (SEQ ID NO: 79).

### [Embodiment 84]

The vector system of any one of the above-described embodiments, wherein
(UₘV)ₙUₒ is such that (i) n is 0 and o is an integer between 1 and 6, or (ii) V is A or G, m and o are each independently an integer between 3 and 6, and n is an integer between 1 and 3.

### [Embodiment 85]

The vector system of any one of the above-described embodiments, wherein
the engineered guide RNA comprises an engineered tracrRNA consisting of any one nucleotide sequence selected from the group consisting of SEQ ID NOS: 87 to 132.

### [Embodiment 86]

The vector system of any one of the above-described embodiments, wherein
the engineered guide RNA comprises an engineered crRNA consisting of any one nucleotide sequence selected from the group consisting of SEQ ID NOS: 133 to 148.

### [Embodiment 87]

The vector system of any one of the above-described embodiments, wherein
the engineered guide RNA is a dual guide RNA or a single guide RNA.

### [Embodiment 88]

The vector system of any one of the above-described embodiments, wherein
the engineered single guide RNA consists of any one nucleotide sequence selected from the group consisting of SEQ ID NOS: 149 to 186.

### [Embodiment 89]

A virus produced by the vector system of any one of the above-described embodiments.

### [Embodiment 90]

The virus of any one of the above-described embodiments, wherein
the virus is selected from the group consisting of retrovirus, lentivirus, adenovirus, adeno-associated virus, vaccinia virus, poxvirus, herpes simplex virus, and phage.

### [Embodiment 91]

A composition, comprising the virus of any one of the above-described embodiments.

### [Embodiment 92]

A method for introducing a desired sequence into a target region on a double-stranded nucleic acid in a cell, comprising:
bringing, into contact with the cell, the system or composition of any one of the above-described embodiments, or the vector system of any one of the above-described embodiments, or expressing the same in the cell; and
allowing the desired sequence to be introduced in a target nucleic acid or a region adjacent thereto by repair of double-strand breaks using the donor nucleic acid molecule as a template.

### [Embodiment 93]

The method of any one of the above-described embodiments, wherein
the repair of double-strand breaks is by homology-directed repair mechanism.

### [Embodiment 94]

The method of any one of the above-described embodiments, wherein
the cell is a prokaryotic cell or eukaryotic cell in which a target nucleic acid or target gene is present.

### [Embodiment 95]

The method of any one of the above-described embodiments, wherein
the eukaryotic cell is a yeast, an insect cell, a plant cell, a non-human-animal cell, or a human cell.

### [Embodiment 96]

The method of any one of the above-described embodiments, wherein
the vector system is introduced into a packaging virus selected from the group consisting of retrovirus, lentivirus, adenovirus, adeno-associated virus, vaccinia virus, poxvirus, herpes simplex virus, and phage, and is delivered into a prokaryotic cell or eukaryotic cell in a form of a virus produced by the packaging virus.

### [Embodiment 97]

The method of any one of the above-described embodiments, wherein
the vector system is delivered into a prokaryotic cell or eukaryotic cell by electroporation, gene gun, sonoporation, magnetofection, transient cell compression or squeezing, cationic liposome method, lithium acetate-DMSO, lipid-mediated transfection, calcium phosphate precipitation, lipofection, polyethyleneimine (PEI)-mediated transfection, DEAE-dextran mediated transfection, or nanoparticle-mediated nucleic acid delivery.

### [Embodiment 98]

The method of any one of the above-described embodiments, wherein
the vector system is delivered directly into a prokaryotic cell or eukaryotic cell through at least one lipid nanoparticle (LNP).

### [Embodiment 99]

The method of any one of the above-described embodiments, wherein
the bringing-into-contact or the expressing occurs in vivo or in vitro.

Hereinafter, the present disclosure will be described in more detail through examples. These examples are only for illustrating the present disclosure, and it would be obvious to those of ordinary skill in the art that a scope of the disclosure is not to be construed as being limited by these examples.

### Examples

### Example 1. Production of components of nucleic acid editing system for homology-directed repair

### Example 1.1. Human codon-optimized nucleic acid encoding Cas12f1, TnpB, or variant protein thereof

The nucleic acid editing system for homology-directed repair of the present disclosure comprises Cas12f1, TnpB, or a variant protein thereof (that is, Cas12f1 variant protein or TnpB variant protein) as one component. The Cas12f1, TnpB, or the variant protein thereof comprises an amino acid sequence having at least 70% sequence identity with an amino acid sequence selected from the group consisting of SEQ ID NOS: 1 to 5. Preferably, the Cas12f1 protein comprises a protein comprising or consisting of the amino acid sequence of SEQ ID NO: 5, and the TnpB protein comprises a protein comprising or consisting of the amino acid sequence of SEQ ID NO: 1 (here, TnpB may be also classified and referred to as CWCas12f1).

In addition, the Cas12f1 variant or TnpB variant protein comprises a protein comprising or consisting of an amino acid sequence having the amino acid sequence of SEQ ID NO: 1 in which 1 to 28 amino acids at the N-terminus have been removed or substituted. As a representative example of the Cas12f1 variant or TnpB variant protein consisting of an amino acid sequence having the amino acid sequence of SEQ ID NO: 1 in which 1 to 28 amino acids at the N-terminus have been removed or substituted, there is provided herein TnpB-v1 protein (SEQ ID NO: 2) that comprises N-terminal 26 aa of CasX at the N-terminus of the Cas12f1 protein, TnpB-v2 protein (SEQ ID NO: 3) that comprises a 28 aa random sequence at the N-terminus of the Cas12f1 protein, or TnpB-v3 protein (SEQ ID NO: 4) that comprises a 26 aa random sequence at the N-terminus of the Cas12f1 protein. In addition, the Cas12f1 variant or TnpB variant protein comprises a protein consisting of an amino acid sequence obtained by adding 1 to 600 amino acids to the N-terminus or C-terminus of TnpB that comprises or consists of the amino acid sequence of SEQ ID NO: 1. Here, the 1 to 600 amino acids added to the N-terminus or C-terminus may comprise or consist of the amino acid sequence of SEQ ID NO: 294 or 295, and one or more NLS sequences may be further included between the added sequence and the variant protein. In addition, the TnpB variant protein corresponding to TnpB derived from another species may be a protein comprising or consisting of any one amino acid sequence selected from SEQ ID NOS: 202 to 293.

In order to construct a target nucleic acid editing system expressed in human cells and a nucleic acid construct encoding a target nucleic acid editing system for nucleic acid cleavage, a codon optimization program was used to obtain human codon-optimized genes for Cas12f1, TnpB, or variant proteins thereof. The thus obtained human codon-optimized nucleotide sequences encoding Cas12f1, TnpB, or variant proteins thereof are represented by SEQ ID NO: 6 (TnpB), SEQ ID NO: 7 (TnpB-v1), SEQ ID NO: 8 (TnpB-v2), SEQ ID NO: 9 (TnpB-v3), and SEQ ID NO: 10 (Cas12f1), respectively.

Table 4 shows the amino acid sequences of the Cas12f1, TnpB, or the variant proteins as produced above. In addition, Table 5 shows nucleotide sequences of the human codon-optimized nucleic acids encoding the Cas12f1, TnpB, or the variant proteins thereof, respectively. These were used as nucleic acids, each of which encodes the nucleic acid editing protein that constitutes the target nucleic acid editing system in the examples.

**[Table 4]**

| **Name** | **Amino acid sequence** | **SEQ ID NO** |
|---|---|---|
| TnpB protein | MGEKSSRRRRNGKSGAWTAAITSCVGGKMAKNTITKTLKLRI VRPYNSAEVEKIVADEKNNREKIALEKNKDKVKEACSKHLKV | 1 |
| | AAYCTTQVERNACLFCKARKLDDKFYQKLRGQFPDAVFWQEI SEIFRQLQKQAAEIYNQSLIELYYEIFIKGKGIANASSVEHYLSD VCYTRAAELFKNAAIASGLRSKIKSNFRLKELKNMKSGLPTTK SDNFPIPLVKQKGGQYTGFEISNHNSDFIIKIPFGRWQVKKEIDK YRPWEKFDFEQVQKSPKPISLLLSTQRRKRNKGWSKDEGTEAE IKKVMNGDYQTSYIEVKRGSKIGEKSAWMLNLSIDVPKIDKGV DPSIIGGIDVGVKSPLVCAINNAFSRYSISDNDLFHFNKKMFARR RILLKKNRHKRAGHGAKNKLKPITILTEKSERFRKKLIERWACE IADFFIKNKVGTVQMENLESMKRKEDSYFNIRLRGFWPYAEM QNKIEFKLKQYGIEIRKVAPNNTSKTCSKCGHLNNYFNFEYRK KNKFPHFKCEKCNFKENADYNAALNISNPKLKSTKEEP | |
| TnpB-v1 protein | MEKRINKIRKKLSADNATKPVSRSGPMAKNTITKTLKLRIVRPY NSAEVEKIVADEKNNREKIALEKNKDKVKEACSKHLKVAAYC TTQVERNACLFCKARKLDDKFYQKLRGQFPDAVFWQEISEIFR QLQKQAAEIYNQSLIELYYEIFIKGKGIANASSVEHYLSDVCYT RAAELFKNAAIASGLRSKIKSNFRLKELKNMKSGLPTTKSDNF PIPLVKQKGGQYTGFEISNHNSDFIIKIPFGRWQVKKEIDKYRP WEKFDFEQVQKSPKPISLLLSTQRRKRNKGWSKDEGTEAEIKK VMNGDYQTSYIEVKRGSKIGEKSAWMLNLSIDVPKIDKGVDPS IIGGIDVGVKSPLVCAINNAFSRYSISDNDLFHFNKKMFARRRIL LKKNRHKRAGHGAKNKLKPITILTEKSERFRKKLIERWACEIAD FFIKNKVGTVQMENLESMKRKEDSYFNIRLRGFWPYAEMQNK IEFKLKQYGIEIRKVAPNNTSKTCSKCGHLNNYFNFEYRKKNK FPHFKCEKCNFKENADYNAALNISNPKLKSTKEEP | 2 |
| TnpB-v2 protein | MAGGPGAGSAAPVSSTSSLPLAALNMRVMAKNTITKTLKLRIV RPYNSAEVEKIVADEKNNREKIALEKNKDKVKEACSKHLKVA AYCTTQVERNACLFCKARKLDDKFYQKLRGQFPDAVFWQEIS EIFRQLQKQAAEIYNQSLIELYYEIFIKGKGIANASSVEHYLSDV CYTRAAELFKNAAIASGLRSKIKSNFRLKELKNMKSGLPTTKS | 3 |
| | DNFPIPLVKQKGGQYTGFEISNHNSDFIIKIPFGRWQVKKEIDKY RPWEKFDFEQVQKSPKPISLLLSTQRRKRNKGWSKDEGTEAEI KKVMNGDYQTSYIEVKRGSKIGEKSAWMLNLSIDVPKIDKGV DPSIIGGIDVGVKSPLVCAINNAFSRYSISDNDLFHFNKKMFARR RILLKKNRHKRAGHGAKNKLKPITILTEKSERFRKKLIERWACE IADFFIKNKVGTVQMENLESMKRKEDSYFNIRLRGFWPYAEM QNKIEFKLKQYGIEIRKVAPNNTSKTCSKCGHLNNYFNFEYRK KNKFPHFKCEKCNFKENADYNAALNISNPKLKSTKEEP | |
| TnpB-v3 protein | MAGGPGAGSAAPVSSTSSLPLAALNMMAKNTITKTLKLRIVRP YNSAEVEKIVADEKNNREKIALEKNKDKVKEACSKHLKVAAY CTTQVERNACLFCKARKLDDKFYQKLRGQFPDAVFWQEISEIF RQLQKQAAEIYNQSLIELYYEIFIKGKGIANASSVEHYLSDVCY TRAAELFKNAAIASGLRSKIKSNFRLKELKNMKSGLPTTKSDN FPIPLVKQKGGQYTGFEISNHNSDFIIKIPFGRWQVKKEIDKYRP WEKFDFEQVQKSPKPISLLLSTQRRKRNKGWSKDEGTEAEIKK VMNGDYQTSYIEVKRGSKIGEKSAWMLNLSIDVPKIDKGVDPS IIGGIDVGVKSPLVCAINNAFSRYSISDNDLFHFNKKMFARRRIL LKKNRHKRAGHGAKNKLKPITILTEKSERFRKKLIERWACEIAD FFIKNKVGTVQMENLESMKRKEDSYFNIRLRGFWPYAEMQNK IEFKLKQYGIEIRKVAPNNTSKTCSKCGHLNNYFNFEYRKKNK FPHFKCEKCNFKENADYNAALNISNPKLKSTKEEP | 4 |
| Cas12f1 protein | MAKNTITKTLKLRIVRPYNSAEVEKIVADEKNNREKIALEKNK DKVKEACSKHLKVAAYCTTQVERNACLFCKARKLDDKFYQK LRGQFPDAVFWQEISEIFRQLQKQAAEIYNQSLIELYYEIFIKGK GIANASSVEHYLSDVCYTRAAELFKNAAIASGLRSKIKSNFRLK ELKNMKSGLPTTKSDNFPIPLVKQKGGQYTGFEISNHNSDFIIKI PFGRWQVKKEIDKYRPWEKFDFEQVQKSPKPISLLLSTQRRKR NKGWSKDEGTEAEIKKVMNGDYQTSYIEVKRGSKIGEKSAW | 5 |
| | MLNLSIDVPKIDKGVDPSIIGGIDVGVKSPLVCAINNAFSRYSIS DNDLFHFNKKMFARRRILLKKNRHKRAGHGAKNKLKPITILTE KSERFRKKLIERWACEIADFFIKNKVGTVQMENLESMKRKEDS YFNIRLRGFWPYAEMQNKIEFKLKQYGIEIRKVAPNNTSKTCSK CGHLNNYFNFEYRKKNKFPHFKCEKCNFKENADYNAALNISN PKLKSTKEEP | |

**[Table 5]**

| **Name** | **Nucleotide sequence (5' to *S*')** | **SEQ ID NO** |
|---|---|---|
| Human codon-optimized nucleic acid encoding TnpB protein | ATGGGGGAGAAAAGTTCCCGCCGCCGACGGAATGGAAAAA GCGGTGCGTGGACTGCTGCTATAACAAGCTGTGTTGGGGGT AAGATGGCCAAGAACACAATTACAAAGACACTGAAGCTGA GGATCGTGAGACCATACAACAGCGCTGAGGTCGAGAAGATT GTGGCTGATGAAAAGAACAACAGGGAAAAGATCGCCCTCG AGAAGAACAAGGATAAGGTGAAGGAGGCCTGCTCTAAGCA CCTGAAAGTGGCCGCCTACTGCACCACACAGGTGGAGAGG AACGCCTGTCTGTTTTGTAAAGCTCGGAAGCTGGATGATAAG TTTTACCAGAAGCTGCGGGGCCAGTTCCCCGATGCCGTCTTT TGGCAGGAGATTAGCGAGATCTTCAGACAGCTGCAGAAGCA GGCCGCCGAGATCTACAACCAGAGCCTGATCGAGCTCTACT ACGAGATCTTCATCAAGGGCAAGGGCATTGCCAACGCCTCC TCCGTGGAGCACTACCTGAGCGACGTGTGCTACACAAGAGC CGCCGAGCTCTTTAAGAACGCCGCTATCGCTTCCGGGCTGAG GAGCAAGATTAAGAGTAACTTCCGGCTCAAGGAGCTGAAGA ACATGAAGAGCGGCCTGCCCACTACAAAGAGCGACAACTTC CCAATTCCACTGGTGAAGCAGAAGGGGGGCCAGTACACAG GGTTCGAGATTTCCAACCACAACAGCGACTTTATTATTAAGA | 6 |
| | TCCCCTTTGGCAGGTGGCAGGTCAAGAAGGAGATTGACAAG TACAGGCCCTGGGAGAAGTTTGATTTCGAGCAGGTGCAGAA GAGCCCCAAGCCTATTTCCCTGCTGCTGTCCACACAGCGGC GGAAGAGGAACAAGGGGTGGTCTAAGGATGAGGGGACCGA GGCCGAGATTAAGAAAGTGATGAACGGCGACTACCAGACAA GCTACATCGAGGTCAAGCGGGGCAGTAAGATTGGCGAGAAG AGCGCCTGGATGCTGAACCTGAGCATTGACGTGCCAAAGAT TGATAAGGGCGTGGATCCCAGCATCATCGGAGGGATCGATGT GGGGGTCAAGAGCCCCCTCGTGTGCGCCATCAACAACGCCT TCAGCAGGTACAGCATCTCCGATAACGACCTGTTCCACTTTA ACAAGAAGATGTTCGCCCGGCGGAGGATTTTGCTCAAGAAG AACCGGCACAAGCGGGCCGGACACGGGGCCAAGAACAAGC TCAAGCCCATCACTATCCTGACCGAGAAGAGCGAGAGGTTC AGGAAGAAGCTCATCGAGAGATGGGCCTGCGAGATCGCCGA TTTCTTTATTAAGAACAAGGTCGGAACAGTGCAGATGGAGA ACCTCGAGAGCATGAAGAGGAAGGAGGATTCCTACTTCAAC ATTCGGCTGAGGGGGTTCTGGCCCTACGCTGAGATGCAGAA CAAGATTGAGTTTAAGCTGAAGCAGTACGGGATTGAGATCC GGAAGGTGGCCCCCAACAACACCAGCAAGACCTGCAGCAA GTGCGGGCACCTCAACAACTACTTCAACTTCGAGTACCGGA AGAAGAACAAGTTCCCACACTTCAAGTGCGAGAAGTGCAA CTTTAAGGAGAACGCCGATTACAACGCCGCCCTGAACATCA GCAACCCTAAGCTGAAGAGCACTAAGGAGGAGCCC | |
| Human codon-optimized nucleic acid encoding TnpB-v1 protein | ATGGAAAAGAGAATCAACAAGATCAGGAAGAAGCTGAGCG CCGACAACGCCACCAAGCCTGTGTCTAGGAGTGGCCCCCAT GGCCAAGAACACAATTACAAAGACACTGAAGCTGAGGATCG TGAGACCATACAACAGCGCTGAGGTCGAGAAGATTGTGGCT GATGAAAAGAACAACAGGGAAAAGATCGCCCTCGAGAAGA ACAAGGATAAGGTGAAGGAGGCCTGCTCTAAGCACCTGAAA | 7 |
| | GTGGCCGCCTACTGCACCACACAGGTGGAGAGGAACGCCTG TCTGTTTTGTAAAGCTCGGAAGCTGGATGATAAGTTTTACCA GAAGCTGCGGGGCCAGTTCCCCGATGCCGTCTTTTGGCAGG AGATTAGCGAGATCTTCAGACAGCTGCAGAAGCAGGCCGCC GAGATCTACAACCAGAGCCTGATCGAGCTCTACTACGAGATC TTCATCAAGGGCAAGGGCATTGCCAACGCCTCCTCCGTGGA GCACTACCTGAGCGACGTGTGCTACACAAGAGCCGCCGAGC TCTTTAAGAACGCCGCTATCGCTTCCGGGCTGAGGAGCAAG ATTAAGAGTAACTTCCGGCTCAAGGAGCTGAAGAACATGAA GAGCGGCCTGCCCACTACAAAGAGCGACAACTTCCCAATTC CACTGGTGAAGCAGAAGGGGGGCCAGTACACAGGGTTCGA GATTTCCAACCACAACAGCGACTTTATTATTAAGATCCCCTTT GGCAGGTGGCAGGTCAAGAAGGAGATTGACAAGTACAGGC CCTGGGAGAAGTTTGATTTCGAGCAGGTGCAGAAGAGCCCC AAGCCTATTTCCCTGCTGCTGTCCACACAGCGGCGGAAGAG GAACAAGGGGTGGTCTAAGGATGAGGGGACCGAGGCCGAG ATTAAGAAAGTGATGAACGGCGACTACCAGACAAGCTACAT CGAGGTCAAGCGGGGCAGTAAGATTGGCGAGAAGAGCGCC TGGATGCTGAACCTGAGCATTGACGTGCCAAAGATTGATAAG GGCGTGGATCCCAGCATCATCGGAGGGATCGATGTGGGGGT CAAGAGCCCCCTCGTGTGCGCCATCAACAACGCCTTCAGCA GGTACAGCATCTCCGATAACGACCTGTTCCACTTTAACAAGA AGATGTTCGCCCGGCGGAGGATTTTGCTCAAGAAGAACCGG CACAAGCGGGCCGGACACGGGGCCAAGAACAAGCTCAAGC CCATCACTATCCTGACCGAGAAGAGCGAGAGGTTCAGGAAG AAGCTCATCGAGAGATGGGCCTGCGAGATCGCCGATTTCTTT ATTAAGAACAAGGTCGGAACAGTGCAGATGGAGAACCTCGA GAGCATGAAGAGGAAGGAGGATTCCTACTTCAACATTCGGC TGAGGGGGTTCTGGCCCTACGCTGAGATGCAGAACAAGATT GAGTTTAAGCTGAAGCAGTACGGGATTGAGATCCGGAAGGT GGCCCCCAACAACACCAGCAAGACCTGCAGCAAGTGCGGG CACCTCAACAACTACTTCAACTTCGAGTACCGGAAGAAGAA CAAGTTCCCACACTTCAAGTGCGAGAAGTGCAACTTTAAGG | |
| | AGAACGCCGATTACAACGCCGCCCTGAACATCAGCAACCCT AAGCTGAAGAGCACTAAGGAGGAGCCC | |
| Human codon-optimized nucleic acid encoding TnpB-v2 protein | ATGGCTGGCGGACCAGGCGCAGGTAGTGCTGCGCCAGTTTC TTCAACTTCCTCCCTGCCCCTGGCTGCGCTTAACATGCGCGT GATGGCCAAGAACACAATTACAAAGACACTGAAGCTGAGG ATCGTGAGACCATACAACAGCGCTGAGGTCGAGAAGATTGT GGCTGATGAAAAGAACAACAGGGAAAAGATCGCCCTCGAG AAGAACAAGGATAAGGTGAAGGAGGCCTGCTCTAAGCACCT GAAAGTGGCCGCCTACTGCACCACACAGGTGGAGAGGAAC GCCTGTCTGTTTTGTAAAGCTCGGAAGCTGGATGATAAGTTT TACCAGAAGCTGCGGGGCCAGTTCCCCGATGCCGTCTTTTG GCAGGAGATTAGCGAGATCTTCAGACAGCTGCAGAAGCAGG CCGCCGAGATCTACAACCAGAGCCTGATCGAGCTCTACTACG AGATCTTCATCAAGGGCAAGGGCATTGCCAACGCCTCCTCC GTGGAGCACTACCTGAGCGACGTGTGCTACACAAGAGCCGC CGAGCTCTTTAAGAACGCCGCTATCGCTTCCGGGCTGAGGA GCAAGATTAAGAGTAACTTCCGGCTCAAGGAGCTGAAGAAC ATGAAGAGCGGCCTGCCCACTACAAAGAGCGACAACTTCCC AATTCCACTGGTGAAGCAGAAGGGGGGCCAGTACACAGGG TTCGAGATTTCCAACCACAACAGCGACTTTATTATTAAGATC CCCTTTGGCAGGTGGCAGGTCAAGAAGGAGATTGACAAGTA CAGGCCCTGGGAGAAGTTTGATTTCGAGCAGGTGCAGAAGA GCCCCAAGCCTATTTCCCTGCTGCTGTCCACACAGCGGCGG AAGAGGAACAAGGGGTGGTCTAAGGATGAGGGGACCGAGG CCGAGATTAAGAAAGTGATGAACGGCGACTACCAGACAAGC TACATCGAGGTCAAGCGGGGCAGTAAGATTGGCGAGAAGAG CGCCTGGATGCTGAACCTGAGCATTGACGTGCCAAAGATTG ATAAGGGCGTGGATCCCAGCATCATCGGAGGGATCGATGTGG GGGTCAAGAGCCCCCTCGTGTGCGCCATCAACAACGCCTTC AGCAGGTACAGCATCTCCGATAACGACCTGTTCCACTTTAAC AAGAAGATGTTCGCCCGGCGGAGGATTTTGCTCAAGAAGAA | 8 |
| | CCGGCACAAGCGGGCCGGACACGGGGCCAAGAACAAGCTC AAGCCCATCACTATCCTGACCGAGAAGAGCGAGAGGTTCAG GAAGAAGCTCATCGAGAGATGGGCCTGCGAGATCGCCGATT TCTTTATTAAGAACAAGGTCGGAACAGTGCAGATGGAGAAC CTCGAGAGCATGAAGAGGAAGGAGGATTCCTACTTCAACAT TCGGCTGAGGGGGTTCTGGCCCTACGCTGAGATGCAGAACA AGATTGAGTTTAAGCTGAAGCAGTACGGGATTGAGATCCGG AAGGTGGCCCCCAACAACACCAGCAAGACCTGCAGCAAGT GCGGGCACCTCAACAACTACTTCAACTTCGAGTACCGGAAG AAGAACAAGTTCCCACACTTCAAGTGCGAGAAGTGCAACTT TAAGGAGAACGCCGATTACAACGCCGCCCTGAACATCAGCA ACCCTAAGCTGAAGAGCACTAAGGAGGAGCCC | |
| Human codon-optimized nucleic acid encoding TnpB-v3 protein | ATGGCTGGCGGACCAGGCGCAGGTAGTGCTGCGCCAGTTTC TTCAACTTCCTCCCTGCCCCTGGCTGCGCTTAACATGATGGC CAAGAACACAATTACAAAGACACTGAAGCTGAGGATCGTGA GACCATACAACAGCGCTGAGGTCGAGAAGATTGTGGCTGAT GAAAAGAACAACAGGGAAAAGATCGCCCTCGAGAAGAACA AGGATAAGGTGAAGGAGGCCTGCTCTAAGCACCTGAAAGTG GCCGCCTACTGCACCACACAGGTGGAGAGGAACGCCTGTCT GTTTTGTAAAGCTCGGAAGCTGGATGATAAGTTTTACCAGAA GCTGCGGGGCCAGTTCCCCGATGCCGTCTTTTGGCAGGAGA TTAGCGAGATCTTCAGACAGCTGCAGAAGCAGGCCGCCGAG ATCTACAACCAGAGCCTGATCGAGCTCTACTACGAGATCTTC ATCAAGGGCAAGGGCATTGCCAACGCCTCCTCCGTGGAGCA CTACCTGAGCGACGTGTGCTACACAAGAGCCGCCGAGCTCT TTAAGAACGCCGCTATCGCTTCCGGGCTGAGGAGCAAGATT AAGAGTAACTTCCGGCTCAAGGAGCTGAAGAACATGAAGA GCGGCCTGCCCACTACAAAGAGCGACAACTTCCCAATTCCA CTGGTGAAGCAGAAGGGGGGCCAGTACACAGGGTTCGAGA TTTCCAACCACAACAGCGACTTTATTATTAAGATCCCCTTTGG CAGGTGGCAGGTCAAGAAGGAGATTGACAAGTACAGGCCC | 9 |
| | TGGGAGAAGTTTGATTTCGAGCAGGTGCAGAAGAGCCCCAA GCCTATTTCCCTGCTGCTGTCCACACAGCGGCGGAAGAGGA ACAAGGGGTGGTCTAAGGATGAGGGGACCGAGGCCGAGATT AAGAAAGTGATGAACGGCGACTACCAGACAAGCTACATCGA GGTCAAGCGGGGCAGTAAGATTGGCGAGAAGAGCGCCTGG ATGCTGAACCTGAGCATTGACGTGCCAAAGATTGATAAGGG CGTGGATCCCAGCATCATCGGAGGGATCGATGTGGGGGTCA AGAGCCCCCTCGTGTGCGCCATCAACAACGCCTTCAGCAGG TACAGCATCTCCGATAACGACCTGTTCCACTTTAACAAGAAG ATGTTCGCCCGGCGGAGGATTTTGCTCAAGAAGAACCGGCA CAAGCGGGCCGGACACGGGGCCAAGAACAAGCTCAAGCCC ATCACTATCCTGACCGAGAAGAGCGAGAGGTTCAGGAAGAA GCTCATCGAGAGATGGGCCTGCGAGATCGCCGATTTCTTTAT TAAGAACAAGGTCGGAACAGTGCAGATGGAGAACCTCGAG AGCATGAAGAGGAAGGAGGATTCCTACTTCAACATTCGGCT GAGGGGGTTCTGGCCCTACGCTGAGATGCAGAACAAGATTG AGTTTAAGCTGAAGCAGTACGGGATTGAGATCCGGAAGGTG GCCCCCAACAACACCAGCAAGACCTGCAGCAAGTGCGGGC ACCTCAACAACTACTTCAACTTCGAGTACCGGAAGAAGAAC AAGTTCCCACACTTCAAGTGCGAGAAGTGCAACTTTAAGGA GAACGCCGATTACAACGCCGCCCTGAACATCAGCAACCCTA AGCTGAAGAGCACTAAGGAGGAGCCC | |
| Human codon-optimized nucleic acid encoding Cas12f1 protein | ATGGCCAAGAACACAATTACAAAGACACTGAAGCTGAGGAT CGTGAGACCATACAACAGCGCTGAGGTCGAGAAGATTGTGG CTGATGAAAAGAACAACAGGGAAAAGATCGCCCTCGAGAA GAACAAGGATAAGGTGAAGGAGGCCTGCTCTAAGCACCTGA AAGTGGCCGCCTACTGCACCACACAGGTGGAGAGGAACGC CTGTCTGTTTTGTAAAGCTCGGAAGCTGGATGATAAGTTTTA CCAGAAGCTGCGGGGCCAGTTCCCCGATGCCGTCTTTTGGC AGGAGATTAGCGAGATCTTCAGACAGCTGCAGAAGCAGGCC GCCGAGATCTACAACCAGAGCCTGATCGAGCTCTACTACGA | 10 |
| | GATCTTCATCAAGGGCAAGGGCATTGCCAACGCCTCCTCCGT GGAGCACTACCTGAGCGACGTGTGCTACACAAGAGCCGCCG AGCTCTTTAAGAACGCCGCTATCGCTTCCGGGCTGAGGAGC AAGATTAAGAGTAACTTCCGGCTCAAGGAGCTGAAGAACAT GAAGAGCGGCCTGCCCACTACAAAGAGCGACAACTTCCCA ATTCCACTGGTGAAGCAGAAGGGGGGCCAGTACACAGGGTT CGAGATTTCCAACCACAACAGCGACTTTATTATTAAGATCCC CTTTGGCAGGTGGCAGGTCAAGAAGGAGATTGACAAGTACA GGCCCTGGGAGAAGTTTGATTTCGAGCAGGTGCAGAAGAGC CCCAAGCCTATTTCCCTGCTGCTGTCCACACAGCGGCGGAA GAGGAACAAGGGGTGGTCTAAGGATGAGGGGACCGAGGCC GAGATTAAGAAAGTGATGAACGGCGACTACCAGACAAGCTA CATCGAGGTCAAGCGGGGCAGTAAGATTGGCGAGAAGAGC GCCTGGATGCTGAACCTGAGCATTGACGTGCCAAAGATTGAT AAGGGCGTGGATCCCAGCATCATCGGAGGGATCGATGTGGG GGTCAAGAGCCCCCTCGTGTGCGCCATCAACAACGCCTTCA GCAGGTACAGCATCTCCGATAACGACCTGTTCCACTTTAACA AGAAGATGTTCGCCCGGCGGAGGATTTTGCTCAAGAAGAAC CGGCACAAGCGGGCCGGACACGGGGCCAAGAACAAGCTCA AGCCCATCACTATCCTGACCGAGAAGAGCGAGAGGTTCAGG AAGAAGCTCATCGAGAGATGGGCCTGCGAGATCGCCGATTT CTTTATTAAGAACAAGGTCGGAACAGTGCAGATGGAGAACC TCGAGAGCATGAAGAGGAAGGAGGATTCCTACTTCAACATT CGGCTGAGGGGGTTCTGGCCCTACGCTGAGATGCAGAACAA GATTGAGTTTAAGCTGAAGCAGTACGGGATTGAGATCCGGA AGGTGGCCCCCAACAACACCAGCAAGACCTGCAGCAAGTG CGGGCACCTCAACAACTACTTCAACTTCGAGTACCGGAAGA AGAACAAGTTCCCACACTTCAAGTGCGAGAAGTGCAACTTT AAGGAGAACGCCGATTACAACGCCGCCCTGAACATCAGCAA CCCTAAGCTGAAGAGCACTAAGGAGGAGCCC | |

The hypercompact gene editing nucleic acid construct as produced above was constructed by the following method. The nucleic acid construct used in the present disclosure comprises a gene sequence of human codon-optimized Cas12f1, TnpB, or a variant thereof (including an engineered variant). PCR amplification was performed using the gene sequence as a template, and cloning was performed according to a desired cloning sequence for a vector having a promoter capable of expression in a eukaryotic system and a polyA signal sequence by the Gibson assembly method. The sequence of a plasmid vector obtained after cloning was finally identified by the Sanger sequencing method.

### Example 1.2. Expression and purification of Cas12f1 and TnpB proteins

The gene produced in Example 1.1 was expressed, and the protein was purified.

First, the nucleic acid construct was cloned into the pMAL-c2 plasmid vector and transformed into BL21 (DE3) *E. coli* cells. The transformed *E. coli* colonies were grown in LB broth at 37°C until the optical density reached 0.7. The transformed *E. coli* cells were cultured at 18°C overnight in the presence of 0.1 mM isopropylthio-β-D-galactoside. The cells were then centrifuged at 3,500 g for 30 minutes and collected. The collected cells were resuspended in 20 mM Tris-HCl (pH 7.6), 500 mM NaCl, 5 mM β-mercaptoethanol, 5% glycerol. The cells were lysed in lysis buffer and then disrupted by sonication. The sample containing the disrupted cells was centrifuged at 15,000 g for 30 minutes, and the obtained supernatant was filtered through a 0.45 µm syringe filter (Millipore), and the filtered supernatant was loaded onto a Ni²⁺-affinity column using a FPLC purification system (KTA Purifier, GE Healthcare). The bound fractions were eluted at a gradient of 80 to 400 mM imidazole, 20 mM Tris-HCl (pH 7.5).

The eluted protein was cleaved by treatment with TEV protease for 16 hours. The cleaved protein was purified on a heparin column with a linear gradient of 0.15 to 1.6 M NaCl. The recombinant Cas12f1 variant proteins purified on the heparin column was dialyzed against a solution of 20 mM Tris (pH 7.6), 150 mM NaCl, 5 mM β-mercaptoethanol, 5% glycerol. The dialyzed proteins were purified by passing through an MBP column, and then re-purified on a monoS column (GE Healthcare) or EnrichS with a linear gradient of 0.5 to 1.2 M NaCl.

The re-purified proteins were pooled and dialyzed against a solution of 20 mM Tris (pH 7.6), 150 mM NaCl, 5 mM β-mercaptoethanol, 5% glycerol for purification of the hypercompact gene editing proteins (miniature endonucleases) used in the present disclosure. The concentration of the produced hypercompact gene editing proteins was quantified using the Bradford quantitative method using bovine serum albumin (BSA) as a standard and was electrophoretically determined on a Coomassie Blue-stained SDS-PAGE gel.

### Example 1.3. Construction of guide RNA used in nucleic acid editing system for homology-directed repair

For guide RNA (gRNA), which is a component of the nucleic acid editing system (TaRGET system) for use in homology-directed repair, a plurality of engineered gRNAs were tested that have a modification(s) at five major regions (designated as MS1 to MS5, respectively) as illustrated in FIG. 1. Exemplary sequences of the gRNA are shown in Table 6.

**[Table 6]**

| gRNA | Sequence (5' to 3') | SEQ ID NO |
|---|---|---|
| Canonical sgRNA | CUUCACUGAUAAAGUGGAGAACCGCUUCACCAAAAGC UGUCCCuuagGGGAUUAGAACUUGAGUGAAGGUGGGCU GCUUGCAUCAGCCUAAUGUCGAGAAGUGCUUUCUUCG GAAAGUAACCCUCGAAACAAAUUCAUUUUUCCUCUCC AAUUCUGCACAAgaaaGUUGCAGAACCCGAAUAGacgaaU GAAGGAAUGCAACNNNNNNNNNNNNNNNNNNNN | 13 |
| MS1 | CUUCACUGAUAAAGUGGAGAACCGCUUCACCAAAAGC UGUCCCUUAGGGGAUUAGAACUUGAGUGAAGGUGGGC UGCUUGCAUCAGCCUAAUGUCGAGAAGUGCUUUCUUC GGAAAGUAACCCUCGAAACAAAUUCAGUGCUCCUCUC CAAUUCUGCACAAgaaaGUUGCAGAACCCGAAUAGAGC AAUGAAGGAAUGCAACNNNNNNNNNNNNNNNNNNNN | 149 |
| MS1/MS2 | CUUCACUGAUAAAGUGGAGAACCGCUUCACCAAAAGC UGUCCCUUAGGGGAUUAGAACUUGAGUGAAGGUGGGC UGCUUGCAUCAGCCUAAUGUCGAGAAGUGCUUUCUUC GGAAAGUAACCCUCGAAACAAAUUCAGUGCUCCUCUC CAAUUCUGCACAAgaaaGUUGCAGAACCCGAAUAGAGC AAUGAAGGAAUGCAACNNNNNNNNNNNNNNNNNNNN | 150 |
| | UUUUAUUUUUU | |
| MS1/MS2/MS 3 (ge3.0) | ACCGCUUCACCAAAAGCUGUCCCUUAGGGGAUUAGAA CUUGAGUGAAGGUGGGCUGCUUGCAUCAGCCUAAUGU CGAGAAGUGCUUUCUUCGGAAAGUAACCCUCGAAACA AAUUCAGUGCUCCUCUCCAAUUCUGCACAAgaaaGUUGC AGAACCCGAAUAGAGCAAUGAAGGAAUGCAACNNNNN NNNNNNNNNNNNNNNUUUUAUUUUUU | 151 |
| MS2/MS3/MS 4(ge4.0) | ACCGCUUCACCAAAAGCUGUCCCUUAGGGGAUUAGAA CUUGAGUGAAGGUGGGCUGCUUGCAUCAGCCUAAUGU CGAGAAGUGCUUUCUUCGGAAAGUAACCCUCGAAACA AAgaaaGGAAUGCAACNNNNNNNNNNNNNNNNNNNNUU UUAUUUUUU | 152 |
| MS2/MS3/MS 4/MS5(ge4.1) | ACCGCUUCACUUAGAGUGAAGGUGGGCUGCUUGCAUC AGCCUAAUGUCGAGAAGUGCUUUCUUCGGAAAGUAAC CCUCGAAACAAAgaaaGGAAUGCAACNNNNNNNNNNNN NNNNNNNNUUUUAUUUUUU | 153 |
| MS1/MS3-1 | GAUAAAGUGGAGAACCGCUUCACCAAAAGCUGUCCCU UAGGGGAUUAGAACUUGAGUGAAGGUGGGCUGCUUGC AUCAGCCUAAUGUCGAGAAGUGCUUUCUUCGGAAAGU AACCCUCGAAACAAAUUCAGUGCUCCUCUCCAAUUCU GCACAAgaaaGUUGCAGAACCCGAAUAGAGCAAUGAAG GAAUGCAACNNNNNNNNNNNNNNNNNNNN | 154 |
| MS1/MS3-2 | UGGAGAACCGCUUCACCAAAAGCUGUCCCUUAGGGGA UUAGAACUUGAGUGAAGGUGGGCUGCUUGCAUCAGCC UAAUGUCGAGAAGUGCUUUCUUCGGAAAGUAACCCUC GAAACAAAUUCAGUGCUCCUCUCCAAUUCUGCACAAga aaGUUGCAGAACCCGAAUAGAGCAAUGAAGGAAUGCA ACNNNNNNNNNNNNNNNNNNNN | 155 |
| MS1/MS3-3 | ACCGCUUCACCAAAAGCUGUCCCUUAGGGGAUUAGAA CUUGAGUGAAGGUGGGCUGCUUGCAUCAGCCUAAUGU | 156 |
| | CGAGAAGUGCUUUCUUCGGAAAGUAACCCUCGAAACA AAUUCAGUGCUCCUCUCCAAUUCUGCACAAgaaaGUUGC AGAACCCGAAUAGAGCAAUGAAGGAAUGCAACNNNNN NNNNNNNNNNNNNNN | |
| MS1/MS4*-1 | CUUCACUGAUAAAGUGGAGAACCGCUUCACCAAAAGC UGUCCCUUAGGGGAUUAGAACUUGAGUGAAGGUGGGC UGCUUGCAUCAGCCUAAUGUCGAGAAGUGCUUUCUUC GGAAAGUAACCCUCGAAACAAAUUCAGUGCUCCUCUC CAAUUCgaaaGAACCCGAAUAGAGCAAUGAAGGAAUGC AACNNNNNNNNNNNNNNNNNNNN | 157 |
| MS1/MS4*-2 | CUUCACUGAUAAAGUGGAGAACCGCUUCACCAAAAGC UGUCCCUUAGGGGAUUAGAACUUGAGUGAAGGUGGGC UGCUUGCAUCAGCCUAAUGUCGAGAAGUGCUUUCUUC GGAAAGUAACCCUCGAAACAAAUUCAGUGCUCCUCUC gaaaGAAUAGAGCAAUGAAGGAAUGCAACNNNNNNNNN NNNNNNNNNNN | 158 |
| MS1/MS4*-3 | CUUCACUGAUAAAGUGGAGAACCGCUUCACCAAAAGC UGUCCCUUAGGGGAUUAGAACUUGAGUGAAGGUGGGC UGCUUGCAUCAGCCUAAUGUCGAGAAGUGCUUUCUUC GGAAAGUAACCCUCGAAACAAAUUCAGUGCUgaaaAGC AAUGAAGGAAUGCAACNNNNNNNNNNNNNNNNNNNN | 159 |
| MS1/MS5-1 | CUUCACUGAUAAAGUGGAGAACCGCUUCACCAAAAGC UGUuuagAUUAGAACUUGAGUGAAGGUGGGCUGCUUGC AUCAGCCUAAUGUCGAGAAGUGCUUUCUUCGGAAAGU AACCCUCGAAACAAAUUCAGUGCUCCUCUCCAAUUCU GCACAAgaaaGUUGCAGAACCCGAAUAGAGCAAUGAAG GAAUGCAACNNNNNNNNNNNNNNNNNNNN | 160 |
| MS1/MS5-2 | CUUCACUGAUAAAGUGGAGAACCGCUUCACCAAAAGC uuagGAACUUGAGUGAAGGUGGGCUGCUUGCAUCAGCC UAAUGUCGAGAAGUGCUUUCUUCGGAAAGUAACCCUC | 161 |
| | GAAACAAAUUCAGUGCUCCUCUCCAAUUCUGCACAAga aaGUUGCAGAACCCGAAUAGAGCAAUGAAGGAAUGCA ACNNNNNNNNNNNNNNNNNNNN | |
| MS1/MS5-3 | CUUCACUGAUAAAGUGGAGAACCGCUUCACCAAuuagU UGAGUGAAGGUGGGCUGCUUGCAUCAGCCUAAUGUCG AGAAGUGCUUUCUUCGGAAAGUAACCCUCGAAACAAA UUCAGUGCUCCUCUCCAAUUCUGCACAAgaaaGUUGCAG AACCCGAAUAGAGCAAUGAAGGAAUGCAACNNNNNNN NNNNNNNNNNNNN | 162 |
| MS1/MS2/MS 4*-2 | CUUCACUGAUAAAGUGGAGAACCGCUUCACCAAAAGC UGUCCCUUAGGGGAUUAGAACUUGAGUGAAGGUGGGC UGCUUGCAUCAGCCUAAUGUCGAGAAGUGCUUUCUUC GGAAAGUAACCCUCGAAACAAAUUCAGUGCUCCUCUC gaaaGAAUAGAGCAAUGAAGGAAUGCAACNNNNNNNNN NNNNNNNNNNNUUUUAUUUU | 163 |
| MS1/MS3-3/MS4*-2 | ACCGCUUCACCAAAAGCUGUCCCUUAGGGGAUUAGAA CUUGAGUGAAGGUGGGCUGCUUGCAUCAGCCUAAUGU CGAGAAGUGCUUUCUUCGGAAAGUAACCCUCGAAACA AAUUCAGUGCUCCUCUCgaaaGAAUAGAGCAAUGAAGG AAUGCAACNNNNNNNNNNNNNNNNNNNN | 164 |
| MS1/MS2/MS 5-3 | CUUCACUGAUAAAGUGGAGAACCGCUUCACCAAuuagU UGAGUGAAGGUGGGCUGCUUGCAUCAGCCUAAUGUCG AGAAGUGCUUUCUUCGGAAAGUAACCCUCGAAACAAA UUCAGUGCUCCUCUCCAAUUCUGCACAAgaaaGUUGCAG AACCCGAAUAGAGCAAUGAAGGAAUGCAACNNNNNNN NNNNNNNNNNNNNUUUUAUUUU | 165 |
| MS1/MS3-3/MS5-3 | ACCGCUUCACCAAuuagUUGAGUGAAGGUGGGCUGCUU GCAUCAGCCUAAUGUCGAGAAGUGCUUUCUUCGGAAA GUAACCCUCGAAACAAAUUCAGUGCUCCUCUCCAAUU CUGCACAAgaaaGUUGCAGAACCCGAAUAGAGCAAUGA | 166 |
| | AGGAAUGCAACNNNNNNNNNNNNNNNNNNNNNNNN | |
| MS1/MS4*-2/MS5-3 | CUUCACUGAUAAAGUGGAGAACCGCUUCACCAAuuagU UGAGUGAAGGUGGGCUGCUUGCAUCAGCCUAAUGUCG AGAAGUGCUUUCUUCGGAAAGUAACCCUCGAAACAAA UUCAGUGCUCCUCUCgaaaGAAUAGAGCAAUGAAGGAA UGCAACNNNNNNNNNNNNNNNNNNNN | 167 |
| MS1/MS2/MS 3-3/MS4*-2 | ACCGCUUCACCAAAAGCUGUCCCuuagGGGAUUAGAAC UUGAGUGAAGGUGGGCUGCUUGCAUCAGCCUAAUGUC GAGAAGUGCUUUCUUCGGAAAGUAACCCUCGAAACAA AUUCAGUGCUCCUCUCgaaaGAAUAGAGCAAUGAAGGA AUGCAACNNNNNNNNNNNNNNNNNNNNUUUUAUUUU | 168 |
| MS1/MS2/MS 3-3/MS5-3 | ACCGCUUCACCAAuuagUUGAGUGAAGGUGGGCUGCUU GCAUCAGCCUAAUGUCGAGAAGUGCUUUCUUCGGAAA GUAACCCUCGAAACAAAUUCAGUGCUCCUCUCCAAUU CUGCACAAgaaaGUUGCAGAACCCGAAUAGAGCAAUGA AGGAAUGCAACNNNNNNNNNNNNNNNNNNNNUUUUA UUUU | 169 |
| MS1/MS2/MS 4*-2/MS5-3 | CUUCACUGAUAAAGUGGAGAACCGCUUCACCAAuuagU UGAGUGAAGGUGGGCUGCUUGCAUCAGCCUAAUGUCG AGAAGUGCUUUCUUCGGAAAGUAACCCUCGAAACAAA UUCAGUGCUCCUCUCgaaaGAAUAGAGCAAUGAAGGAA UGCAACNNNNNNNNNNNNNNNNNNNNUUUUAUUUU | 170 |
| MS1/MS3-3/MS4*-2/MS5-3 | ACCGCUUCACCAAuuagUUGAGUGAAGGUGGGCUGCUU GCAUCAGCCUAAUGUCGAGAAGUGCUUUCUUCGGAAA GUAACCCUCGAAACAAAUUCAGUGCUCCUCUCgaaaGAA UAGAGCAAUGAAGGAAUGCAACNNNNNNNNNNNNNNN NNNNNN | 171 |
| MS1/MS2/MS 3-3/MS4*-2/MS5-3 | ACCGCUUCACCAAuuagUUGAGUGAAGGUGGGCUGCUU GCAUCAGCCUAAUGUCGAGAAGUGCUUUCUUCGGAAA GUAACCCUCGAAACAAAUUCAGUGCUCCUCUCgaaaGAA | 172 |
| | UAGAGCAAUGAAGGAAUGCAACNNNNNNNNNNNNNN NNNNNNUUUUAUUUU | |

In addition, a mature form of gRNA (mature form gRNA) was produced by removing the MS1 sequence, which is one of the modification sites, from the canonical sgRNA. Exemplary sequences of the mature form gRNA are shown in Table 7

**[Table 7]**

| gRNA | Sequence (5' to 3') | SEQ ID NO |
|---|---|---|
| Mature form gRNA | CUUCACUGAUAAAGUGGAGAACCGCUUCACCAAAAGC UGUCCCuuagGGGAUUAGAACUUGAGUGAAGGUGGGCU GCUUGCAUCAGCCUAAUGUCGAGAAGUGCUUUCUUCG GAAAGUAACCCUCGAAACAAAUUCAUUUgaaaGAAUGA AGGAAUGCAACNNNNNNNNNNNNNNNNNNNN | 173 |
| MS3-1 | GAUAAAGUGGAGAACCGCUUCACCAAAAGCUGUCCCuu agGGGAUUAGAACUUGAGUGAAGGUGGGCUGCUUGCA UCAGCCUAAUGUCGAGAAGUGCUUUCUUCGGAAAGUA ACCCUCGAAACAAAUUCAUUUgaaaGAAUGAAGGAAUG CAACNNNNNNNNNNNNNNNNNNNN | 174 |
| MS3-2 | UGGAGAACCGCUUCACCAAAAGCUGUCCCuuagGGGAU UAGAACUUGAGUGAAGGUGGGCUGCUUGCAUCAGCCU AAUGUCGAGAAGUGCUUUCUUCGGAAAGUAACCCUCG AAACAAAUUCAUUUgaaaGAAUGAAGGAAUGCAACNNN NNNNNNNNNNNNNNNNN | 175 |
| MS3-3 | ACCGCUUCACCAAAAGCUGUCCCuuagGGGAUUAGAAC UUGAGUGAAGGUGGGCUGCUUGCAUCAGCCUAAUGUC GAGAAGUGCUUUCUUCGGAAAGUAACCCUCGAAACAA AUUCAUUUgaaaGAAUGAAGGAAUGCAACNNNNNNNNN NNNNNNNNNNN | 176 |
| MS4-1 | CUUCACUGAUAAAGUGGAGAACCGCUUCACCAAAAGC | 177 |
| | UGUCCCuuagGGGAUUAGAACUUGAGUGAAGGUGGGCU GCUUGCAUCAGCCUAAUGUCGAGAAGUGCUUUCUUCG GAAAGUAACCCUCGAAACAAAUUCAUgaaaAUGAAGGA AUGCAACNNNNNNNNNNNNNNNNNNNN | |
| MS4-2 | CUUCACUGAUAAAGUGGAGAACCGCUUCACCAAAAGC UGUCCCuuagGGGAUUAGAACUUGAGUGAAGGUGGGCU GCUUGCAUCAGCCUAAUGUCGAGAAGUGCUUUCUUCG GAAAGUAACCCUCGAAACAAAUUCgaaaGAAGGAAUGC AACNNNNNNNNNNNNNNNNNNNN | 178 |
| MS4-3 | CUUCACUGAUAAAGUGGAGAACCGCUUCACCAAAAGC UGUCCCuuagGGGAUUAGAACUUGAGUGAAGGUGGGCU GCUUGCAUCAGCCUAAUGUCGAGAAGUGCUUUCUUCG GAAAGUAACCCUCGAAACAAAgaaaGGAAUGCAACNNN NNNNNNNNNNNNNNNNN | 179 |
| MS5-1 | CUUCACUGAUAAAGUGGAGAACCGCUUCACCAAAAGC UGUuuagAUUAGAACUUGAGUGAAGGUGGGCUGCUUGC AUCAGCCUAAUGUCGAGAAGUGCUUUCUUCGGAAAGU AACCCUCGAAACAAAUUCAUUUgaaaGAAUGAAGGAAU GCAACNNNNNNNNNNNNNNNNNNNN | 180 |
| MS5-2 | CUUCACUGAUAAAGUGGAGAACCGCUUCACCAAAAGC UuuagAGAACUUGAGUGAAGGUGGGCUGCUUGCAUCAG CCUAAUGUCGAGAAGUGCUUUCUUCGGAAAGUAACCC UCGAAACAAAUUCAUUUgaaaGAAUGAAGGAAUGCAAC NNNNNNNNNNNNNNNNNNNN | 181 |
| MS5-3 | CUUCACUGAUAAAGUGGAGAACCGCUUCACCAAuuagU UGAGUGAAGGUGGGCUGCUUGCAUCAGCCUAAUGUCG AGAAGUGCUUUCUUCGGAAAGUAACCCUCGAAACAAA UUCAUUUgaaaGAAUGAAGGAAUGCAACNNNNNNNNNN NNNNNNNNNN | 182 |
| MS3-3/MS4-3 | ACCGCUUCACCAAAAGCUGUCCCuuagGGGAUUAGAAC | 183 |
| | UUGAGUGAAGGUGGGCUGCUUGCAUCAGCCUAAUGUC GAGAAGUGCUUUCUUCGGAAAGUAACCCUCGAAACAA AgaaaGGAAUGCAACNNNNNNNNNNNNNNNNNNNN | |
| MS3-3/MS5-3 | ACCGCUUCACCAAuuagUUGAGUGAAGGUGGGCUGCUU GCAUCAGCCUAAUGUCGAGAAGUGCUUUCUUCGGAAA GUAACCCUCGAAACAAAUUCAUUUgaaaGAAUGAAGGA AUGCAACNNNNNNNNNNNNNNNNNNNN | 184 |
| MS4-3/MS5-3 | CUUCACUGAUAAAGUGGAGAACCGCUUCACCAAuuagU UGAGUGAAGGUGGGCUGCUUGCAUCAGCCUAAUGUCG AGAAGUGCUUUCUUCGGAAAGUAACCCUCGAAACAAA gaaaGGAAUGCAACNNNNNNNNNNNNNNNNNNNN | 185 |
| MS3-3/MS4-3/MS5-3 | ACCGCUUCACCAAuuagUUGAGUGAAGGUGGGCUGCUU GCAUCAGCCUAAUGUCGAGAAGUGCUUUCUUCGGAAA GUAACCCUCGAAACAAAgaaaGGAAUGCAACNNNNNNN NNNNNNNNNNNNN | 186 |

The sequence indicated by 'NNNNNNNNNNNNNNNNNNNN' in Tables 6 and 7 refers to any guide sequence (spacer sequence) that can hybridize with a target sequence in a target gene. The guide sequence may be appropriately designed by a person skilled in the art depending on a desired target gene and/or a target sequence in the target gene, and thus is not limited to a specific sequence of a particular length.

### Example 2. Homology-directed repair (HDR) using nucleic acid editing system

### Example 2.1. Analysis of homology-directed repair efficiency of nucleic acid editing system

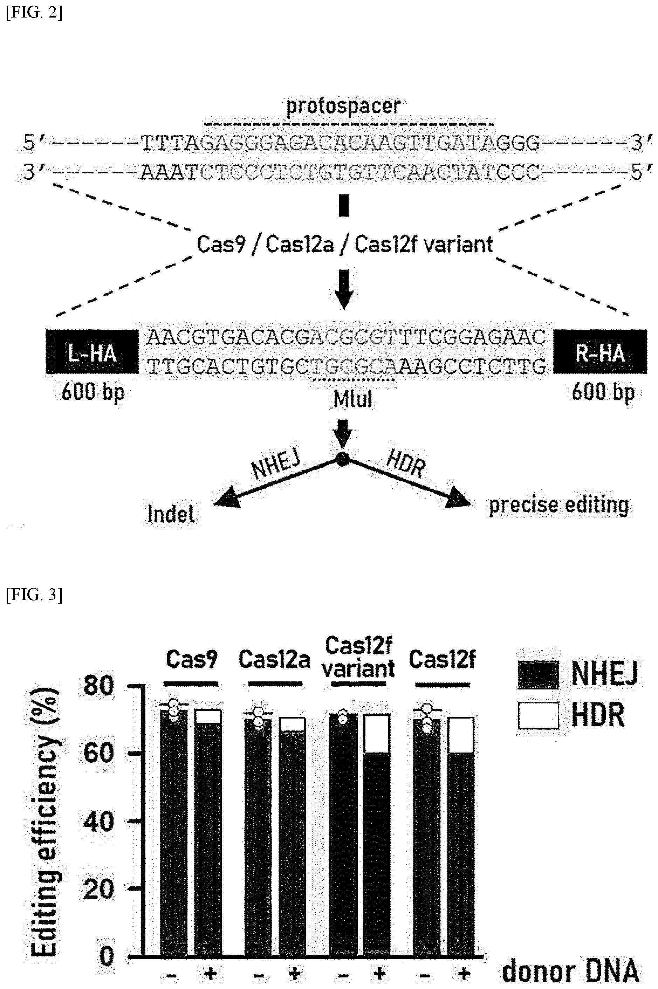

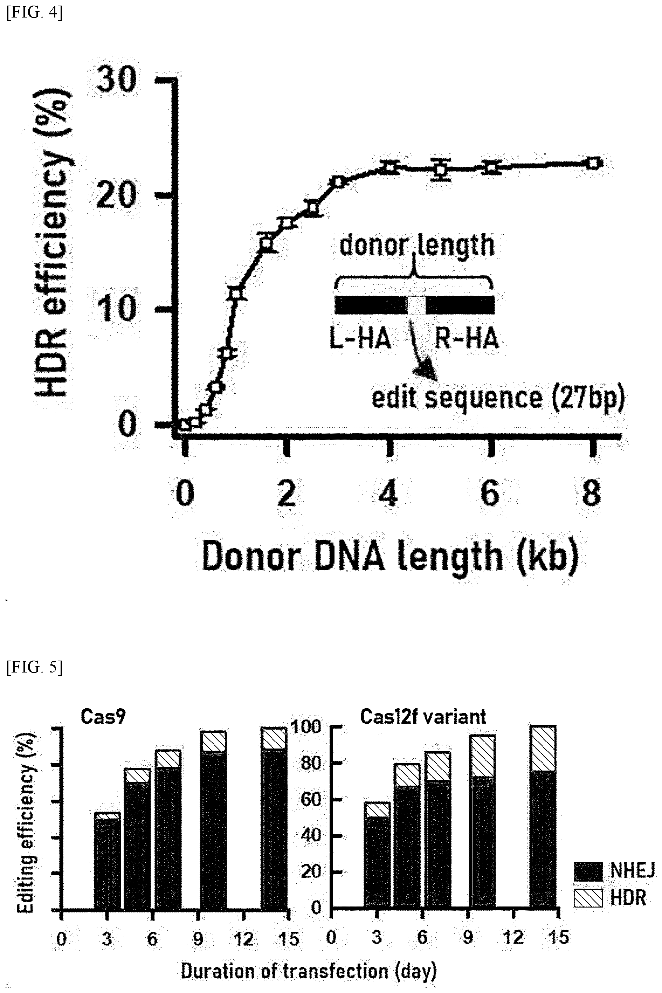

Homology-directed repair requires a nucleic acid degrading enzyme for double-strand breaks in DNA and a donor nucleic acid. A study was conducted to analyze homology-directed repair efficiency of the nucleic acid editing system (TaRGET system) of the present disclosure, which comprises Cas12f1, TnpB, or a variant protein thereof, and to compare the resulting efficiency with homology-directed repair efficiency of the existing Cas9 and Cas12a. FIG. 2 illustrates a structure of an exemplary donor nucleic acid and a gene editing process using the same (gene editing by non-homologous end joining or homology-directed repair). In FIG. 2, 5'-TTTAGAGGGAGACACAAGTTGATAGGG-3' (SEQ ID NO: 296) was used as a target nucleotide sequence.

First, the efficiency of homology-directed repair and non-homologous end joining of Cas9, Cas12a, Cas12f, and TnpB was investigated for NLRC4 (NLR Family CARD Domain Containing 4) as an exemplary target gene. For TnpB, a protein with the amino acid sequence of SEQ ID NO: 1 was used. For Cas12f, a protein with the amino acid sequence of SEQ ID NO: 5 was used. Specific sequence information for Cas9 and Cas12a is provided in Table 8.

**[Table 8]**

| **Name** | **Amino acid sequence** | **SEQ ID NO** |
|---|---|---|
| Cas9 protein | DKKYSIGLDIGTNSVGWAVITDEYKVPSKKFKVLGNTDRHSI KKNLIGALLFDSGETAEATRLKRTARRRYTRRKNRICYLQEIF SNEMAKVDDSFFHRLEESFLVEEDKKHERHPIFGNIVDEVAYH EKYPTIYHLRKKLVDSTDKADLRLIYLALAHMIKFRGHFLIEG DLNPDNSDVDKLFIQLVQTYNQLFEENPINASGVDAKAILSAR LSKSRRLENLIAQLPGEKKNGLFGNLIALSLGLTPNFKSNFDL AEDAKLQLSKDTYDDDLDNLLAQIGDQYADLFLAAKNLSDA ILLSDILRVNTEITKAPLSASMIKRYDEHHQDLTLLKALVRQQL PEKYKEIFFDQSKNGYAGYIDGGASQEEFYKFIKPILEKMDGT EELLVKLNREDLLRKQRTFDNGSIPHQIHLGELHAILRRQEDF YPFLKDNREKIEKILTFRIPYYVGPLARGNSRFAWMTRKSEETI TPWNFEEVVDKGASAQSFIERMTNFDKNLPNEKVLPKHSLLY EYFTVYNELTKVKYVTEGMRKPAFLSGEQKKAIVDLLFKTNR KVTVKQLKEDYFKKIECFDSVEISGVEDRFNASLGTYHDLLKI IKDKDFLDNEENEDILEDIVLTLTLFEDREMIEERLKTYAHLFD DKVMKQLKRRRYTGWGRLSRKLINGIRDKQSGKTILDFLKS DGFANRNFMQLIHDDSLTFKEDIQKAQVSGQGDSLHEHIANL AGSPAIKKGILQTVKVVDELVKVMGRHKPENIVIEMARENQT | 297 |
| | TQKGQKNSRERMKRIEEGIKELGSQILKEHPVENTQLQNEKL YLYYLQNGRDMYVDQELDINRLSDYDVDHIVPQSFLKDDSID NKVLTRSDKNRGKSDNVPSEEVVKKMKNYWRQLLNAKLIT QRKFDNLTKAERGGLSELDKAGFIKRQLVETRQITKHVAQILD SRMNTKYDENDKLIREVKVITLKSKLVSDFRKDFQFYKVREI NNYHHAHDAYLNAVVGTALIKKYPKLESEFVYGDYKVYDVR KMIAKSEQEIGKATAKYFFYSNIMNFFKTEITLANGEIRKRPLI ETNGETGEIVWDKGRDFATVRKVLSMPQVNIVKKTEVQTGG FSKESILPKRNSDKLIARKKDWDPKKYGGFDSPTVAYSVLVV AKVEKGKSKKLKSVKELLGITIMERSSFEKNPIDFLEAKGYKE VKKDLIIKLPKYSLFELENGRKRMLASAGELQKGNELALPSK YVNFLYLASHYEKLKGSPEDNEQKQLFVEQHKHYLDEIIEQIS EFSKRVILADANLDKVLSAYNKHRDKPIREQAENIIHLFTLTN LGAPAAFKYFDTTIDRKRYTSTKEVLDATLIHQSITGLYETRID LSQLGGD | |
| Cas12a protein | TQFEGFTNLYQVSKTLRFELIPQGKTLKHIQEQGFIEEDKARN DHYKELKPIIDRIYKTYADQCLQLVQLDWENLSAAIDSYRKE KTEETRNALIEEQATYRNAIHDYFIGRTDNLTDAINKRHAEIY KGLFKAELFNGKVLKQLGTVTTTEHENALLRSFDKFTTYFSG FYENRKNVFSAEDISTAIPHRIVQDNFPKFKENCHIFTRLITAVP SLREHFENVKKAIGIFVSTSIEEVFSFPFYNQLLTQTQIDLYNQL LGGISREAGTEKIKGLNEVLNLAIQKNDETAHIIASLPHRFIPLF KQILSDRNTLSFILEEFKSDEEVIQSFCKYKTLLRNENVLETAE ALFNELNSIDLTHIFISHKKLETISSALCDHWDTLRNALYERRI SELTGKITKSAKEKVQRSLKHEDINLQEIISAAGKELSEAFKQ KTSEILSHAHAALDQPLPTTLKKQEEKEILKSQLDSLLGLYHL LDWFAVDESNEVDPEFSARLTGIKLEMEPSLSFYNKARNYATK KPYSVEKFKLNFQMPTLASGWDVNKEKNNGAILFVKNGLYY LGIMPKQKGRYKALSFEPTEKTSEGFDKMYYDYFPDAAKMIP | 298 |
| | KCSTQLKAVTAHFQTHTTPILLSNNFIEPLEITKEIYDLNNPEK EPKKFQTAYAKKTGDQKGYREALCKWIDFTRDFLSKYTKTTS IDLSSLRPSSQYKDLGEYYAELNPLLYHISFQRIAEKEIMDAVE TGKLYLFQIYNKDFAKGHHGKPNLHTLYWTGLFSPENLAKTS IKLNGQAELFYRPKSRMKRMAHRLGEKMLNKKLKDQKTPIP DTLYQELYDYVNHRLSHDLSDEARALLPNVITKEVSHEIIKDR RFTSDKFFFHVPITLNYQAANSPSKFNQRVNAYLKEHPETPIIG IDRGERNLIYITVIDSTGKILEQRSLNTIQQFDYQKKLDNREKE RVAARQAWSVVGTIKDLKQGYLSQVIHEIVDLMIHYQAVVVL ENLNFGFKSKRTGIAEKAVYQQFEKMLIDKLNCLVLKDYPAE KVGGVLNPYQLTDQFTSFAKMGTQSGFLFYVPAPYTSKIDPLT GFVDPFVWKTIKNHESRKHFLEGFDFLHYDVKTGDFILHFKM NRNLSFQRGLPGFMPAWDIVFEKNETQFDAKGTPFIAGKRIVP VIENHRFTGRYRDLYPANELIALLEEKGIVFRDGSNILPKLLEN DDSHAIDTMVALIRSVLQMRNSNAATGEDYINSPVRDLNGVC FDSRFQNPEWPMDADANGAYHIALKGQLLLNHLKESKDLKL QNGISNQDWLAYIQELRN | |

Sequence information for the guide RNA used for each of Cas9 and Cas12a is provided in Table 9.

**[Table 9]**

| **Item** | **Nucleotide sequence** | **SEQ ID NO** |
|---|---|---|
| Cas9 gRNA | GAGGGAGACACAAGTTGATAgttttagagctagaaatagcaagttaaaataaggc tagtccgttatcaacttgaaaaagtggcaccgagtcggtgcttttttt | 299 |
| Cas9 gRNA scaffold | gttttagagctagaaatagcaagttaaaataaggctagtccgttatcaacttgaaaaagtggcaccga gtcggtgc | 300 |
| Cas12a gRNA | taatttctactcttgtagatGAGGGAGACACAAGTTGATA | 301 |
| Cas12a crRNA | taatttctactcttgtagat | 302 |

The sequence of SEQ ID NO: 190 (GAGGGAGACACAAGTTGATA) was used as the sequence of the target gene NLRC4. In the target gene sequence, the PAM sequence of Cas12a and Cas12f1 is 5'-TTTA-3' located toward the 5' end of the target gene sequence, and the PAM sequence of Cas9 is 5'-GGG-3' located toward the 3' end of the target gene sequence. 1 µg of the vector comprising nucleotide sequences, which encode nuclease and gRNA, respectively, and 1 µg of the donor nucleic acid were transfected into HEK293T cells. Then, genomic DNA was extracted therefrom, the target region of NLRC4 was amplified by PCR, and deep sequencing (Illumina iSeq 100) was performed to investigate the efficiency of homology-directed repair and non-homologous end joining. The results of this experiment are illustrated in FIG. 3.

As can be seen in FIG. 3, under conditions where the donor nucleic acid (donor DNA in FIG. 3) is absent, non-homologous end joining (NHEJ) occurred with a similar efficiency of approximately 70% over all of the three different Cas systems (Cas9, Cas12a, and Cas12f). On the other hand, homology-directed repair occurred only in the presence of the donor nucleic acid. Cas9 and Cas12a showed low homology-directed repair efficiency of 4.13% and 4.26%, respectively, whereas TnpB showed high homology-directed repair efficiency of 11.87%. These results demonstrate that the target nucleic acid editing system of the present disclosure has significantly higher homology-directed repair efficiency than Cas9 and Cas12a.

### Example 2.2. Analysis of homology-directed repair efficiency depending on length of donor nucleic acid

Homology-directed repair efficiency was analyzed depending on the length of the donor nucleic acid. Specifically, unlike in Example 2.1 where the donor nucleic acid having a single length of 600 bp, donor nucleic acids with various sequence lengths of 8 kb, 6 kb, 5 kb, 4 kb, 3 kb, 2.5 kb, 2 kb, 1.6 kb, 1 kb, 800 bp, 600 bp, 400 bp, and 200 bp, respectively, were used in this example. The homology-directed repair efficiency was analyzed for the target gene NLRC4 in the same manner as in Example 2.1. The results of this experiment are illustrated in FIG. 4.

As shown in FIG. 4, it was identified that the target nucleic acid editing system according to an embodiment showed increased homology-directed repair efficiency in proportion to the length of the donor nucleic acid until it reached about 4 kb.

### Example 2.3. Analysis of homology-directed repair and non-homologous end-joining efficiency over time

Analysis was conducted to determine whether efficiency of homology-directed repair and non-homologous end joining varies depending on the time elapsed after transfection. Specifically, each of 1 µg of the vector comprising a nucleotide sequence encoding Cas9 or TaRGET system (TnpB) and 1 µg of the donor nucleic acid were transfected into HEK293T cells. NLRC4 was used as a target gene as in Example 2.1. After a certain period of time following the transfection, the cells were harvested, the target region was amplified, and the efficiency of homology-directed repair and non-homologous end joining was analyzed by deep sequencing. The transfected HEK293T cells were subcultured 5 days after the initial transfection date. Immediately after the subculture, the cells were transfected again with 1 µg of the vector comprising the nucleotide sequence encoding Cas9 or TaRGET (TnpB) system and 1 µg of the donor nucleic acid, as previously described. This procedure was also carried out 10 days after the initial transfection, and the gene editing efficiency was analyzed up to day 14 post-transfection. The results of this experiment are illustrated in FIG. 5.

As shown in FIG. 5, it was identified that both Cas9 and TaRGET (TnpB) systems showed increased homology-directed repair efficiency over time. In particular, the TaRGET system showed significantly increased homology-directed repair efficiency as compared with the Cas9 system. At the same time, it was found that the TaRGET system showed further decreased non-homologous end joining efficiency as compared with Cas9. In other words, it was identified that the TaRGET system showed significantly increased homology-directed repair efficiency, relative to non-homologous end joining efficiency, over time.

### Example 2.4. Analysis of homology-directed repair efficiency of Cas9, Cas12a, and TaRGET systems on various target genes

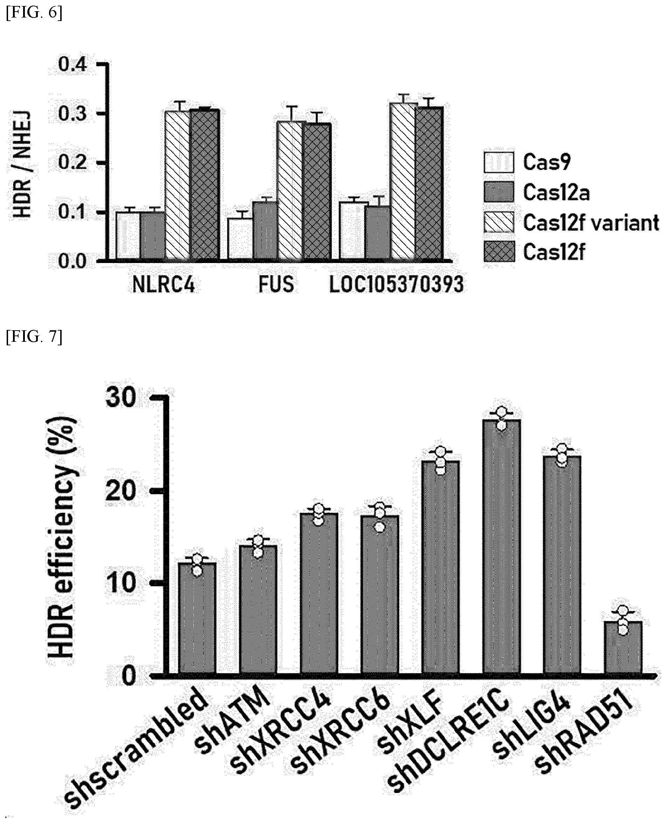

Homology-directed repair efficiency was analyzed for NLRC4, FUS, and LOC105370393 as target genes. The target sequences of the respective target genes used were the sequence of SEQ ID NO: 190 (GAGGGAGACACAAGTTGATA), the sequence of SEQ ID NO: 191 (GTGGGTAGGTCCAGTTTGGG), and the sequence of SEQ ID NO: 192 (GCAGTACACCTGAGGGAACA) in this order. In the target gene sequences, the PAM sequence of Cas12a and Cas12f variants is 5'-TTTA-3' located toward the 5' end of each target gene sequence, and the PAM sequence of Cas9 is 5'-GGG-3' located toward the 3' end of each target gene sequence. The sequence of SEQ ID NO: 193 (AACGTGACACGACGCGTTTCGGAGAAC) with a length of 27 bp was used as a desired sequence, and ge_4.0 (in a case where the target gene is LOC105370393) or ge_4.1 (in a case where the target gene is NLRC4 or FUS) in Tables 3 and 4 was used as gRNA. Each of 1 µg of the vector comprising a nucleotide sequence encoding Cas9, Cas12a, or TaRGET (Cas12f, TnpB) and 1 µg of the donor nucleic acid were transfected into HEK293T cells. Five days after the transfection, the cells were harvested, the target region was amplified, and the efficiency of homology-directed repair and non-homologous end joining was analyzed by deep sequencing. The results of this experiment are illustrated in FIG. 6.

As can be seen in FIG. 6, as compared with Cas9 and Cas12a, the TaRGET system (Cas12f, TnpB) showed significantly higher homology-directed repair efficiency, relative to non-homologous end joining efficiency, for all three target genes. For all three target genes, there was no significant difference in editing efficiency between Cas9 and Cas12a. However, as compared with Cas9 and Cas12a, the TaRGET system showed nearly three times higher homology-directed repair efficiency relative to non-homologous end joining efficiency.

### Example 3. Analysis of homology-directed repair efficiency through inhibited expression of gene involved in gene repair mechanism

### Example 3.1. Analysis of homology-directed repair efficiency through inhibited expression of non-homologous end joining (NHEJ)-related gene

As described above, in the presence of a donor DNA, both homology-directed repair and non-homologous end joining can occur, and thus the two gene repair mechanisms occur in a competitive manner. Therefore, to enhance induction toward homology-directed repair, expression of various genes known to be involved in non-homologous end joining was inhibited using shRNAs. For comparison, an experiment was also conducted to inhibit expression of Rad51 that is a gene involved in homology-directed repair.

Specifically, shRNAs targeting DCLRE1C, LIG4, XRCC4, KU70, XLF, ATM, and Rad51 genes (SEQ ID NO: 194 to 200 in this order) and a control (scrambled) shRNA (SEQ ID NO: 201) were produced. Specific sequence information for the shRNAs is provided in Table 10.

**[Table 10]**

| **Name** | **Nucleotide sequence** | **SEQ ID NO** |
|---|---|---|
| shRNA for DCLRE1C | GCAGAGCTCTCGTTTCACATTCAAGAGATGTGAAACGAGAG CTCTGC | 194 |
| shRNA for LIG4 | GCATGATCCTTCTGTAGGATTCAAGAGATCCTACAGAAGGAT CATGC | 195 |
| shRNA for XRCC4 | GAATCCACCTTGTTTCTGATTCAAGAGATCAGAAACAAGGT GGATTC | 196 |
| shRNA for KU70 | GCAGCATTGTGCAGATACATTCAAGAGATGTATCTGCACAAT GCTGC | 197 |
| shRNA for XLF | GCATGAGTCTGGCATTACATTCAAGAGATGTAATGCCAGACT CATGC | 198 |
| shRNA for ATM | GCAAGCAGCTGAAACAAATTTCAAGAGAATTTGTTTCAGCT GCTTGC | 199 |
| shRNA for Rad51 | CGCCAAAGAAGGAGCTAATAATTCAAGAGATTATTAGCTCCT TCTTTGGCG | 200 |
| shRNA for Scrambled | CAGAGCTAACTCAGATAGTACTTTCAAGAGAAGTACTATCTG AGTTAGCTCTG | 201 |

Each shRNA was transfected into the cells in the same manner as in Example 2.1 together with the TaRGET system comprising the CBA promoter and ge_4.1 gRNA and a 1.6 kb-sized donor nucleic acid (with 27 bp substitution), and then the homology-directed repair efficiency was analyzed. The results of this experiment are illustrated in FIG. 7. As can be seen in FIG. 7, the TaRGET system showed significantly increased homology-directed repair efficiency in a case where expression of the genes involved in non-homologous end joining was inhibited. On the other hand, it was found that in a case where expression of the Rad51 gene, which induces homology-directed repair, was inhibited, homology-directed repair efficiency was decreased.

These results indicated that inhibiting expression of genes involved in non-homologous end joining can increase homology-directed repair efficiency of the TaRGET system. In particular, due to the very small size of Cas protein and gRNA in the TaRGET system, there is an advantage that an shRNA molecule can be co-introduced into one delivery vector, thereby causing increased homology-directed repair efficiency.

### Example 3.2. Verification of homology-directed repair efficiency in DCLRE1C-knockout cell line

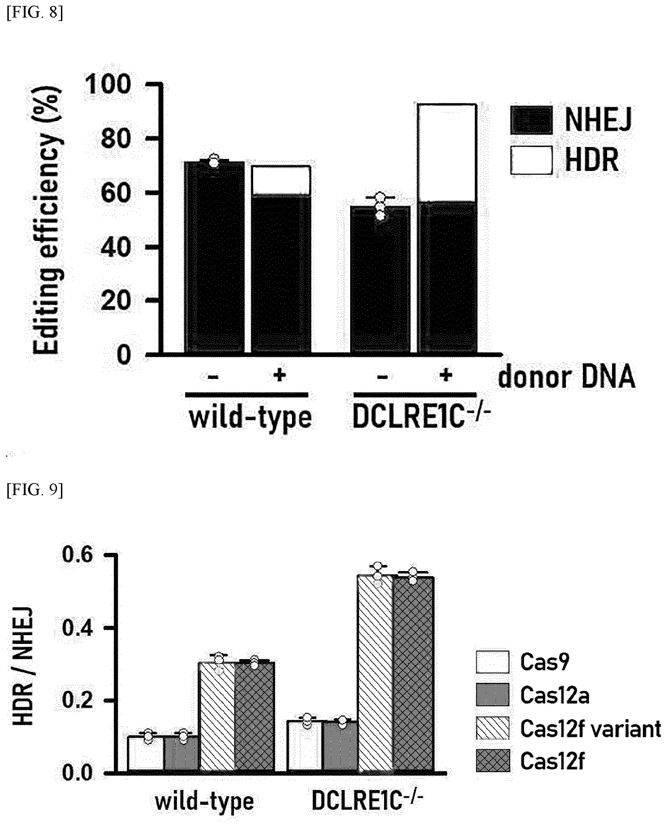

To further verify the effect of inhibiting DCLRE1C gene, which showed the greatest increase in homology-directed repair efficiency in Example 3.1, DCLRE1C-knockout HEK293T cell line was produced. Homology-directed repair efficiency, relative to non-homologous end joining efficiency, was investigated for the target gene DCLR4 in the DCLRE1C^{-/-} cell line and the wild-type HEK293T cell line. The results of this experiment are illustrated in FIG. 8.

As can be seen in FIG. 8, the DCLRE1C-knockout cells showed significantly increased homology-directed repair efficiency relative to non-homologous end joining efficiency, as compared with the wild-type cells.

Then, the two types of cells were transfected with each of 1 µg of the vector comprising a nucleotide sequence encoding Cas9, Cas12a, or TaRGET (Cas12f1 variant) system and 1 µg of the donor nucleic acid. Five days after the transfection, the cells were harvested, the target region was amplified, and the efficiency of homology-directed repair and non-homologous end joining was analyzed by deep sequencing. The results of this experiment are illustrated in FIG. 9.

As can be seen in FIG. 9, as compared with Cas9 and Cas12a, the TaRGET (Cas12f1 variant) system showed high homology-directed repair efficiency relative to non-homologous end joining efficiency in the wild-type cell line. Such comparative advantage of the TaRGET system was more strikingly observed in the DCLRE1C-knockout cell line. Specifically, an increase in homology-directed repair efficiency due to knockout of DCLRE1C gene was also observed for the Cas9 and Cas12a systems; however, the increase was minimal. In contrast, a significantly larger increase in homology-directed repair efficiency was observed for the TaRGET system. These results demonstrate that in a case where a module capable of inhibiting expression of DCLRE1C gene is additionally incorporated into the TaRGET system, the TaRGET system can achieve a superior level of homology-directed repair efficiency that is not achievable with Cas9 and Cas12a.

### Example 4. Optimization of homology-directed repair efficiency using AAV delivery vehicle

In order to efficiently deliver a target nucleic acid editing system for homology-directed repair using an AAV delievery vehicle, all components must be included within the packaging limit of AAV which is approximately 4.7 kb. The components comprise, as essential elements, the TaRGET system comprising a guide RNA, and the donor nucleic acid, and may further comprise the shRNAs and the like for regulating expression of genes involved in non-homologous end joining identified in Example 3. On the other hand, as identified in Example 2.2, the homology-directed repair efficiency increased in proportion to the length of the donor nucleic acid until it reached about 4 kb, and therefore, the donor nucleic acid with the maximum possible length was used depending on the type of promoter and the presence or absence of shRNA.

Specifically, four different TaRGET system vector compositions were prepared which correspond to cases where a relatively longer CBA (chicken β-actin) promoter or a relatively shorter EFS (elongation factor 1α short) promoter is used and shDCLRE1C is present or absent. The homology-directed repair efficiency of each vector composition was investigated for the NLRC4, FUS, and LOC105370393 genes. 2 µg of the vector composition as prepared above was transfected into wild-type HEK293T cells. After 5 days, the cells were harvested, the target region was amplified, and the homology-directed repair efficiency was analyzed by deep sequencing. The results of this experiment are illustrated in FIG. 10.

As shown in FIG. 10A, a comparison was performed, through respective vector compositions, between the CBA promoter and the EFS promoter as promoters for TnpB expression (see the comparisons between A and C, and between B and D in FIG. 10A). For these promoters, four different vector compositions were constructed depending on whether shDCLRE1C was present or absent. For each vector composition, the entire construct was uniformly sized to have a length within 4.7 kb by adjusting the length of the donor nucleic acid to the maximum extent depending on the length of the promoter and the presence or absence of shDCLRE1C. In this way, the construct was made feasible for delivery using AAV

As shown in FIG. 10B, the experimental results indicated that in a case where a longer CBA promoter was used, the homology-directed repair efficiency did not significantly increase even with addition of shDCLREIC (see the comparison between A and B in FIG. 10B). This result is believed to be because addition of shDCLRE1C led to a decrease in the length of the donor nucleic acid, which in turn decreased the homology-directed repair efficiency so that the effect of shDCLRE1C is partially offset. On the other hand, in a case where the EFS promoter was used, the homology-directed repair efficiency significantly increased with addition of shDCLRE1C (see the comparison between C and D in FIG. 10B). This is because, even with addition of shDCLRE1C, a longer donor nucleic acid can be used as compared with a case where the CBA promoter is used, which allows the highest homology-directed repair efficiency to be achieved.

These results demonstrate that by adjusting and selecting the type of promoter, the length of donor nucleic acid, the use of molecules for inhibiting expression of non-homologous end joining regulatory genes, and the like, it is possible to achieve the optimal homology-directed repair efficiency upon AAV vector-mediated delivery.

The description of the present disclosure as stated above is for illustrative purposes only, and those skilled in the art of the present disclosure will understand that the present disclosure can be readily modified into other specific forms without departing from its technical spirit or essential features. Therefore, the embodiments as described above should be understood in all respects as illustrative and not restrictive.

## Claims

1. An editing system for a target nucleic acid, comprising:
an endonuclease comprising Cas12f1, TnpB, or a variant protein thereof, or a nucleic acid encoding the endonuclease;
an engineered guide RNA comprising a guide sequence or a nucleic acid encoding the guide RNA; and
a donor nucleic acid molecule or a nucleic acid encoding the donor nucleic acid molecule.

2. The system of claim 1, wherein the system causes double-strand breaks in the target nucleic acid.

3. The system of claim 1, wherein the system is such that a desired sequence is introduced in the target nucleic acid or a region adjacent thereto by homology-directed repair of double-strand breaks using the donor nucleic acid molecule as a template.

4. A gene editing composition for a target nucleic acid, comprising:
an endonuclease comprising Cas12f1, TnpB, or a variant protein thereof, or a nucleic acid encoding the endonuclease;
an engineered guide RNA comprising a guide sequence or a nucleic acid encoding the guide RNA; and
a donor nucleic acid molecule or a nucleic acid encoding the donor nucleic acid molecule.

5. The system or composition of claim 1 or 4, wherein the Cas12f1, TnpB, or the variant protein thereof comprises an amino acid sequence having at least 70% sequence identity with an amino acid sequence selected from the group consisting of SEQ ID NOS: 1 to 5.

6. The system or composition of claim 1 or 4, wherein the TnpB protein comprises an amino acid sequence having at least 70% sequence identity with an amino acid sequence selected from the group consisting of SEQ ID NOS: 202 to 293.

7. The system or composition of claim 1 or 4, wherein the Cas12f1, TnpB, or the variant protein thereof comprises one selected from the following sequences:
(i) the amino acid sequence of SEQ ID NO: 5;
(ii) the amino acid sequence of SEQ ID NO: 1;
(iii) an amino acid sequence having the amino acid sequence of SEQ ID NO: 1 in which 1 to 28 amino acids at the N-terminus have been removed or substituted; or
(iv) an amino acid sequence having the amino acid sequence of SEQ ID NO: 1 in which 1 to 600 amino acids have been added to the N-terminus or C-terminus.

8. The system or composition of claim 7, wherein the added 1 to 600 amino acids are the amino acid sequence of SEQ ID NO: 294 or 295.

9. The system or composition of claim 1 or 4, wherein the Cas12f1, TnpB, or the variant protein thereof has at least 70% sequence identity with an amino acid sequence selected from the group consisting of SEQ ID NOS: 1 to 4.

10. The system or composition of claim 1 or 4, wherein the Cas12f1, TnpB, or the variant protein thereof has at least 70% sequence identity with the amino acid sequence of SEQ ID NO: 1.

11. The system or composition of claim 1 or 4, wherein the engineered guide RNA comprises substitution, deletion, insertion, or addition of one or more nucleotides in reference with a wild-type Cas12f1 guide RNA sequence, and a portion of the engineered guide RNA, excluding the guide sequence, has at least 50% sequence identity with the wild-type Cas12f1 guide RNA.

12. The system or composition of claim 1 or 4, wherein the wild-type Cas12f1 guide RNA comprises a trans-activating CRISPR RNA (tracrRNA) and a CRISPR RNA (crRNA) which comprise (i) one or more stem regions, (ii) a tracrRNA-crRNA complementarity region, and optionally (iii) a region containing three or more consecutive uracil (U) residues, and
the engineered guide RNA comprises at least one modification selected from the group consisting of (a) to (d):
(a) deletion of at least a part of one or more stem regions;
(b) deletion of at least a part of the tracrRNA-crRNA complementarity region;
(c) replacement of one or more uracil (U) residues with A, G, or C in three or more consecutive U residues when the consecutive U residues are present in the tracrRNA-crRNA complementarity region; and
(d) addition of one or more uridine residues to the 3'-end of the crRNA sequence.

13. The system or composition of claim 12, wherein the wild-type Cas12f1 guide RNA comprises tracrRNA consisting of the nucleotide sequence of SEQ ID NO: 11 and crRNA consisting of the nucleotide sequence of SEQ ID NO: 12.

14. The system or composition of claim 12, wherein the wild-type Cas12f1 guide RNA comprises a tracrRNA and a crRNA which sequentially comprise, from the 5'-end, a first stem region, a second stem region, a third stem region, a fourth stem region, and the tracrRNA-crRNA complementarity region, and
the engineered guide RNA comprises at least one modification selected from the group consisting of:
(a1) deletion of at least a part of the first stem region;
(a2) deletion of at least a part of the second stem region;
(b) deletion of at least a part of the tracrRNA-crRNA complementarity region;
(c) replacement of one or more uracil (U) residues with A, G, or C in three or more consecutive U residues when the consecutive U residues are present in the tracrRNA-crRNA complementarity region; and
(d1) addition of a U-rich tail to the 3'-end of the crRNA sequence (in which a sequence of the U-rich tail is represented by 5'-(UₘV)ₙUₒ-3', where V is each independently A, C, or G, m and o are integers between 1 and 20, and n is an integer between 0 and 5).

15. The system or composition of claim 14, wherein the engineered guide RNA comprises (d1) addition of a U-rich tail to the 3'-end of the crRNA sequence, (c) replacement of one or more uracil (U) residues with A, G, or C in three or more consecutive U residues when the consecutive U residues are present in the tracrRNA-crRNA complementarity region, or both modifications.

16. The system or composition of claim 14, wherein the engineered guide RNA comprises at least one modification selected from the group consisting of (a1) deletion of at least a part of the first stem region; (a2) deletion of at least a part of the second stem region; and (b) deletion of at least a part of the tracrRNA-crRNA complementarity region.

17. The system or composition of claim 16, wherein the engineered guide RNA comprises (b 1) deletion of a part of the tracrRNA-crRNA complementarity region, and the part of the complementary region consists of 1 to 54 nucleotides.

18. The system or composition of claim 16, wherein the engineered guide RNA comprises (b2) deletion of the entire tracrRNA-crRNA complementarity region, and the entire complementary region consists of 55 nucleotides.

19. The system or composition of claim 14, wherein the engineered guide RNA comprises (a1) deletion of at least a part of the first stem region, and the at least a part of the stem region consists of 1 to 20 nucleotides.

20. The system or composition of claim 14, wherein the engineered guide RNA comprises (a2) deletion of at least a part of the second stem region, and the at least a part of the stem region consists of 1 to 27 nucleotides.

21. The system or composition of claim 14, wherein the engineered guide RNA comprises (a1) deletion of at least a part of the first stem region; (d1) addition of a U-rich tail to the 3'-end of the crRNA sequence; or both modifications.

22. The system or composition of claim 1 or 4, wherein the engineered guide RNA consists of a sequence represented by Formula (I) or has at least 80% sequence identity therewith: 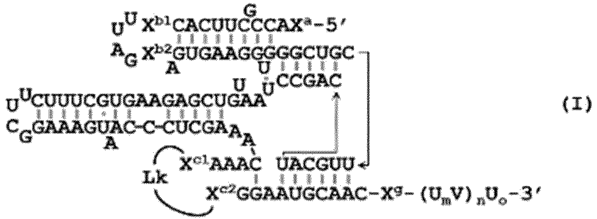 in Formula (I),
X^{a}, X^{b1}, X^{b2}, X^{c1}, and X^{c2} each independently consists of 0 to 35 (poly)nucleotides,
X^{g} is a guide sequence that consists of 10 to 30 nucleotides and is hybridizable with or complementary to a target sequence,
Lk is a polynucleotide linker of 2 to 20 nucleotides or is absent, and
(UₘV)ₙUₒ is present as a U-rich tail or absent, and when (UₘV)ₙUₒ is present, U is uridine, V is each independently A, C, or G, m and o are each independently an integer between 1 and 20, and n is an integer between 0 and 5.

23. The system or composition of claim 22, wherein X^{a} comprises the nucleotide sequence of SEQ ID NO: 14 or a nucleotide sequence having the sequence of SEQ ID NO: 14 from which 1 to 20 nucleotides are deleted.

24. The system or composition of claim 22, wherein X^{b1} comprises the nucleotide sequence of SEQ ID NO: 25 or a nucleotide sequence having the sequence of SEQ ID NO: 25 from which 1 to 13 nucleotides are deleted.

25. The system or composition of claim 22, wherein X^{b2} comprises the nucleotide sequence of SEQ ID NO: 29 or a nucleotide sequence having the sequence of SEQ ID NO: 29 from which 1 to 14 nucleotides are deleted.

26. The system or composition of claim 22, wherein the sequence 5'-X^{b1}UUAGX^{b2}-3' in Formula (I) is any one nucleotide sequence selected from the group consisting of SEQ ID NOS: 34 to 38.

27. The method of claim 22, wherein X^{c1} comprises the nucleotide sequence of SEQ ID NO: 39 or a nucleotide sequence having the sequence of SEQ ID NO: 39 from which 1 to 28 nucleotides are deleted.

28. The system or composition of claim 27, wherein when three or more consecutive uracil (U) residues are present in a sequence of X^{c1}, the sequence of X^{c1} comprises a modification in which at least one uracil residue thereof is replaced with A, G, or C.

29. The system or composition of claim 22, wherein X^{c2} comprises the nucleotide sequence of SEQ ID NO: 58 or a nucleotide sequence having the sequence of SEQ ID NO: 58 from which 1 to 27 nucleotides are deleted.

30. The system or composition of claim 29, wherein when the sequence 5'-ACGAA-3' is present in X^{c2}, the sequence is replaced with 5'-NGNNN-3', and N is each independently A, C, G, or U.

31. The system or composition of claim 22, wherein the sequence 5'-X^{c1}-Lk-X^{c2}-3' in Formula (I) is any one nucleotide sequence selected from the group consisting of SEQ ID NOS: 80 to 86.

32. The system or composition of claim 22, wherein Lk comprises any one nucleotide sequence selected from the group consisting of 5'-GAAA-3', 5'-UUAG-3', 5'-UGAAAA-3', 5'-UUGAAAAA-3', 5'-UUCGAAAGAA-3' (SEQ ID NO: 76), 5'-UUCAGAAAUGAA-3' (SEQ ID NO: 77), 5'-UUCAUGAAAAUGAA-3' (SEQ ID NO: 78), and 5'-UUCAUUGAAAAAUGAA-3' (SEQ ID NO: 79).

33. The system or composition of claim 22, wherein (UₘV)ₙUₒ is such that (i) n is 0 and o is an integer between 1 and 6, or (ii) V is A or G, m and o are each independently an integer between 3 and 6, and n is an integer between 1 and 3.

34. The system or composition of claim 14, wherein the engineered guide RNA comprises an engineered tracrRNA consisting of any one nucleotide sequence selected from the group consisting of SEQ ID NOS: 87 to 132.

35. The system or composition of claim 14, wherein the engineered guide RNA comprises an engineered crRNA consisting of any one nucleotide sequence selected from the group consisting of SEQ ID NOS: 133 to 148.

36. The system or composition of claim 12, wherein the engineered guide RNA is a dual guide RNA or a single guide RNA.

37. The system or composition of claim 36, wherein the engineered single guide RNA consists of any one nucleotide sequence selected from the group consisting of SEQ ID NOS: 149 to 186.

38. The system or composition of claim 1 or 4, wherein the endonuclease comprising Cas12f1, TnpB, or a variant protein thereof; and the guide RNA are included in a form of a ribonucleoprotein (RNP).

39. The system or composition of claim 1 or 4, wherein the donor nucleic acid molecule is a sequence used as a template in homology-directed repair and has a length of 1 bp to 20 kb.

40. The system or composition of claim 1 or 4, wherein the system or composition further comprises a molecule that inhibits expression of a gene involved in non-homologous end joining (NHEJ).

41. The system or composition of claim 40, wherein the gene involved in non-homologous end joining is at least one selected from the group consisting of ATM1, XRCC4, XLF, XRCC6, LIG4, and DCLRE1C.

42. The system or composition of claim 40, wherein the molecule is shRNA, siRNA, miRNA, or antisense oligonucleotide.

43. A vector system comprising at least one vector that comprises:
a first nucleic acid construct to which a nucleotide sequence encoding an endonuclease is operably linked, the endonuclease comprising Cas12f1, TnpB, or a variant protein thereof;
a second nucleic acid construct to which a nucleotide sequence encoding an engineered guide RNA is operably linked, the engineered guide RNA comprising a guide sequence that binds complementarily to a target nucleic acid; and
a third nucleic acid construct comprising a donor nucleic acid molecule.

44. The vector system of claim 43, wherein the nucleic acid constructs included in the vector system are located in the same or different vectors.

45. The vector system of claim 43, further comprising at least one nucleic acid construct to which a nucleotide sequence encoding a molecule is operably linked, the molecule inhibiting expression of a gene involved in non-homologous end joining (NHEJ).

46. The vector system of claim 45, wherein the gene involved in non-homologous end joining is at least one selected from the group consisting of ATM1, XRCC4, XLF, XRCC6, LIG4, and DCLRE1C.

47. The vector system of claim 45, wherein the molecule is shRNA, siRNA, miRNA, or antisense oligonucleotide.

48. The vector system of claim 43, wherein respective components in the vector are included in one vector.

49. The vector system of claim 43, wherein the donor nucleic acid molecule has a length of 1 bp to 20 kb.

50. The vector system of claim 43, wherein the vector further comprises a promoter or an enhancer.

51. The vector system of claim 50, wherein the promoter is U6 promoter, EFS promoter, EF1-α promoter, H1 promoter, 7SK promoter, CMV promoter, LTR promoter, Ad MLP promoter, HSV promoter, SV40 promoter, CBA promoter, or RSV promoter.

52. The vector system of claim 43, wherein the vector is at least one viral vector selected from the group consisting of a retroviral (retrovirus) vector, a lentiviral (lentivirus) vector, an adenoviral (adenovirus vector), an adeno-associated viral (adeno-associated virus; AAV) vector, a vaccinia viral (vaccinia virus) vector, a poxviral (poxvirus) vector, a herpes simplex viral (herpes simplex virus) vector, and a phagemid vector.

53. The vector system of claim 52, wherein the vector is an adeno-associated viral vector, and the adeno-associated viral vector is such that all components within the vector can be included in one vector.

54. The vector system of claim 43, wherein the vector is at least one non-viral vector selected from the group consisting of plasmid, naked DNA, DNA complex, mRNA (transcript), and amplicon.

55. The vector system of claim 54, wherein the plasmid is at least one selected from the group consisting of pcDNA series, pSC101, pGV1106, pACYC177, ColE1, pKT230, pME290, pBR322, pUC8/9, pUC6, pBD9, pHC79, pIJ61, pLAFR1, pHV14, pGEX series, pET series, and pUC19.

56. The vector system of claim 43, wherein the Cas12f1, TnpB, or the variant protein thereof comprises an amino acid sequence having at least 70% sequence identity with an amino acid sequence selected from the group consisting of SEQ ID NOS: 1 to 5.

57. The vector system of claim 43, wherein the TnpB protein comprises an amino acid sequence having at least 70% sequence identity with an amino acid sequence selected from the group consisting of SEQ ID NOS: 202 to 293.

58. The vector system of claim 43, wherein the Cas12f1, TnpB, or the variant protein thereof comprises one selected from the following sequences:
(i) the amino acid sequence of SEQ ID NO: 5;
(ii) the amino acid sequence of SEQ ID NO: 1;
(iii) an amino acid sequence having the amino acid sequence of SEQ ID NO: 1 in which 1 to 28 amino acids at the N-terminus have been removed or substituted; or
(iv) an amino acid sequence having the amino acid sequence of SEQ ID NO: 1 in which 1 to 600 amino acids have been added to the N-terminus or C-terminus.

59. The vector system of claim 58, wherein the added 1 to 600 amino acids are the amino acid sequence of SEQ ID NO: 294 or 295.

60. The vector system of claim 43, wherein the Cas12f1, TnpB, or the variant protein thereof has at least 70% sequence identity with an amino acid sequence selected from the group consisting of SEQ ID NOS: 1 to 4.

61. The vector system of claim 43, wherein the Cas12f1, TnpB, or the variant protein thereof has at least 70% sequence identity with the amino acid sequence of SEQ ID NO: 1.

62. The vector system of claim 43, wherein the engineered guide RNA comprises substitution, deletion, insertion, or addition of one or more nucleotides in reference with a wild-type Cas12f1 guide RNA sequence, and the engineered guide RNA, excluding the guide sequence, has at least 50% sequence identity with the wild-type Cas12f1 guide RNA.

63. The vector system of claim 43, wherein the wild-type Cas12f1 guide RNA comprises a trans-activating CRISPR RNA (tracrRNA) and a CRISPR RNA (crRNA) which comprise (i) at least one stem region, (ii) a tracrRNA-crRNA complementarity region, and optionally (iii) a region containing three or more consecutive uracil (U) residues, and
the engineered guide RNA comprises at least one modification selected from the group consisting of (a) to (d):
(a) deletion of at least a part of one or more stem regions;
(b) deletion of at least a part of the tracrRNA-crRNA complementarity region;
(c) replacement of one or more uracil (U) residues with A, G, or C in three or more consecutive U residues when the consecutive U residues are present in the tracrRNA-crRNA complementarity region; and
(d) addition of one or more uridine residues to the 3'-end of the crRNA sequence.

64. The vector system of claim 63, wherein the wild-type Cas12f1 guide RNA comprises tracrRNA consisting of the nucleotide sequence of SEQ ID NO: 11 and crRNA consisting of the nucleotide sequence of SEQ ID NO: 12.

65. The vector system of claim 43, wherein the wild-type Cas12f1 guide RNA comprises a tracrRNA and a crRNA which sequentially comprise, from the 5'-end, a first stem region, a second stem region, a third stem region, a fourth stem region, and the tracrRNA-crRNA complementarity region, and
the engineered guide RNA comprises at least one modification selected from the group consisting of:
(a1) deletion of at least a part of the entire first stem region;
(a2) deletion of at least a part of the second stem region;
(b) deletion of at least a part of the tracrRNA-crRNA complementarity region;
(c) replacement of one or more uracil (U) residues with A, G, or C in three or more consecutive U residues when the consecutive U residues are present in the tracrRNA-crRNA complementarity region; and
(d1) addition of a U-rich tail to the 3'-end of the crRNA sequence (in which a sequence of the U-rich tail is represented by 5'-(UₘV)ₙUₒ-3', where V is each independently A, C, or G, m and o are integers between 1 and 20, and n is an integer between 0 and 5).

66. The vector system of claim 65, wherein the engineered guide RNA comprises (d1) addition of a U-rich tail to the 3'-end of the crRNA sequence, (c) replacement of one or more uracil (U) residues with A, G, or C in three or more consecutive U residues when the consecutive U residues are present in the tracrRNA-crRNA complementarity region, or both modifications.

67. The vector system of claim 65, wherein the engineered guide RNA comprises at least one modification selected from the group consisting of (a1) deletion of at least a part of the first stem region; (a2) deletion of at least a part of the second stem region; and (b) deletion of at least a part of the tracrRNA-crRNA complementarity region.

68. The vector system of claim 67, wherein the engineered guide RNA comprises (b1) deletion of a part of the tracrRNA-crRNA complementarity region, and the part of the complementary region consists of 1 to 54 nucleotides.

69. The system or composition of claim 67, wherein the engineered guide RNA comprises (b2) deletion of the entire tracrRNA-crRNA complementarity region, and the entire complementary region consists of 55 nucleotides.

70. The vector system of claim 67, wherein the engineered guide RNA comprises (a1) deletion of at least a part of the first stem region, and the at least a part of the stem region consists of 1 to 20 nucleotides.

71. The vector system of claim 67, wherein the engineered guide RNA comprises (a2) deletion of at least a part of the second stem region, and the at least a part of the stem region consists of 1 to 27 nucleotides.

72. The vector system of claim 67, wherein the engineered guide RNA comprises (a1) deletion of at least a part of the first stem region; (d1) addition of a U-rich tail to the 3'-end of the crRNA sequence; or both modifications.

73. The system or composition of claim 43, wherein the engineered guide RNA consists of a sequence represented by Formula (I) or has at least 80% sequence identity therewith: 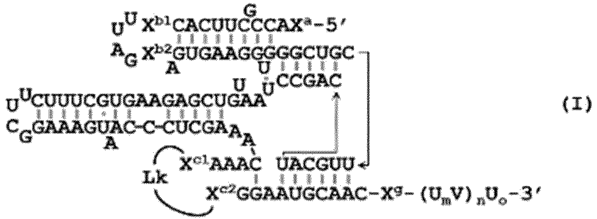 in Formula (I),
X^{a}, X^{b1}, X^{b2}, X^{c1}, and X^{c2} each independently consists of 0 to 35 (poly)nucleotides,
X^{g} is a guide sequence that consists of 10 to 30 nucleotides and is hybridizable with or complementary to a target sequence,
Lk is a polynucleotide linker of 2 to 20 nucleotides or is absent, and
(UₘV)ₙUₒ is present as a U-rich tail or absent, and when (UₘV)ₙUₒ is present, U is uridine, V is each independently A, C, or G, m and o are each independently an integer between 1 and 20, and n is an integer between 0 and 5.

74. The vector system of claim 73, wherein X^{a} comprises the nucleotide sequence of SEQ ID NO: 14 or a nucleotide sequence having the sequence of SEQ ID NO: 14 from which 1 to 20 nucleotides are deleted.

75. The vector system of claim 73, wherein X^{b1} comprises the nucleotide sequence of SEQ ID NO: 25 or a nucleotide sequence having the sequence of SEQ ID NO: 25 from which 1 to 13 nucleotides are deleted.

76. The vector system of claim 73, wherein X^{b2} comprises the nucleotide sequence of SEQ ID NO: 29 or a nucleotide sequence having the sequence of SEQ ID NO: 29 from which 1 to 14 nucleotides are deleted.

77. The vector system of claim 73, wherein the sequence 5'-X^{b1}LTUAGX^{b2}-3' in Formula (I) is any one nucleotide sequence selected from the group consisting of SEQ ID NOS: 34 to 38.

78. The vector system of claim 73, wherein X^{c1} comprises the nucleotide sequence of SEQ ID NO: 39 or a nucleotide sequence having the sequence of SEQ ID NO: 39 from which 1 to 28 nucleotides are deleted.

79. The vector system of claim 78, wherein when three or more consecutive uracil (U) residues are present in a sequence of X^{c1}, the sequence of X^{c1} comprises a modification in which at least one uracil residue thereof is replaced with A, G, or C.

80. The vector system of claim 73, wherein X^{c2} comprises the nucleotide sequence of SEQ ID NO: 58 or a nucleotide sequence having the sequence of SEQ ID NO: 58 from which 1 to 27 nucleotides are deleted.

81. The vector system of claim 80, wherein when the sequence 5'-ACGAA-3' is present in X^{c2}, the sequence is replaced with 5'-NGNNN-3', and N is each independently A, C, G, or U.

82. The vector system of claim 73, wherein the sequence 5'-X^{c1}-Lk-X^{c2}-3' in Formula (I) is any one nucleotide sequence selected from the group consisting of SEQ ID NOS: 80 to 86.

83. The vector system of claim 73, wherein Lk is any one nucleotide sequence selected from the group consisting of 5'-GAAA-3', 5'-UUAG-3', 5'-UGAAAA-3', 5'-UUGAAAAA-3', 5'-UUCGAAAGAA-3' (SEQ ID NO: 76), 5'-UUCAGAAAUGAA-3' (SEQ ID NO: 77), 5'-UUCAUGAAAAUGAA-3' (SEQ ID NO: 78), and 5'-UUCAUUGAAAAAUGAA-3' (SEQ ID NO: 79).

84. The vector system of claim 73, wherein (UₘV)ₙUₒ is such that (i) n is 0 and o is an integer between 1 and 6, or (ii) V is A or G, m and o are each independently an integer between 3 and 6, and n is an integer between 1 and 3.

85. The vector system of claim 43, wherein the engineered guide RNA comprises an engineered tracrRNA consisting of any one nucleotide sequence selected from the group consisting of SEQ ID NOS: 87 to 132.

86. The vector system of claim 43, wherein the engineered guide RNA comprises an engineered crRNA consisting of any one nucleotide sequence selected from the group consisting of SEQ ID NOS: 133 to 148.

87. The vector system of claim 43, wherein the engineered guide RNA is a dual guide RNA or a single guide RNA.

88. The vector system of claim 43, wherein the engineered single guide RNA consists of any one nucleotide sequence selected from the group consisting of SEQ ID NOS: 149 to 186.

89. A virus produced by the vector system of any one of claims 43 to 88.

90. The virus of claim 89, wherein the virus is selected from the group consisting of retrovirus, lentivirus, adenovirus, adeno-associated virus, vaccinia virus, poxvirus, herpes simplex virus, and phage.

91. A composition comprising the virus of claim 89.

92. A method for introducing a desired sequence into a target region on a double-stranded nucleic acid in a cell, comprising
bringing, into contact with the cell, the system or composition of any one of claims 1 to 42, or the vector system of any one of claims 43 to 88, or expressing the same in the cell; and
allowing the desired sequence to be introduced in a target nucleic acid or a region adjacent thereto by repair of double-strand breaks using the donor nucleic acid molecule as a template.

93. The method of claim 92, wherein the repair of double-strand breaks is by homology-directed repair mechanism.

94. The method of claim 92, wherein the cell is a prokaryotic cell or eukaryotic cell in which a target nucleic acid or target gene is present.

95. The method of claim 94, wherein the eukaryotic cell is a yeast, an insect cell, a plant cell, a non-human-animal cell, or a human cell.

96. The method of claim 92, wherein the vector system is introduced into a packaging virus selected from the group consisting of retrovirus, lentivirus, adenovirus, adeno-associated virus, vaccinia virus, poxvirus, herpes simplex virus, and phage, and is delivered into a prokaryotic cell or eukaryotic cell in a form of a virus produced by the packaging virus.

97. The method of claim 92, wherein the vector system is delivered into a prokaryotic cell or eukaryotic cell by electroporation, gene gun, sonoporation, magnetofection, transient cell compression or squeezing, cationic liposome method, lithium acetate-DMSO, lipid-mediated transfection, calcium phosphate precipitation, lipofection, polyethyleneimine (PEI)-mediated transfection, DEAE-dextran mediated transfection, or nanoparticle-mediated nucleic acid delivery.

98. The method of claim 92, wherein the vector system is delivered directly into a prokaryotic cell or eukaryotic cell through at least one lipid nanoparticle (LNP).

99. The method of claim 92, wherein the bringing-into-contact or the expressing occurs *in vivo* or *ex vivo.*
